(19) Europäisches Patentamt
European Patent Office
Office européen des brevets

(11) EP 2 906 526 B1

(12) **FASCICULE DE BREVET EUROPEEN**

(45) Date de publication et mention de la délivrance du brevet:
**14.08.2019 Bulletin 2019/33**

(21) Numéro de dépôt: **13766594.9**

(22) Date de dépôt: **28.08.2013**

(51) Int Cl.:
***C07C 41/30*** *(2006.01)* ***C07C 43/23*** *(2006.01)*
***C08G 8/12*** *(2006.01)*

(86) Numéro de dépôt international:
**PCT/FR2013/051989**

(87) Numéro de publication internationale:
**WO 2014/033407 (06.03.2014 Gazette 2014/10)**

(54) **PROCEDE DE PREPARATION A HAUTS RENDEMENTS DE P-(R)CALIXARENES GEANTS**

VERFAHREN ZUR ERGIEBIGE PRODUKTION VON RIESEN P-(R) CALIXARENE

METHOD FOR THE HIGH-YIELD PRODUCTION OF GIANT P-(R)CALIXARENES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.08.2012 FR 1258054**

(43) Date de publication de la demande:
**19.08.2015 Bulletin 2015/34**

(73) Titulaire: **Université Paris Sud-Paris XI**
**97405 Orsay Cedex (FR)**

(72) Inventeurs:
- **HUC, Vincent, Germain**
  **91400 Orsay (FR)**
- **MARTINI, Cyril**
  **91140 Villebon-sur-Yvette (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**EP-A1- 1 361 208 CA-A1- 2 251 070**
**FR-A1- 2 795 077**

- **ALESSANDRO CASNATI ET AL: "p -(Benzyloxy)calix[8]arene: One-Pot Synthesis and Functionalization", THE JOURNAL OF ORGANIC CHEMISTRY, vol. 62, no. 18, 1 septembre 1997 (1997-09-01), pages 6236-6239, XP55034562, ISSN: 0022-3263, DOI: 10.1021/jo970620r**
- **VINCENT HUC ET AL: "C 3 v (Trimethyl) p -(Benzyloxy)calix[6]arene: A Versatile Platform for the Synthesis of Functionalized C 3 v Calix[6]arenes", EUROPEAN JOURNAL OF ORGANIC CHEMISTRY, vol. 2010, no. 11, 1 avril 2010 (2010-04-01), pages 2199-2205, XP55057188, ISSN: 1434-193X, DOI: 10.1002/ejoc.200901281**
- **C DAVID GUTSCHE ET AL: "Calixarenes: paradoxes and paradigms in molecular baskets", PURE & APPLIED CHEMISTRY, vol. 62, no. 3, 1 janvier 1990 (1990-01-01), pages 485-491, XP55057932,**
- **OSTASZEWSKI R ET AL: "Influence of Base and Solvent on the Reaction between p-Cresol and Formaldehyde Leading to p-Methalcalix(n)arenes", POLISH JOURNAL OF CHEMISTRY, POLSKIE TOWARZYSTWO CHEMICZNE, PL, vol. 71, no. 8, 1 janvier 1997 (1997-01-01), pages 1053-1059, XP009168220, ISSN: 0137-5083**
- **NAKAYAMA T ET AL: "NEW POSITIVE-TYPE PHOTORESIST BASED ON MONO-SUBSTITUTED HYDROQUINONE CALIX (8) ARENE AND DIAZONAPHTHOQUINONE", JOURNAL OF MATERIALS CHEMISTRY, ROYAL SOCIETY OF CHEMISTRY, GB, vol. 9, no. 3, 1 mars 1999 (1999-03-01), pages 697-702, XP000853243, ISSN: 0959-9428, DOI: 10.1039/A807718E**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 906 526 B1

- **DATABASE REGISTRY [Online] CHEMICAL ABSTRACTS SERVICE, COLUMBUS, OHIO, US; 26 avril 2004 (2004-04-26), XP002694618, Database accession no. 676613-35-7 -& DATABASE chemcats [Online] CHEMICAL ABSTRACTS SERVICE, COLUMBUS, OHIO, US; 21 août 2012 (2012-08-21), XP002694619, Database accession no. 0033740586**
- **ROGER LAMARTINE ET AL: "Solid State Polycondensation of Precursors of Phenolic Resins", MOLECULAR CRYSTALS AND LIQUID CRYSTALS, vol. 134, no. 1, 1 avril 1986 (1986-04-01) , pages 219-236, XP55058975, ISSN: 0026-8941, DOI: 10.1080/00268948608079586**
- **TAKEHARU HAINO ET AL: "Synthesis and binding behavior of an artificial receptor based on "upper rim" functionalized calix[5]arene", TETRAHEDRON, vol. 54, no. 40, 1 octobre 1998 (1998-10-01), pages 12185-12196, XP55058970, ISSN: 0040-4020, DOI: 10.1016/S0040-4020(98)00750-9**
- **TYO SONE: "INCLUSION PROPERTIES OF ACYCLIC P-SUBSTITUTED PHENOL-FORMALDEHYDE OLIGOMERS", BULLETIN OF THE CHEMICAL SOCIETY OF JAPAN, vol. 62, no. 4, 1 janvier 1989 (1989-01-01), pages 1111-1116, XP55058967,**
- **CHOI SUNG-SEEN: "Properties of butyl rubber vulcanizates cured by different type resoles", POLYMER (KOREA), DEPARTMENT OF CHEMICAL ENGINEERING, SOGON UNIVERSITY; SEOUL, KOREA, vol. 7, no. 3, 1 janvier 1999 (1999-01-01) , pages 172-180, XP009168668, ISSN: 1225-5947**
- **BERND GARSKA ET AL: "Molecular Reinforcement of Transparent Materials With Acrylamide-Modified Calix[4]arene as a Crosslinker", MACROMOLECULAR MATERIALS AND ENGINEERING, vol. 297, no. 8, 10 février 2012 (2012-02-10), pages 785-789, XP55086943, ISSN: 1438-7492, DOI: 10.1002/mame.201100354**


Remarques:

Le dossier contient des informations techniques présentées postérieurement au dépôt de la demande et ne figurant pas dans le présent fascicule.

## Description

La présente invention concerne un procédé de préparation à hauts rendements de *p*-(R)calixarènes géants.

Les calixarènes ont été particulièrement étudiés ces dix dernières années en raison des possibilités immenses offertes par ces macrocycles aisément accessibles. Ces macrocycles possèdent une forme de calice dont la cavité est généralement inférieure ou égale à environ 1 nm.

Quelques unes des propriétés les plus étudiées incluent les phénomènes de reconnaissance des ions et/ou des molécules, les assemblages supramoléculaires ou bien encore la synthèse des nanoparticules, entre autres.

Dans la plupart des cas, ces études ont été effectuées avec les calixarènes fonctionnalisés par un *p*-(*t*-butyl) ou par des calixarènes obtenus par modification chimiques de ces *p*-(*t*-butyl)calixarènes, étant donné que la synthèse des *p*-(*t*-butyl)calixarènes est de loin la plus documentée depuis le travail pionnier de Gutsche et al.D. Gutsche, Calixarenes: An Introduction, The Royal Society of Chemistry, Cambridge, 2008**.** Z. Asfari, V. Böhmer, J. Harrowfield, J. Vicens, Calixarenes 2001, Kluwer, Dordrecht, the Netherlands, 2001**.**

Par conséquent, l’utilisation d’autres monomères de type phénols fonctionnalisés pour la synthèse des calixarènes est en grande partie inexplorée, même si des synthèses en une étape d’autres *p*-(alkyl)calixarènes sont connues (T. Patrick, P. Egan, J. Org. Chem. 1977, 42, 382 ; T. Patrick, P. Egan, J. Org. Chem. 1978, 43, 4280; Z. Asfari, J. Vicens, Tetrahedron Lett. 1988, 29, 2659 ; F. Vocanson, M. Perrin, R. Lamartine, J. Inclusion Phenom. Macrocyclic Chem. 2001, 39, 127 ; Jerry L. Atwood et al., Org. Lett. 1999, 1, 1523).

Les calixarènes *p*-substitués sont habituellement obtenus en mélange de *p*-calix[4, 5, 6, 7, 8]arènes par réaction d’un phénol *p*-substitué avec du paraformaldéhyde en présence d’au moins une base telle que la potasse ou la soude (B. Dahwan et al., Macromolec. Chem. 1987, 188, 921 ; C. D. Gutsche et al., Org. Synth. 1990, 68, 234 ; C. D. Gutsche et al. ; Org. Synth. 1990, 68, 238).

En général, les *p*-(alkyl)calix[8]arènes sont les calixarènes obtenus le plus facilement, car ils correspondent au produit cinétique de la réaction de polycondensation. Les *p*-(alkyl)calix[4]arènes correspondent, eux, au produit thermodynamique.

Dans la plupart des cas, le retrait ou la substitution de la position alkyle en para de la fonction hydroxyle de ces calixarènes, lorsqu’elle est possible, est au mieux délicate. Dans le cas le plus courant des *p*-(*t*-butyl)calixarènes, il s’agit en général d’un procédé en plusieurs étapes. Dans un premier temps, un réactif de type acide de Lewis est généralement associé à un phénol pour retirer le groupement *t*-butyle, puis dans un second temps, une autre fonction peut être introduite en lieu et place du groupement *t*-butyle en ayant au préalable halogéné la position. La réaction n’étant pas quantitative, la « déterbutylation » devient problématique, notamment lorsque le nombre d’unités du calixarène augmente. La présence de sous-produits limite le rendement, impose une étape de purification et restreint la pureté du produit. Ceci peut limiter le rendement final en produit entièrement déprotégé ainsi que son utilisation.

Les *p*-(benzyloxy)calixarènes représentent une alternative très intéressante aux *p*-(alkyl)calixarènes. Les motifs (benzyloxy)phénols sont en effet quantitativement réduits en phénols par hydrogénolyse catalysée par le palladium sur charbon, autorisant une post-fonctionnalisation aisée.

Peu de documents décrivent la synthèse de grands calixarènes, c’est-à-dire comportant de 9 à 20 motifs de répétition.

Ainsi le brevet CA 2,251,070 décrit l’obtention avec de faibles rendements des calixarènes comportant 9 et 11-14 motifs de répétition avec un procédé en deux étapes par réaction d’un *p*-(alkyl) ou *p*-(aralkyl)phénol en milieu aqueux en présence d’une base en quantité inférieure à 0,5 eq puis chauffage dans un solvant organique sans eau. Ce brevet ne décrit, en ce qui concerne les calixarènes comportant 9 et 11-14 motifs de répétition, que les *t*-(butyl)calixarènes.

L’article de Gutsche et al. (J. Am. Chem. Soc. 1999, 121, 4136) décrit l’obtention de *p*-(*t*-butyl)calix[9-20]arènes en milieu acide.

Garska et al. (Macromolecular Materials and Engineering 2012, 297, 785) décrit l’utilisation de calix[4]arènes dans le renfort de matériau.

Par contre, il n’existe pas de documents décrivant la synthèse de calixarènes géants, c’est-à-dire comportant plus de 20 motifs de répétition, notamment de 21 à plus de 200 motifs de répétition.

Par conséquent, l’obtention de calixarènes géants, qui sont de plus aisément fonctionnalisables sur la couronne haute par une large variété de groupements chimiques dans des conditions douces, est toujours une question ouverte et notamment l’obtention de calixarènes tels que les *p*-(R)-calixarènes géants avec de bons rendements.

L’un des objets de l’invention est la fabrication de *p*-(R)calixarènes géants avec des rendements cumulés supérieurs à 50%.

Un autre objet de l’invention est de fournir un procédé de synthèse en deux étapes permettant :

l’obtention d’un mélange *p*-(R)calixarènes géants ainsi qu’une procédure de purification en une étape par simple cristallisation du mélange de *p*-(R)calixarènes géants, permettant de les obtenir purs, en particuliers exempts des *p*-(R)calix[7, 8]arènes et d’oligomères phénoliques linéaires.

Un autre objet de la description est la fourniture d’un dimère phénolique substitué en position 4 par un groupe R.

Encore un autre objet est la fourniture d'un précurseur solide comprenant un mélange *de p*-(R)-calixarènes géants et d'oligomères phénoliques linéaires substitués en position 4 par un groupe R.

Encore un autre objet est l'utilisation de *p*-(R)calixarènes géants en mélange ou séparés pour la constitution d'un matériau ou dans le cadre du renfort des matériaux.

La présente description concerne l'utilisation d'au moins une base en solution aqueuse, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I) suivante :

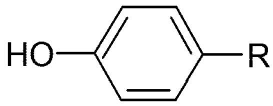

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther $-S-CH_2-Ph$ éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),
- un groupe $-NR_aR_b$, $R_a$ et $R_b$ représentant indépendamment l'un de l'autre un alkyle Ci-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

et une source de formaldéhyde aqueux,
en l'absence ou en présence de solvant organique, constituant ainsi un milieu réactionnel,
ladite base étant à une concentration totale comprise d'environ 0,01 à 1 équivalent, notamment comprise d'environ 0,1 équivalent à environ 0,4 équivalent, en particulier égale à 0,13 ou 0,4 ou 1 équivalent, par rapport audit au moins un phénol substitué en position 4 de formule (I),
ledit milieu réactionnel étant chauffé à une température comprise de 100 à 130°C, et, ledit milieu réactionnel étant éventuellement soumis ensuite à un traitement thermique en présence de moyens de transmission de chaleur, notamment un four ou un liquide,
pour la mise en oeuvre d'une réaction de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200.

La présente invention concerne l'utilisation d'au moins une base en solution aqueuse, avec au moins un phénol substitué en position 4 de formule (I) suivante :
dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NRaR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ linéaire,

- un groupe benzyloxy éventuellement substitué en ortho et/ou para et/ou méta par un ou plusieurs substituants choisis parmi un halogène, SR' et OR', R' représentant une chaine alkyle en C1-C20 linéaire ou ramifiée, un groupe cycloalkyle en $C_3$ à $C_{20}$, $NRaR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther $-S-CH_2-Ph$ éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),
- un groupe $-NR_aR_b$, $R_a$ et $R_b$ représentant indépendamment l'un de l'autre un alkyle $C_1$-$C_{20}$ ramifié ou linéaire,
- un groupe dibenzylamine de formule $-N(\text{benzyle})_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

et une source de formaldéhyde aqueux,
en l'absence ou en présence de solvant organique, constituant ainsi un milieu réactionnel,
ladite base étant à une concentration totale comprise de 0,01 à 1 équivalent, par rapport audit au moins un phénol substitué en position 4 de formule (I),
ledit milieu réactionnel étant chauffé à une température comprise de 100 à 130°C, et, ledit milieu réactionnel étant éventuellement soumis ensuite à un traitement thermique en présence de moyens de transmission de chaleur,
pour la mise en oeuvre d'une réaction de préparation d'un mélange de p-(R)calixarènes géants dont la taille est supérieure à 20.

Selon un autre aspect de la description, R représente également tout autre groupement capable d'induire en solution ou en phase solide une préorganisation des unités phénoliques entre elles, par exemple par un effet de micellisation, en conférant un caractère amphiphile marqué au monomère ou encore par interaction avec des groupes de même nature (par exemple des interactions d'empilement pi-pi (« stacking pi-pi »)) entre unités aromatiques étendues présentes sur le monomère, de type naphtalène, anthracène, pyrène, pérylène, ou encore par exemple par effet de coordination de cations métalliques sur des sites coordinants présents sur les monomères).

Les inventeurs ont trouvé de façon surprenante que la combinaison d'au moins une base en solution aqueuse, en particulier choisie parmi l'hydroxyde de baryum, l'hydroxyde de rubidium, l'hydroxyde de lithium, l'hydroxyde de césium, la potasse ou la soude, en l'absence ou en présence de solvant organique, avec au moins un phénol substitué en position 4 de formule (I) et d'une concentration en base totale comprise d'environ 0,01 à 1 équivalent, notamment comprise d'environ 0,1 équivalent à environ 0,4 équivalent, en particulier égale à 0,15 ou 0,4 équivalent, ou 1 équivalent par rapport audit au moins un phénol substitué en position 4 de formule (I), permettait d'obtenir un mélange *p*-(benzyloxy)calix[21-220]arènes de façon majoritaire ou purifiée, avec de bons rendements.

Ce résultat est d'autant plus inattendu que :

- l'utilisation d'une base telle que la potasse, la soude ou l'hydroxyde de césium à une concentration équivalente à celle de l'invention dans un solvant organique tel que le xylène, avec un *p*-(benzyloxy)phénol, conduit majoritairement avec de bons rendements à un mélange de *p*-(benzyloxy)calix[6-8]arènes,
- l'utilisation de l'hydroxyde de baryum en solution aqueuse à une concentration équivalente à celle de l'invention en milieu organique ou à une concentration supérieure à 0,5 eq en milieu organique ne permet d'accéder ni aux petits calixarènes (c'est-à-dire des calixarènes dont la taille est inférieure à neuf motifs) et notamment les calix[6-8] arènes, ni aux grands calixarènes, et,
- l'utilisation d'une base telle que la potasse, la soude ou l'hydroxyde de césium à une concentration équivalente à celle de l'invention en l'absence de solvant organique, avec un p-(*tert*-butyl)phénol ne permet pas d'obtenir des calixarènes géants.

Ce résultat inattendu peut s'expliquer par la présence concomitante d'un groupement hydrophobe faiblement encombrée (R) en para de l'hydroxyde du phénol rendant ainsi le composé de formule (I) amphiphile, de telle sorte que les monomères peuvent s'orienter voire s'organiser les uns par rapport aux autres en solution aqueuse. La présence d'un atome d'oxygène (potentiellement coordinant) en para du groupement (-OH) du phénol peut aussi induire des effets de coordination sur les cations métalliques présents dans le milieu réactionnel. De tels effets de coordination sont absents dans le cas des *p*-(alkyl)phénols, et sont donc susceptibles de jouer un rôle dans le comportement surprenant observé par les inventeurs.

Le terme base désigne toute base soluble en milieu aqueux de type hydroxyde métallique, amine tertiaire, carbonate, sulfate, carboxylate, par exemple.

Il peut également désigner l'utilisation d'une base organique type amine en combinaison avec un sel métallique (CsI par exemple).

L'expression « source de formaldéhyde aqueux » signifie que du formaldéhyde en solution aqueuse tel que de la formaline ou du formol peut être utilisé.

L'expression « en solution aqueuse » dans toute la description signifie que la base est dissoute dans un milieu majoritairement constitué d'eau.

Avantageusement, l'expression « en solution aqueuse » désigne exclusivement de l'eau.

L'expression « en l'absence ou en présence de solvant organique » signifie que le milieu réactionnel (qui comprend de l'eau) est respectivement dépourvu ou pourvu de solvants qui sont des composés organiques qui contiennent des atomes de carbone, la base en tant que telle, le formaldéhyde ou la source de formaldéhyde et le phénol n'étant pas non plus considérés comme solvant organique.

L'expression « milieu réactionnel » signifie le mélange des différents composants, comprenant la ou les base(s), le ou les phénol(s) substitué(s) en position 4 de formule (I), la source de formaldéhyde, le tout étant en solution aqueuse, en l'absence de solvant organique, ou dans un milieu eau-solvant organique, en présence de solvant organique.

Lorsque ledit mélange vient d'être constitué, il est dénommé milieu réactionnel initial, c'est-à-dire un milieu réactionnel dans lequel la réaction de préparation des *p*-(R)calixarènes n'a pas encore été effectuée, en particulier avant tout chauffage dudit mélange des différents composés.

Les *p*-(R)calixarènes géants possèdent la structure suivante :

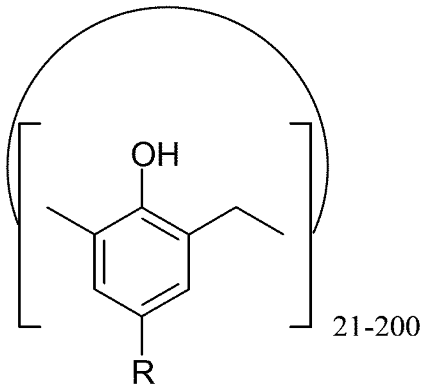

et sont donc constitués :

- d'un mélange d'au moins deux à plus de 180 de *p*-(R)calixarènes géants choisis parmi le groupe de *p*-(R)calix[21-220] arènes quelle que soit la proportion de chaque *p*-(R)calixarène présent dans le mélange et peuvent également être dénommés dans toute la description «p-(R)calixarènes géants»,
- ou de l'un des *p*-(R)calix[21-200]arènes, purifié ou pur.

Les *p*-(R)calix[9-20]arènes sont dénommés grands calixarènes et ne font pas partie de la portée de l'invention en tant que tels. Il est cependant possible qu'il soient obtenus en quantité minoritaire en mélange avec les *p*-(R)calix[21-220]arènes.

Dans toute la description, le terme « purifié » ou « pur » désigne un composé ayant une pureté supérieure ou égale à 90%.

Le mélange de *p*-(R)calix[21-220]arènes ou les *p*-(R)calixarènes purifiés sont en règle générale obtenus sous forme neutralisée après neutralisation de la base présente dans le milieu réactionnel final.

Par l'expression « sous forme neutralisée », il faut comprendre un *p*-(R)calixarène purifié dont tous les hydroxyles des groupements phénols ont été neutralisés, c'est-à-dire sont sous forme OH, non salifiée.

Sans neutralisation de la base, le mélange de *p*-(R)calix[21-220]arènes ou les *p*-(R)calixarènes purifiés sont obtenus sous forme potentiellement salifiée (selon la concentration en base et le nombre de motifs du calixarène), c'est-à-dire qu'au moins l'un des groupements phénol est sous forme salifiée avec le cation métallique provenant de la base, en particulier le mélange de *p*-(R)calixarènes ou les *p*-(R)calixarènes purifiés sont obtenus sous forme mono-salifiée, c'est-à-dire qu'un seul des groupements phénol libres est sous forme salifiée avec le cation métallique provenant de la base.

Le terme alkyle en $C_1$-$C_{20}$ utilisé dans toute la description désigne un groupe alkyle linéaire ou ramifié comprenant 1 à 20 atomes de carbones.

Par groupe alkyle linéaire en $C_1$ à $C_{20}$ il faut entendre: un groupe méthyle, un éthyle, un propyle, un butyle, un pentyle, un héxyle, un heptyle, un octyle un nonyle, un décyle, un undécyle, un dodécyle, le tridécyle, tétradécyle, le pentadécyle, l'hexadécyle, l'heptadécyle, l'octadécyle, le nonadécyle et l'eicosyle, ainsi que certains de leurs isomères capables d'interagir avec des groupes de même nature (effets de « packing » de type van der Waals) et/ou d'induire des préorganisations en solution et en phase solide.

Par groupe alkyle ramifié, il faut comprendre un groupe alkyle tel que défini ci-dessus comprenant des substituants choisis parmi la liste des groupes alkyles linéaires définie ci-dessus, lesdits groupes alkyles linéaires étant également susceptibles d'être ramifiés.

Par groupe cycloalkyle en $C_3$ à $C_{20}$ il faut comprendre un groupe cyclopropyle, cyclobutyle, cyclopentyle, cyclohéxyle, cycloheptyle, cyclooctyle, cyclononyle, cyclodécyle, cycloundécyle, cyclododécyle, cyclotridécyle, cyclotétradécyle, cyclopentadécyle, cyclohexadécyle, cycloheptadécyle, cyclooctadécyle, cyclononadécyle et cycloeicosyle.

De tels groupes cycloalkyles peuvent être eux-mêmes substitués par un groupe alkyle linéaire ou ramifié tel que défini ci-dessus.

Ledit milieu réactionnel est chauffé à une température comprise de 100 à 130°C durant 30 minutes à 5 heures.

L'expression « et éventuellement soumis ensuite à un traitement thermique en présence de moyens de transmission de chaleur » signifie qu'après ledit chauffage à une température comprise de 100 à 130°C, au cours duquel l'eau est éventuellement éliminée, des moyens de transmission de chaleur sont appliqués au milieu réactionnel, puis ledit milieu réactionnel est chauffé à nouveau.

Les moyens de transmission de chaleur sont en particulier un four ou un solvant, en particulier un solvant chauffé à une température comprise de 120°C à 140°C, par exemple un solvant dont la température d'ébullition est comprise de 120°C à 140°C.

Par solvant, on entend :

- un solvant, en particulier un solvant organique, dans lequel les composés organiques du milieu réactionnel sont au moins partiellement solubles, par exemple un solvant aromatique, de point d'ébullition supérieur à 50°C, notamment supérieur à 100°C,
  ou
- un liquide dans lequel les composés organiques du milieu réactionnel ne sont pas solubles, par exemple un alcane linéaire ou ramifié, en particulier l'octane, ou une huile de silicone, de point d'ébullition supérieur à 50°C, notamment supérieur à 100°C.

Par « solvant dans lequel les composés organiques du milieu réactionnel sont au moins partiellement solubles », on entend un solvant pouvant éventuellement, selon sa nature, solubiliser certains des constituants dudit milieu réactionnel, les disperser (pour former une suspension colloïdale) ou faire passer tout ou partie d'entre eux à l'état de gel.

Ainsi, lorsque les moyens de transmission de chaleur sont par exemple un four ou un solvant dans lequel les composés organiques du milieu réactionnel ne sont pas solubles, ledit traitement thermique se fait en phase solide, de préférence sans agitation.

Dans un mode de réalisation avantageux, le milieu réactionnel est dépourvu de solvant organique, l'eau est éliminée au cours dudit chauffage à une température comprise de 100 à 130°C, et ledit traitement thermique est réalisé en présence d'un solvant dans lequel les composés organiques du milieu réactionnel sont au moins partiellement solubles.

Dans un autre mode de réalisation avantageux, le milieu réactionnel est dépourvu de solvant organique, l'eau est éliminée au cours dudit chauffage à une température comprise de 100 à 130°C, et ledit traitement thermique est réalisé en présence d'un solvant dans lequel les composés organiques du milieu réactionnel ne sont pas solubles.

Dans un autre mode de réalisation avantageux, le milieu réactionnel est dépourvu de solvant organique, l'eau n'est pas éliminée au cours dudit chauffage à une température comprise de 100 à 130°C, et ledit traitement thermique est réalisé en présence d'un solvant dans lequel les composés organiques du milieu réactionnel sont au moins partiellement solubles.

Dans un autre mode de réalisation avantageux, le milieu réactionnel est dépourvu de solvant organique, l'eau n'est pas éliminée au cours dudit chauffage à une température comprise de 100 à 130°C, et ledit traitement thermique est réalisé en présence d'un solvant dans lequel les composés organiques du milieu réactionnel ne sont pas solubles.

Dans un autre mode de réalisation avantageux, le milieu réactionnel comprend, outre de l'eau, un solvant organique, l'eau n'est pas éliminée au cours du chauffage, et ledit milieu réactionnel n'est pas soumis audit traitement thermique supplémentaire.

Dans un autre mode de réalisation avantageux, le milieu réactionnel comprend, outre de l'eau, un solvant organique, l'eau n'est pas éliminée au cours du chauffage, et ledit milieu réactionnel est soumis audit traitement thermique supplémentaire, l'eau étant en particulier éliminée au cours dudit traitement thermique.

Le système eau-solvant organique est susceptible de favoriser la solubilisation de l'ensemble des composés organiques et inorganiques, au début de la réaction. Cela permet donc de maintenir les constituants du milieu réactionnel plus longtemps en solution. Ceci se traduit par une meilleure consommation des réactifs et donc permet 1) un meilleur rendement, 2) moins de sous-produits (produits de départ, intermédiaires réactionnels...) et rend donc la purification plus facile.

La présente description concerne également l'utilisation d'au moins une base en solution aqueuse, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I) suivante :

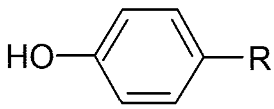
(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther $-S-CH_2-Ph$ éventuellement substitué en ortho et/ou para et/ou méta par un ou plusieurs substituants choisis parmi un halogène, SR' et OR', NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),

et une source de formaldéhyde aqueux,
en l'absence de solvant organique, constituant ainsi un milieu réactionnel,
ladite base étant à une concentration totale comprise d'environ 0,01 à 1 équivalent, notamment comprise d'environ 0,1 équivalent à environ 0,4 équivalent, en particulier égale à 0,13 ou 0,4 ou 1 équivalent, par rapport audit au moins un phénol substitué en position 4 de formule (I),
ledit milieu réactionnel étant chauffé à une température comprise de 100 à 130°C, et éventuellement soumis ensuite à un traitement thermique en présence d'un solvant organique, pour la mise en oeuvre d'une réaction de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200.

L'expression « en l'absence de solvant organique » signifie que le milieu réactionnel est dépourvu de solvants contenant des atomes de carbone, la base en tant que telle, le formaldéhyde ou la source de formaldéhyde et le phénol n'étant pas non plus considérés comme solvant organique.

L'expression « milieu réactionnel » signifie le mélange des différents composants, comprenant la ou les base(s), le ou les phénol(s) substitué(s) en position 4 de formule (I), la source de formaldéhyde, le tout étant en solution aqueuse.

L'expression « et éventuellement soumis ensuite à un traitement thermique en présence d'un solvant organique » signifie qu'après ledit chauffage à une température comprise de 100 à 130°C, un solvant organique tel qu'un alcane linéaire ou ramifié, un solvant aromatique, une huile de silicone de point d'ébullition supérieur à 50°C, notamment supérieur à 100°C est introduit, puis le milieu réactionnel chauffé à nouveau.

Dans un mode de réalisation avantageux, la présente invention concerne l'utilisation d'au moins une base en solution aqueuse avec au moins un phénol substitué en position 4 de formule (I), telle que définie ci-dessus, dans laquelle R est choisi parmi :

- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, Ra et Rb représentant indépendamment l'un

de l'autre un alkyle $C_1$-$C_{20}$ ramifié ou linéaire,

- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),
- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

et une source de formaldéhyde aqueux,
en l'absence ou en présence de solvant organique, constituant ainsi un milieu réactionnel,
ladite base étant à une concentration totale comprise d'environ 0,01 à 1 équivalent, notamment comprise d'environ 0,1 équivalent à environ 0,4 équivalent, en particulier égale à 0,15 ou 0,4 ou 1 équivalent, par rapport audit au moins un phénol substitué en position 4 de formule (I),
ledit milieu réactionnel étant chauffé à une température comprise de 100 à 130°C, et, ledit milieu réactionnel étant éventuellement soumis ensuite à un traitement thermique en présence de moyens de transmission de chaleur, notamment un four ou un solvant,
pour la mise en oeuvre d'une réaction de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100.

Dans un mode de réalisation avantageux, la présente invention concerne l'utilisation d'au moins une base en solution aqueuse avec au moins un phénol substitué en position 4 de formule (I), telle que définie ci-dessus, dans laquelle R est choisi parmi :

- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),

et une source de formaldéhyde aqueux,
en l'absence de solvant organique, constituant ainsi un milieu réactionnel,
ladite base étant à une concentration totale comprise d'environ 0,01 à 1 équivalent, notamment comprise d'environ 0,1 équivalent à environ 0,4 équivalent, en particulier égale à 0,15 ou 0,4 ou 1 équivalent, par rapport audit au moins un phénol substitué en position 4 de formule (I),
ledit milieu réactionnel étant chauffé à une température comprise de 100 à 130°C, et éventuellement soumis ensuite à un traitement thermique en présence d'un solvant organique,
pour la mise en oeuvre d'une réaction de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100.

Dans ce qui précède, R peut également représenter :

- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire.

Dans ce mode de réalisation, le phénol en position 4 est substitué par un O-alkyle en $C_1$-$C_{20}$ (alkyloxy) ou un O-benzyle (benzyloxy), substitué ou non, mais ne peut avoir un alkyle ou un benzyle directement lié en position 4.

Dans un mode de réalisation avantageux, la présente description concerne l'utilisation d'au moins une base en solution aqueuse avec au moins un phénol substitué en position 4 de formule (I), telle que définie ci-dessus, dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_5$-$C_{20}$ ramifié ou linéaire,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_5$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,

  - un groupe benzylthioéther -S-$CH_2$-Ph éventuellement substitué en ortho et/ou para et/ou méta par un ou plusieurs substituants choisis parmi un halogène, SR' et OR', NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
  - un groupe alkylthioéther en $C_5$-$C_{20}$ ramifié ou linéaire, de formule -S-($C_5$-$C_{20}$-alkyle).

Dans ce qui précède, R peut également représenter :

- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire.

Dans ce mode de réalisation, le phénol de formule (I) ne peut donc pas être substitué en position 4 par un méthyle, éthyle, isopropyle, propyle, butyle, isobutyle, *sec*-butyle ou *t*-butyle ou par un méthyloxy, éthyloxy, isopropyloxy, propyloxy, butyloxy, isobutyloxy, sec-butyloxy ou t-butyloxy.

Dans un mode de réalisation avantageux, la présente invention concerne l'utilisation d'au moins une base en solution aqueuse avec au moins un phénol substitué en position 4 de formule (I), telle que définie ci-dessus, dans laquelle de l'eau est produite lors de la réaction, ladite eau étant éliminée du milieu réactionnel durant ledit chauffage à une température comprise de 100 à 130°C et dans laquelle un précurseur solide sous forme de résine dure cassante, éventuellement isolé, est obtenu.

Lors de la réaction de condensation du phénol sur le formaldéhyde en présence de la base, de l'eau est également formée au cours de la réaction.

L'eau formée dans le milieu réactionnel durant la réaction ainsi qu'une partie de l'eau présente initialement est éliminée par des techniques bien connues de l'homme du métier, par exemple au moyen d'un appareil de Dean-Stark ou par balayage du milieu réactionnel durant la réaction au moyen d'un gaz inerte tel que l'azote ou l'argon.

Il est bien entendu que toute l'eau présente initialement n'est pas éliminée en totalité.

Dans ce cas, la proportion d'eau présente après obtention de la résine est de moins de 5% en poids.

L'élimination de l'eau conduit à la solidification complète du milieu, c'est-à-dire la prise en masse du milieu et à la formation d'un précurseur solide qui comprend essentiellement un mélange de *p*-(R)calixarènes géants dont la distribution en taille et la pureté dépendent de R et de la nature de la base. Le précurseur comprend de plus une proportion variable d'oligomères linéaires dont des dimères linéaires précurseurs des *p*-(R)calixarènes géants.

Le précurseur étant sous forme solide, il peut être aisément isolé par simple filtration du milieu réactionnel.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus,

dans laquelle ledit précurseur solide obtenu sous forme de résine dure cassante est isolé du milieu réactionnel, et dépourvu de traitement thermique,

pour la préparation d'un mélange de *p*-(R)calixarènes géants dont la taille est comprise de 21 à 50 unités phénoliques.

Dans ce mode de réalisation, le précurseur solide obtenu est isolé mais n'est pas soumis audit traitement thermique en présence de moyens de transmission de chaleur.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus,

dans laquelle ledit précurseur solide obtenu sous forme de résine dure cassante est isolé du milieu réactionnel, et dépourvu de traitement thermique en présence d'un solvant organique, pour la préparation d'un mélange de *p*-(R)calixarènes géants dont la taille est comprise de 21 à 50 unités phénoliques.

Dans ce mode de réalisation, le précurseur solide obtenu est isolé mais n'est pas soumis audit traitement thermique en présence d'un solvant organique.

Le produit obtenu dans la résine dure cassante est donc constitué essentiellement de *p*-(R)calixarènes géants et ne nécessite donc pas de traitement thermique pour compléter la formation des macrocycles (*p*-(R)calixarènes géants).

En fonction de R, de la base utilisée (ainsi que de sa concentration), et du temps, il peut contenir cependant également, en proportion variable, des oligomères linéaires issus de la condensation du phénol substitué en position 4 de formule (I) et nécessite alors une étape de purification.

Dans un mode de réalisation avantageux, ledit chauffage à une température comprise de 100 à 130°C est effectué durant 30 minutes à 5 heures, avantageusement durant 1h à 5h, plus avantageusement durant 2 h à 5h.

Avantageusement, ledit chauffage à une température comprise de 100 à 130°C est effectué notamment durant 3h.

Avantageusement, ledit chauffage à une température comprise de 100 à 130°C est effectué notamment durant 4h.

Avantageusement, ledit chauffage à une température comprise de 100 à 130°C est effectué notamment durant 5h.

La durée dudit chauffage dépend de la concentration en base. A faible concentration en base, par exemple 0,1 équivalent par rapport au phénol, la solidification complète sous forme de résine dure cassante est lente, par exemple 3h, alors qu'à forte concentration en base, par exemple de 0,4 à 1 équivalent par rapport au phénol, la solidification est plus rapide, par exemple 1h.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus, dans laquelle ledit précurseur solide obtenu sous forme de résine dure cassante est isolé du milieu réactionnel, et dépourvu de traitement thermique, tel que défini ci-dessus,

dans laquelle le phénol substitué en position 4 de formule (I) est le 4-octyloxyphénol ou le 4-octylphénol.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus, dans laquelle ledit précurseur solide obtenu sous forme de résine dure cassante est isolé du milieu réactionnel, et dépourvu de traitement thermique, tel que défini ci-dessus,

dans laquelle le phénol substitué en position 4 de formule (I) est le 4-octyloxyphénol, le 4-méthoxyphénol, le 4-benzyloxyphénol, le 4-dibenzylaminophénol, le 4-méthylphénol, le 4-éthylphénol ou le 4-benzylphénol.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus, dans laquelle ledit précurseur solide obtenu sous forme de résine dure cassante est isolé du milieu réactionnel, et dépourvu de traitement thermique en présence d'un solvant organique, tel que défini ci-dessus,

dans laquelle le phénol substitué en position 4 de formule (I) est le 4-octyloxyphénol ou le 4-octylphénol.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus, dans laquelle ledit précurseur solide obtenu sous forme de résine dure cassante est isolé du milieu réactionnel, et dépourvu de traitement thermique en présence d'un solvant organique, tel que défini ci-dessus,

dans laquelle le phénol substitué en position 4 de formule (I) est 4-octyloxyphénol, le 4-méthoxyphénol, le 4-benzyloxyphénol, le 4-dibenzylaminophénol, le 4-méthylphénol, le 4-éthylphénol ou le 4-benzylphénol.

Dans ces modes de réalisation, la taille moyenne des *p*-(octyloxy)calix[21-50]arènes géants obtenus est de

35, déterminée par la gaussienne centrée mesurée en chromatographie par perméation de gel (GPC), avec une taille moyenne de 54 motifs phénoliques.

Les *p*-(octyloxy)calix[21-50]arènes géants ont l'avantage d'être plus stables en milieu acide et en milieu réducteur que les *p*-(benzyloxy)calix[21-50]arènes.

Les *p*-(octyloxy)calix[21-50]arènes et les *p*-(octyl)calix[21-50]arènes géants présentent un caractère amphiphile plus marqué, potentiellement intéressant pour la réalisation d'architectures supramoléculaires (micelles, vésicules...). Ceci est démontré par le fait que ces calixarènes géants octyloxy sont parfaitement (et très rapidement) dispersés dans le chloroforme sous la forme d'agrégats de grande taille (signaux RMN très élargis et présence d'un pic en diffusion de lumière à 200nm). Ce comportement n'est pas observé avec les *p*-(benzyloxy)calixarènes.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus, dans laquelle ledit précurseur solide obtenu sous forme de résine dure cassante est isolé du milieu réactionnel, et dépourvu de traitement thermique, dans laquelle le phénol substitué en position 4 de formule (I) est le 4-octyloxyphénol ou le 4-octylphénol, tel que défini ci-dessus,
dans laquelle la base utilisée est l'hydroxyde de baryum.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus, dans laquelle ledit précurseur solide obtenu sous forme de résine dure cassante est isolé du milieu réactionnel, et dépourvu de traitement thermique, dans laquelle le phénol substitué en position 4 de formule (I) est 4-octyloxyphénol, le 4-méthoxyphénol, le 4-benzyloxyphénol, le 4-dibenzylaminophénol, le 4-méthylphénol, le 4-éthylphénol ou le 4-benzylphénol, tel que défini ci-dessus,
dans laquelle la base utilisée est l'hydroxyde de baryum.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus, dans laquelle ledit précurseur solide obtenu sous forme de résine dure cassante est isolé du milieu réactionnel, et dépourvu de traitement thermique en présence d'un solvant organique, dans laquelle le phénol substitué en position 4 de formule (I) est le 4-octyloxyphénol ou le 4-octylphénol, tel que défini ci-dessus,
dans laquelle la base utilisée est l'hydroxyde de baryum.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus, dans laquelle ledit précurseur solide obtenu sous forme de résine dure cassante est isolé du milieu réactionnel, et dépourvu de traitement thermique en présence d'un solvant organique, dans laquelle le phénol substitué en position 4 de formule (I) est 4-octyloxyphénol, le 4-méthoxyphénol, le 4-benzyloxyphénol, le 4-dibenzylaminophénol, le 4-méthylphénol, le 4-éthylphénol ou le 4-benzylphénol, tel que défini ci-dessus,
dans laquelle la base utilisée est l'hydroxyde de baryum.

Dans un mode de réalisation avantageux, la présente invention concerne l'utilisation d'au moins une base en solution aqueuse avec au moins un phénol substitué en position 4 de formule (I) dans laquelle de l'eau est produite lors de la réaction, ladite eau étant éliminée du milieu réactionnel durant ledit chauffage à une température comprise de 100 à 130°C, telle que définie ci-dessus, dans laquelle ledit précurseur solide sous forme de résine dure cassante est précédé de la formation d'un dimère phénolique, éventuellement isolé.

En fonction de la durée du chauffage à une température comprise de 100 à 130°C du phénol substitué en position 4 de formule (I) avec ladite base et le formaldéhyde aqueux, avec élimination de l'eau formée, des oligomères linéaires de formule (III) suivante, de taille variable correspondant à la condensation du phénol substitué en position 4 de formule (I) avec le formaldéhyde avant cyclisation en *p*-(R)calixarènes géants sont tout d'abord obtenus :

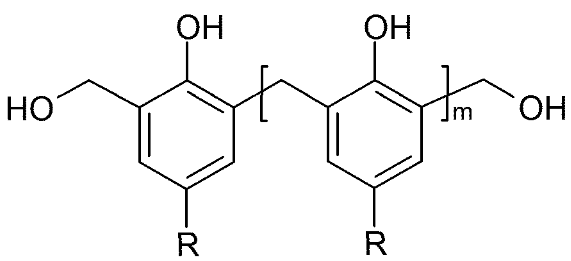

(III)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther $-S-CH_2-Ph$ éventuellement substitué en ortho et/ou para et/ou méta par un ou plusieurs substituants choisis parmi un halogène, SR' et OR', NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),

et m est un entier compris de 2 à plus de 200.

Dans ce qui précède, R peut également représenter :

- un groupe $-NR_aR_b$, $R_a$ et $R_b$ représentant indépendamment l'un de l'autre un alkyle $C_1$-$C_{20}$ ramifié ou linéaire,
- un groupe dibenzylamine de formule $-N(benzyle)_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire.

Avantageusement, le chauffage à une température comprise de 100 à 130°C est effectué d'environ 10 minutes à environ 25 minutes pour obtenir les oligomères.

Plus avantageusement, le chauffage à une température comprise de 100 à 130°C est effectué pendant environ 20 minutes pour obtenir les oligomères.

Ces oligomères sont ensuite transformés en dimère correspondant de formule (II) suivante, si le chauffage du milieu réactionnel initial est effectué durant 30 minutes à 2h :

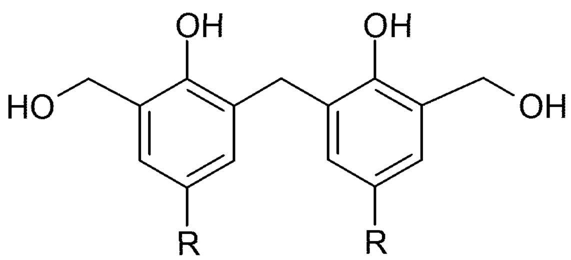

(II)

où R est tel que décrit précédemment.

Ledit dimère peut alors être isolé ou non.

Ledit dimère non isolé soit se réarrange pour former des oligomères de taille variée ou se combine avec un autre dimère ou des oligomères résiduels pour former le matériau solide sous forme de résine comprenant environ 50% à 90% de *p*-(R)calixarènes géants (en fonction du groupement alkyle ou alkyloxy en para et de la base) lorsque le chauffage à une température comprise de 100 à 130°C est poursuivi durant 1h à 3h additionnelles après formation du dimère.

Avantageusement, le chauffage additionnel est effectué durant environ 1h.

Avantageusement, le chauffage additionnel est effectué durant environ 2h.

Avantageusement, le chauffage additionnel est effectué durant environ 3h.

Lorsque le temps de chauffage à une température comprise de 100 à 130°C est contrôlé, en particulier lorsque la base est LiOH, entre 30 minutes et 5 heures, le dimère peut alors être isolé par filtration et ensuite remis à réagir dans un milieu aqueux comprenant au moins une base en solution aqueuse pour la poursuite du chauffage à une

température comprise de 100 à 130°C durant 1 à 3h.

Avantageusement, la poursuite du chauffage additionnel est effectuée durant environ 1h.

Avantageusement, la poursuite du chauffage additionnel est effectuée durant environ 2h.

Avantageusement, la poursuite du chauffage additionnel est effectuée durant environ 3h.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant éliminée du milieu réactionnel durant ledit chauffage à une température comprise de 100 à 130°C et un précurseur solide sous forme de résine dure cassante, éventuellement isolé, est obtenu, ledit précurseur solide sous forme de résine dure cassante étant précédé de la formation d'un dimère phénolique, éventuellement isolé, tel que défini ci-dessus,

dans laquelle ledit précurseur solide sous forme de résine dure cassante ou ledit dimère phénolique, éventuellement isolé du milieu réactionnel, est mis en présence d'un moyen de transmission de chaleur, en particulier un solvant dans lequel ledit précurseur solide sous forme de résine dure cassante et ledit dimère phénolique sont insolubles, notamment dans un alcane linéaire ou ramifié de point d'ébullition supérieur à 50°C, notamment supérieur à 100°C, en particulier dans l'octane ou une huile de silicone, et est soumis audit traitement thermique,

pour la préparation d'un mélange de *p*-(R)calixarènes géants dont la taille est comprise de 21 à 50 unités phénoliques, la taille moyenne des *p*-(R)calix[21-50]arènes géants obtenus est de 28, déterminée par la gaussienne centrée mesurée par des expériences de diffusion de la lumière.

Le traitement thermique est en particulier effectué au reflux dudit solvant, avec ou sans agitation, préférentiellement sans agitation du milieu réactionnel, et a notamment pour effet d'une part de compléter la formation des macrocycles, notamment ceux compris dans le précurseur solide sous forme de résine, mais également d'augmenter la taille des macrocycles obtenus aussi bien à partir du précurseur solide sous forme de résine que dudit dimère.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant éliminée du milieu réactionnel durant ledit chauffage à une température comprise de 100 à 130°C et un précurseur solide sous forme de résine dure cassante, éventuellement isolé, est obtenu, ledit précurseur solide sous forme de résine dure cassante étant précédé de la formation d'un dimère phénolique, éventuellement isolé, tel que défini ci-dessus,

dans laquelle ledit précurseur solide sous forme de résine dure cassante ou ledit dimère phénolique, éventuellement isolé du milieu réactionnel, est mis en présence d'un solvant organique dans lequel ledit précurseur solide sous forme de résine dure cassante et ledit dimère phénolique sont insolubles, notamment dans un alcane linéaire ou ramifié de point d'ébullition supérieur à 50°C, notamment supérieur à 100°C, en particulier dans l'octane ou une huile de silicone, et est soumis audit traitement thermique,

pour la préparation d'un mélange de *p*-(R)calixarènes géants dont la taille est comprise de 21 à 50 unités phénoliques, la taille des *p*-(R)calix[21-50]arènes géants obtenus est de 28, déterminée par la gaussienne centrée observée en GPC et confirmée par des expériences de diffusion de la lumière.

Cette taille de 28 correspond donc à la taille au pic, c'est-à-dire la taille la plus représentée dans l'échantillon.

Le traitement thermique est effectué au reflux dudit solvant organique, avec ou sans agitation, préférentiellement sans agitation du milieu réactionnel, et a notamment pour effet d'une part de compléter la formation des macrocycles, notamment ceux compris dans le précurseur solide sous forme de résine, mais également d'augmenter la taille des macrocycles obtenus aussi bien à partir du précurseur solide sous forme de résine que dudit dimère.

Les calixarènes géants obtenus comprennent cependant une proportion de calix[7]arènes et/ou de calix[8]arènes variable en fonction de R.

Plus le précurseur solide sous forme de résine ou le dimère est insoluble dans le solvant organique et plus la proportion de calixarènes géants augmente.

Lorsque le traitement thermique est effectué avec agitation, les macrocycles obtenus sont de pureté supérieure à celle de ceux obtenus en l'absence d'agitation ; cependant, la proportion de petits calixarènes et notamment de calix[7]arènes et/ou de calix[8]arènes est supérieure à celle susceptible d'être obtenue en l'absence d'agitation.

En revanche, lorsque le traitement thermique est effectué sans agitation, le rendement en calixarènes géants est plus important par rapport à celui obtenu avec agitation, la proportion de petits calixarènes et notamment de calix[7]arènes et/ou de calix[8]arènes étant inférieure à celle susceptible d'être obtenue avec agitation.

Cependant, les macrocycles obtenus sont de pureté inférieure à celle de ceux obtenus avec agitation.

La durée du traitement thermique doit également être contrôlée et doit être comprise de 3 à 8h en fonction de R et de la base utilisée.

Au-delà de 8h, la proportion de petits calixarènes obtenus augmente jusqu'à n'obtenir que des petits calixarènes, les calixarènes géants disparaissant en se dissociant au profit desdits petits calixarènes.

La cinétique des mécanismes de formation et de dissociation des calixarènes géants est directement liée à :

- la taille du contre-ion de la base ; l'apparition des petits calixarènes est plus rapide avec les petits alcalins tels que le lithium et le sodium, plus lente avec les gros alcalins et avec l'hydroxyde de baryum ;
- la succession réactionnelle menant des oligomères vers les dimères, puis les calixarènes géants et enfin les petits calixèrenes est d'autant plus rapide que la concentration en base est faible, c'est-à-dire comprise de 0,1 à 0,4 équivalent.

Avantageusement, le traitement thermique est compris d'environ 3 à 4h.

Avantageusement, lorsque le traitement thermique est compris de 3 à 4h, la base utilisée est LiOH, KOH, NaOH, CsOH ou RbOH.

Avantageusement, le traitement thermique est compris d'environ 6 à 8h, en particulier 7h.

Avantageusement, lorsque le traitement thermique est compris d'environ 6 à 8h, en particulier 7h, la base utilisée est $Ba(OH)_2$.

En fonction du solvant utilisé pour le traitement thermique, la taille des macrocycles obtenue peut être modulée.

Plus la température du milieu réactionnel est basse plus la taille des macrocycles obtenus diminue.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant éliminée du milieu réactionnel durant ledit chauffage à une température comprise de 100 à 130°C et un précurseur solide sous forme de résine dure cassante, éventuellement isolé, est obtenu, ledit précurseur solide sous forme de résine dure cassante étant précédé de la formation d'un dimère phénolique, éventuellement isolé, telle que définie ci-dessus, dans laquelle ledit précurseur solide sous forme de résine dure cassante ou ledit dimère phénolique, éventuellement isolé du milieu réactionnel, est mis en présence d'un solvant organique dans lequel ledit précurseur solide sous forme de résine dure cassante ou ledit dimère phénolique sont au moins partiellement solubles, en particulier dans le xylène ou le toluène, le DMSO ou le DMF, et est soumis audit traitement thermique,
pour la préparation d'un mélange de *p*-(R)calixarènes géants dont la taille est comprise de 21 à environ 212 unités phénoliques.

Le traitement thermique est effectué au reflux dudit solvant organique, avec ou sans agitation, préférentiellement sans agitation du milieu réactionnel.

Lorsque le précurseur solide sous forme de résine ou le dimère est dissout pour l'étape de traitement thermique, les macrocycles obtenus sont de pureté supérieure par rapport à ceux obtenus en l'absence de dissolution du précurseur solide sous forme de résine ou du dimère dans le solvant organique ; cependant, la proportion de petits calixarènes et notamment de calix[7]arènes et/ou de calix[8]arènes est supérieure.

La durée du traitement thermique doit également être contrôlée et doit être comprise de 3 à 8h en fonction de R et de la base utilisée.

Au-delà de 8h, la proportion de petits calixarènes obtenus augmente jusqu'à n'obtenir que des petits calixarènes, les calixarènes géants disparaissant en se dissociant au profit desdits petits calixarènes.

Avantageusement, le traitement thermique est compris d'environ 3 à 4h.

Avantageusement, lorsque le traitement thermique est compris de 3 à 4h, la base utilisée est LiOH, KOH, NaOH, CsOH ou RbOH.

Avantageusement, le traitement thermique est compris d'environ 6 à 8h, en particulier 7h.

Avantageusement, lorsque le traitement thermique est compris d'environ 6 à 8h, en particulier 7h, la base utilisée est $Ba(OH)_2$.

Dans le cas où ledit précurseur solide sous forme de résine dure cassante ou ledit dimère phénolique est mis en présence de moyens de transmission de chaleur sous la forme d'un solvant organique dans lequel il est partiellement soluble, il est à noter que la durée du traitement thermique est plus courte que celle du traitement thermique relatif au cas où ledit précurseur solide sous forme de résine dure cassante ou ledit dimère phénolique est mis en présence de moyens de transmission de chaleur sous la forme d'un four ou d'un solvant dans lequel il est insoluble.

Dans le cas où ledit précurseur solide sous forme de résine dure cassante ou ledit dimère phénolique est mis en présence d'un solvant organique dans lequel il est partiellement soluble, il est à noter que le traitement thermique est plus court que celui relatif au cas où ledit précurseur solide sous forme de résine dure cassante ou ledit dimère phénolique est insoluble dans ledit solvant organique.

En fonction du solvant utilisé pour le traitement thermique avec dissolution, la taille des macrocycles obtenue peut être modulée.

Plus la température du milieu réactionnel est basse, plus la taille des macrocycles obtenus diminue.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant éliminée du milieu réactionnel durant ledit chauffage à une température comprise de 100 à 130°C et un précurseur solide sous forme de résine dure cassante, éventuellement isolé, est obtenu, ledit précurseur solide sous forme de résine dure cassante étant précédé de la formation d'un dimère phénolique, éventuellement isolé, dans laquelle ledit précurseur solide sous forme de résine dure cassante ou ledit dimère phénolique, éventuellement isolé du milieu réactionnel, est mis en présence de moyens de transmission de chaleur sous la forme d'un four ou d'un solvant, dans lequel ledit précurseur solide sous forme de résine dure cassante et ledit dimère phénolique sont insolubles, ou d'un solvant organique dans lequel ledit précurseur solide sous forme de résine dure cassante et ledit dimère phénolique sont au moins partiellement solubles,
ledit phénol substitué en position 4 de formule (I) étant le 4-benzyloxyphénol.

En particulier, le traitement thermique est effectué au reflux dudit solvant organique, dans lequel ledit précurseur solide sous forme de résine dure cassante et ledit dimère phénolique sont au moins partiellement solubles, avec ou sans agitation, préférentiellement sans agitation du milieu réactionnel.

Le traitement thermique peut être effectué au reflux dudit solvant, dans lequel ledit précurseur solide sous forme de résine dure cassante et ledit dimère phénolique sont insolubles, avec ou sans agitation, préférentiellement sans agitation du milieu réactionnel.

Dans le cas où la résine est soumise à un traitement thermique dans un solvant, dans lequel elle est insoluble, on peut par exemple utiliser une huile de silicone, qui comporte un point d'ébullition suffisamment élevé pour ne pas avoir à utiliser de système de réfrigération, d'où une simplification du procédé.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant éliminée du milieu réactionnel durant ledit chauffage à une température comprise de 100 à 130°C et un précurseur solide sous forme de résine dure cassante, éventuellement isolé, est obtenu, ledit précurseur solide sous forme de résine dure cassante étant précédé de la formation d'un dimère phénolique, éventuellement isolé, dans laquelle ledit précurseur solide sous forme de résine dure cassante ou ledit dimère phénolique, éventuellement isolé du milieu réactionnel, est mis en présence d'un solvant organique dans lequel ledit précurseur solide sous forme de résine dure cassante et ledit dimère phénolique sont insolubles, ou d'un solvant organique dans lequel ledit précurseur solide sous forme de résine dure cassante et ledit dimère phénolique sont au moins partiellement solubles,
ledit phénol substitué en position 4 de formule (I) étant le 4-benzyloxyphénol.

Le traitement thermique est effectué au reflux dudit solvant organique, avec ou sans agitation, préférentiellement sans agitation du milieu réactionnel.

Dans le cas où la résine est soumise à un traitement thermique dans un solvant dans lequel elle est insoluble, on peut utiliser une huile de silicone, qui comporte un point d'ébullition suffisamment élevé pour ne pas avoir à utiliser de système de réfrigération, d'où une simplification du procédé.

Dans un mode de réalisation avantageux, la présente invention concerne l'utilisation d'au moins une base en solution aqueuse avec au moins un phénol substitué en position 4 de formule (I), telle que définie ci-dessus, dans laquelle de l'eau est produite lors de la réaction, ladite eau étant conservée dans le milieu réactionnel durant ledit chauffage à une température comprise de 100 à 130°C et un précurseur solide, sous forme de précipité, éventuellement isolé, est obtenu.

Dans ce mode de réalisation, l'eau formée au cours de la réaction n'est pas éliminée mais conservée dans le milieu réactionnel.

Ceci a pour conséquence que le milieu réactionnel ne se solidifie par complètement mais qu'au contraire un précipité, en suspension dans l'eau, est obtenu. Le précipité peut être isolé par simple filtration.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant conservée dans le milieu réactionnel durant ledit chauffage à une température comprise de 100 à 130°C et un précurseur solide, sous forme de précipité, éventuellement isolé, est obtenu, tel que défini ci-dessus,
dans laquelle ledit précurseur solide sous forme de précipité est isolé du milieu réactionnel et dépourvu de traitement thermique,
pour la préparation d'un mélange de *p*-(R)calixarènes géants dont la taille est comprise de 21 à 35 unités phénoliques.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant conservée dans le milieu réactionnel durant ledit chauffage à une température comprise de 100 à 130°C et un précurseur solide, sous forme de précipité, éventuellement isolé, est obtenu, tel que défini ci-dessus,
dans laquelle ledit précurseur solide sous forme de précipité est isolé du milieu réactionnel et dépourvu de traitement thermique en présence d'un solvant organique,
pour la préparation d'un mélange de *p*-(R)calixarènes géants dont la taille est comprise de 21 à 35 unités phénoliques.

Le produit obtenu dans le précipité est donc constitué essentiellement de *p*-(R)calixarènes géants et ne nécessite pas nécessairement de traitement thermique pour compléter la formation des macrocycles (*p*-(R)calixarènes

géants).

Les *p*-(R)calixarènes géants sont de taille inférieure lorsque l'eau est conservée par rapport au mode de réalisation avec élimination de l'eau.

En fonction de R et de la base utilisée, le produit obtenu dans le précipité peut contenir cependant également, en proportion variable, des oligomères linéaires issus de la condensation du phénol substitué en position 4 de formule (I) et nécessite alors une étape de purification.

Dans un mode de réalisation avantageux, ledit chauffage à une température comprise de 100 à 130°C est effectué durant 30 minutes à 5 heures, avantageusement durant 1h à 5h, plus avantageusement durant 2 h à 5h.

Avantageusement, ledit chauffage à une température comprise de 100 à 130°C est effectué notamment durant 3h.

Avantageusement, ledit chauffage à une température comprise de 100 à 130°C est effectué notamment durant 4h.

Avantageusement, ledit chauffage à une température comprise de 100 à 130°C est effectué notamment durant 5h.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant conservée dans le milieu réactionnel durant ledit chauffage à une température comprise de 100 à 130°C et un précurseur solide, sous forme de précipité est obtenu, ledit précurseur solide sous forme de précipité étant isolé du milieu réactionnel et dépourvu de traitement thermique, telle que définie ci-dessus,
dans laquelle la base utilisée est l'hydroxyde de baryum.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant conservée dans le milieu réactionnel durant ledit chauffage à une température comprise de 100 à 130°C et un précurseur solide, sous forme de précipité est obtenu, ledit précurseur solide sous forme de précipité étant isolé du milieu réactionnel et dépourvu de traitement thermique en présence d'un solvant organique, tel que défini ci-dessus,
dans laquelle la base utilisée est l'hydroxyde de baryum.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant conservée dans le milieu réactionnel durant ledit chauffage à une température comprise de 100 à 130°C et un précurseur solide, sous forme de précipité est obtenu, dans laquelle ledit précurseur solide sous forme de précipité est précédé de la formation d'un dimère phénolique, éventuellement isolé.

En fonction de la durée du chauffage à une température comprise de 100 à 130°C, du phénol substitué en position 4 de formule (I) avec ladite base et le formaldéhyde aqueux, avec élimination de l'eau formée, des oligomères linéaires de formule (III) suivante, de taille variable, correspondant à la condensation du phénol substitué en position 4 de formule (I) avec le formaldéhyde sont obtenus avant cyclisation en *p*-(R)calixarènes géants :

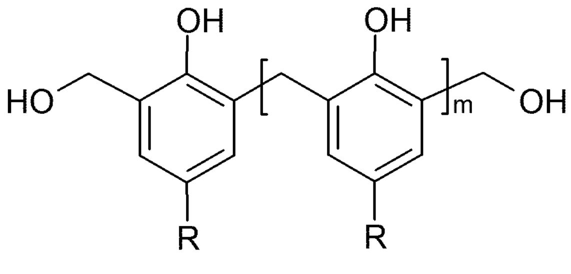

(III)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à C20, NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),

et m est un entier compris de 2 à plus de 200.

Dans ce qui précède, R peut également représenter :

- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire.

Avantageusement, le chauffage à une température comprise de 100 à 130°C est effectué d'environ 10 minutes à environ 25 minutes pour obtenir les oligomères.

Plus avantageusement, le chauffage à une température comprise de 100 à 130°C est effectué environ 20 minutes pour obtenir les oligomères.

Ces oligomères sont ensuite transformés en dimère correspondant de formule (II) suivante, si le chauffage du milieu réactionnel initial est effectué durant 30 minutes à 2h :

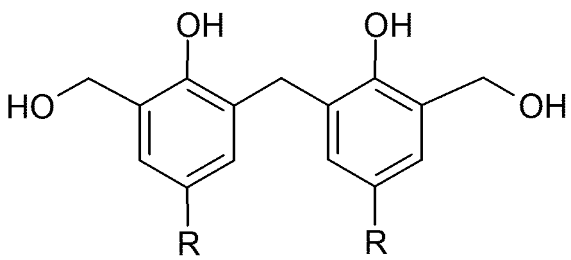

(II)

dans laquelle R est tel que décrit précédemment.

Ledit dimère peut alors être isolé ou non.

Ledit dimère non isolé soit se réarrange pour former des oligomères de taille variée soit se combine avec un autre dimère ou des oligomères résiduels pour former le précurseur solide sous forme de résine comprenant environ 50% de *p*-(R)calixarènes géants lorsque le chauffage à une température comprise de 100 à 130°C est poursuivi durant 1h à 3h additionnelles.

Avantageusement, le chauffage additionnel est effectué durant environ 1h.

Avantageusement, le chauffage additionnel est effectué durant environ 2h.

Avantageusement, le chauffage additionnel est effectué durant environ 3h.

Lorsque le temps de chauffage à une température comprise de 100 à 130°C est contrôlé, entre 30 minutes et 2 heures, le dimère peut alors être isolé par filtration et ensuite remis à réagir dans un milieu aqueux ou une combinaison eau-solvant organique comprenant au moins une base en solution aqueuse pour la poursuite du chauffage à une température comprise de 100 à 130°C durant 1 à 3h.

Avantageusement, la poursuite du chauffage additionnel est effectuée durant environ 1h.

Avantageusement, la poursuite du chauffage additionnel est effectuée durant environ 2h.

Avantageusement, la poursuite du chauffage additionnel est effectuée durant environ 3h.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant conservée dans le milieu réactionnel durant ledit chauffage à une température comprise de 100 à 130°C et un précurseur solide, sous forme de précipité, éventuellement isolé, est obtenu, ledit précurseur solide sous forme de précipité étant précédé de la formation d'un dimère phénolique, éventuellement isolé, tel que défini ci-dessus,

dans laquelle ledit précurseur solide sous forme de précipité ou ledit dimère phénolique, éventuellement isolé du susdit milieu réactionnel, est mis en présence de moyens de transmission de chaleur sous la forme d'un four ou d'un solvant,

en particulier un solvant dans lequel ledit précurseur solide sous forme de précipité et ledit dimère phénolique sont insolubles, notamment dans un alcane linéaire ou ramifié de point d'ébullition supérieur à 50°C, notamment supérieur à 100°C, en particulier dans l'octane, et est soumis audit traitement thermique,
pour la préparation d'un mélange de *p*-(R)calixarènes géants dont la taille est comprise de 21 à environ 212 unités phénoliques.

En particulier, le traitement thermique est effectué au reflux dudit solvant, en particulier organique, avec ou sans agitation, préférentiellement sans agitation du milieu réactionnel, et a notamment pour effet d'une part de compléter la formation des macrocycles, notamment à partir des oligomères linéaires compris dans le précurseur solide sous forme de résine, mais également d'augmenter la taille des macrocycles obtenus aussi bien à partir du précurseur solide sous forme de résine que dudit dimère.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant conservée dans le milieu réactionnel durant ledit chauffage à une température comprise de 100 à 130°C et un précurseur solide, sous forme de précipité, éventuellement isolé, est obtenu, ledit précurseur solide sous forme de précipité étant précédé de la formation d'un dimère phénolique, éventuellement isolé, tel que défini ci-dessus,
dans laquelle ledit précurseur solide sous forme de précipité ou ledit dimère phénolique, éventuellement isolé du susdit milieu réactionnel, est mis en présence d'un solvant organique dans lequel ledit précurseur solide sous forme de précipité et ledit dimère phénolique sont insolubles, notamment dans un alcane linéaire ou ramifié de point d'ébullition supérieur à 50°C, notamment supérieur à 100°C, en particulier dans l'octane, et est soumis audit traitement thermique,
pour la préparation d'un mélange de *p*-(R)calixarènes géants dont la taille est comprise de 21 à environ 212 unités phénoliques.

Le traitement thermique est effectué au reflux dudit solvant organique, avec ou sans agitation, préférentiellement sans agitation du milieu réactionnel, et a notamment pour effet d'une part de compléter la formation des macrocycles, notamment à partir des oligomères linéaires compris dans le précurseur solide sous forme de résine, mais également d'augmenter la taille des macrocycles obtenus aussi bien à partir du précurseur solide sous forme de résine que dudit dimère.

Les calixarènes géants obtenus comprennent cependant une proportion de calix[7]arène et/ou de calix[8]arène variable en fonction de R.

Plus le précurseur solide sous forme de résine ou le dimère est insoluble dans le solvant organique et plus la proportion de calixarènes géants augmente.

La durée du traitement thermique doit également être contrôlée et doit être comprise de 3 à 8h en fonction de R et de la base utilisée.

Au-delà de 8h, la proportion de petits calixarènes obtenus augmente jusqu'à n'obtenir que des petits calixarènes, les calixarènes géants disparaissant en se dissociant au profit desdits petits calixarènes..

Avantageusement, le traitement thermique est compris d'environ 3 à 4h.

Avantageusement, lorsque le traitement thermique est compris de 3 à 4h, la base utilisée est LiOH, KOH, NaOH, CsOH ou RbOH.

Avantageusement, le traitement thermique est compris d'environ 6 à 8h, en particulier 7h.

Avantageusement, lorsque le traitement thermique est compris d'environ 6 à 8h, en particulier 7h, la base utilisée est $Ba(OH)_2$.

En fonction du solvant utilisé pour le traitement thermique, la taille des macrocycles obtenue peut être modulée, la polarité du solvant ayant une action à la fois sur la dissociation et sur la réactivité des composés chimiques.

Plus la température du milieu réactionnel est basse plus la taille des macrocycles obtenus diminue.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant conservée dans le milieu réactionnel durant ledit chauffage à une température comprise de 100 à 130°C et un précurseur solide, sous forme de précipité, éventuellement isolé, est obtenu, ledit précurseur solide sous forme de précipité étant précédé de la formation d'un dimère phénolique, éventuellement isolé, tel que défini ci-dessus,
dans laquelle ledit précurseur solide sous forme de précipité ou ledit dimère phénolique, éventuellement isolé du susdit milieu réactionnel, est mis en présence d'un solvant organique dans lequel ledit précurseur solide sous forme de précipité et ledit dimère phénolique sont au moins partiellement solubles, en particulier dans le xylène, le toluène, le DMSO ou le DMF et est soumis audit traitement thermique, pour la préparation d'un mélange de *p*-(R)calixarènes géants dont la taille est comprise de 21 à environ 212 unités phénoliques.

Le traitement thermique est effectué au reflux dudit solvant organique, avec ou sans agitation, préférentiellement sans agitation du milieu réactionnel.

Lorsque le précurseur solide sous forme de précipité ou le dimère sont dissouts lors de l'étape du traitement

thermique, les macrocycles obtenus sont de pureté supérieure par rapport à ceux obtenus en l'absence de dissolution du précurseur sous forme de précipité ou du dimère dans le solvant organique; cependant, la proportion de petits calixarènes et notamment de calix[7]arène et/ou de calix[8]arène est supérieure.

La durée du traitement thermique doit également être contrôlée et doit être comprise de 3 à 8h en fonction de R et de la base utilisée.

Au-delà de 8h, la proportion de petits calixarènes obtenus augmente jusqu'à n'obtenir que des petits calixarènes, les calixarènes géants disparaissant en se dissociant au profit desdits petits calixarènes.

Avantageusement, le traitement thermique est compris d'environ 3 à 4h.

Avantageusement, lorsque le traitement thermique est compris de 3 à 4h, la base utilisée est LiOH, KOH, NaOH, CsOH ou RbOH.

Avantageusement, le traitement thermique est compris d'environ 6 à 8h, en particulier 7h.

Avantageusement, lorsque le traitement thermique est compris d'environ 6 à 8h, en particulier 7h, la base utilisée est $Ba(OH)_2$.

En fonction du solvant utilisé pour le traitement thermique avec dissolution, la taille des macrocycles peut être modulée.

Plus la température du milieu réactionnel est basse plus la taille des macrocycles obtenus est faible.

Dans un mode de réalisation avantageux, la présente invention concerne l'utilisation d'au moins une base en solution aqueuse avec au moins un phénol substitué en position 4 de formule (I), dans laquelle de l'eau est produite lors de la réaction, ladite eau étant conservée dans le milieu réactionnel durant ledit chauffage à une température comprise de 100 à 130°C et un précurseur solide, sous forme de précipité, éventuellement isolé, est obtenu, ledit précurseur solide sous forme de précipité étant précédé de la formation d'un dimère phénolique, éventuellement isolé, tel que défini ci-dessus, ledit précurseur solide sous forme de précipité ou ledit dimère phénolique, éventuellement isolé du susdit milieu réactionnel, étant mis en présence d'un solvant organique dans lequel ledit précurseur solide sous forme de précipité et ledit dimère phénolique sont insolubles, ou d'un solvant organique dans lequel ledit précurseur solide sous forme de précipité et ledit dimère phénolique sont au moins partiellement solubles, et est soumis audit traitement thermique, tel que défini ci-dessus,

dans laquelle le phénol substitué en position 4 de formule (I) est le 4-benzyloxyphénol.

Le traitement thermique est effectué au reflux dudit solvant organique, avec ou sans agitation, préférentiellement sans agitation du milieu réactionnel.

Il est à noter que ce procédé avec conservation de l'eau permet d'accéder à des calixarènes dont la gamme de taille est différente de ceux obtenus lors du procédé avec élimination de l'eau durant le chauffage.

Selon un autre aspect, la description concerne un dimère phénolique substitué en position 4 de formule (II) suivante :

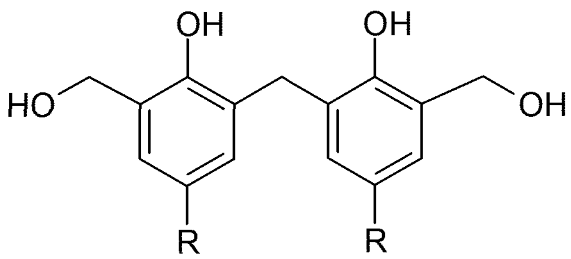

(II)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther $-S-CH_2-Ph$ éventuellement substitué en ortho et/ou para et/ou méta par un ou plusieurs

substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

- un groupe alkylthioéther en $C_1$-$C_{20}$ ramifié ou linéaire, de formule -S-($C_1$-$C_{20}$-alkyle).

Dans ce qui précède, R peut également représenter :

- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire.

Dans un mode de réalisation avantageux, la présente description concerne un dimère phénolique substitué en position 4 de formule (II) tel que défini ci-dessus, dans lequel R est choisi parmi :

- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire.

Dans un mode de réalisation avantageux, la présente description concerne un dimère phénolique substitué en position 4 de formule (II) tel que défini ci-dessus, dans lequel le groupe alkyle ramifié ou linéaire ou le groupe alkyloxy ramifié ou linéaire est en $C_5$-$C_{20}$.

Dans un mode de réalisation avantageux, la présente description concerne un dimère phénolique substitué en position 4 de formule (II) tel que défini ci-dessus, dans lequel R est choisi parmi le groupe benzyloxy, le groupe benzyle, le groupe octyle ou le groupe octyloxy.

Selon un autre aspect, la présente invention concerne un précurseur solide sous forme de résine ou de précipité comprenant un mélange de *p*-(R)calixarènes géants et d'oligomères phénoliques de formule (III) suivante :

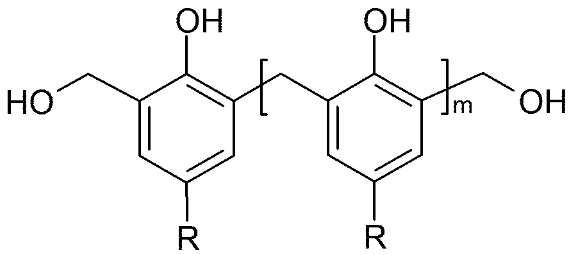

(III)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

- un groupe alkylthioéther en $C_1$-$C_{20}$ ramifié ou linéaire, de formule -S-($C_1$-$C_{20}$-alkyle),

et m est un entier compris de 2 à plus de 200.

Dans ce qui précède, R peut également représenter :

- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire.

Dans un mode de réalisation avantageux, la présente invention concerne un précurseur solide sous forme de résine ou de précipité comprenant un mélange de *p*-(R)calixarènes géants et d'oligomères phénoliques de formule (III) tel que défini ci-dessus, dans lequel R est choisi parmi :

- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire.

Précurseur solide sous forme de résine ou de précipité comprenant un mélange de *p*-(R)calixarènes géants et d'oligomères phénoliques de formule (III) telle que définie ci-dessus, dans lequel R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_5$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_5$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire.

Selon un autre aspect, la présente description concerne un mélange de calixarènes de formule (IV) suivante :

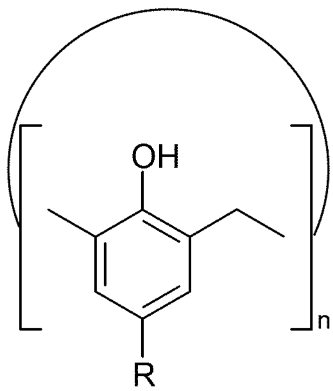

(IV)

dans lequel n est un entier compris de 21 à plus de 220, notamment de 21 à 220, en particulier de 21 à 212, plus particulièrement de 21 à 200, R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle).

Dans ce qui précède, R peut également représenter :

- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire.

Selon un autre aspect, la présente invention concerne un mélange de calixarènes de formule (IV) suivante :

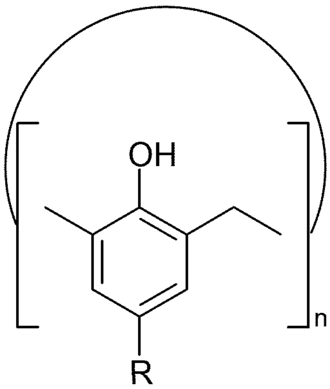

(IV)

dans lequel n est un entier compris de 21 à plus de 220, R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ linéaire,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

- un groupe alkylthioéther en C1-C20 ramifié ou linéaire, de formule -S-($C_1$-$C_{20}$-alkyle),
- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en C3 à C20, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire.

Ledit mélange de calixarènes de formule (IV), dans un milieu dépourvu de base et dans des conditions adéquates, en particulier une température inférieure à 100°C, est stable, c'est-à-dire que les calixarènes de formule (IV) ne se dissocient pas au profit de petits calixarènes de type calix[6-8]arènes au cours du temps, par exemple sur une durée au moins supérieure à 2 ans.

Dans un mode de réalisation avantageux, la présente invention concerne un mélange de calixarènes de formule (IV) tel que défini ci-dessus, avec un nombre moyen de motifs n = environ 100.

Dans un mode de réalisation avantageux, la présente invention concerne un mélange de calixarènes de formule (IV) dans lequel n correspond à un nombre de motifs phénoliques d'environ 35.

Dans un mode de réalisation avantageux, la présente invention concerne un mélange de calixarènes de formule (IV) tel que défini ci-dessus, dans lequel R est choisi parmi :

- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire.

Dans un mode de réalisation avantageux, la présente description concerne un mélange de calixarènes de formule (IV), tel que défini ci-dessus, dans lequel R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_5$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_5$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire.

Dans ce qui précède, R peut également représenter :

- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire.

Dans un mode de réalisation avantageux, la présente invention concerne un mélange de calixarènes de formule (IV) tel que défini ci-dessus, dans lequel R est le groupe octyloxy et présentant en GPC une masse molaire au pic correspondant à 35 unités phénoliques.

Dans un mode de réalisation avantageux, la présente invention concerne un mélange de calixarènes de formule (IV) tel que défini ci-dessus, dans lequel R est le groupe benzyloxy et la taille déterminée par la gaussienne centrée en GPC varie de 21 à environ 212.

Selon un autre aspect, la présente description concerne un procédé de préparation d'un mélange de *p*-(R)calixarènes géants dont la taille est supérieure à 20, notamment de 21 à plus de 220, notamment de 21 à environ 220, en

particulier de 21 à environ 212, plus particulièrement de 21 à environ 200,
comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I) suivante :

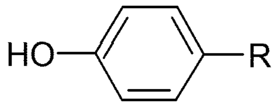

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, Ra et Rb représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),
- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

et une source de formaldéhyde aqueux,
en l'absence ou en présence de solvant organique, constituant ainsi un milieu réactionnel, ladite base étant à une concentration totale comprise d'environ 0,01 à 1 équivalent, notamment comprise d'environ 0,1 équivalent à environ 0,4 équivalent, en particulier égale à 0,15 ou 0,4 équivalent, ou 1 équivalent, par rapport audit au moins un phénol substitué en position 4 de formule (I),
ledit milieu réactionnel étant chauffé à une température comprise de 100 à 130°C, pour obtenir un milieu réactionnel chauffé,
et comprenant, éventuellement, après ledit chauffage, une étape de traitement thermique dudit milieu réactionnel chauffé, en présence de moyens de transmission de chaleur, notamment un four ou un liquide.

Selon un autre aspect, la présente invention concerne un procédé de préparation d'un mélange de *p*-(R)calixarènes géants dont la taille est supérieure à 20, comprenant une étape de mise en contact d'au moins une base, avec au moins un phénol substitué en position 4 de formule (I) suivante :

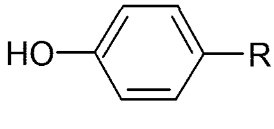

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle

en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

- un groupe alkyle en $C_1$-$C_{20}$ linéaire,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, NRaRb, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),
- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

et une source de formaldéhyde aqueux,
en l'absence ou en présence de solvant organique, constituant ainsi un milieu réactionnel,
ladite base étant à une concentration totale comprise de 0,01 à 1 équivalent, par rapport audit au moins un phénol substitué en position 4 de formule (I),
ledit milieu réactionnel étant chauffé à une température comprise de 100 à 130°C, pour obtenir un milieu réactionnel chauffé,
et comprenant, éventuellement, après ledit chauffage, une étape de traitement thermique dudit milieu réactionnel chauffé, en présence de moyens de transmission de chaleur.

Le terme base désigne toute base soluble en milieu aqueux de type hydroxyde métallique, amine tertiaire, carbonate, sulfate, carboxylate, par exemple.

Il peut également désigner l'utilisation d'une base organique de type amine en combinaison avec un sel métallique (CsI par exemple).

L'expression « source de formaldéhyde aqueux » signifie que du formaldéhyde en solution aqueuse tel que de la formaline ou du formol peuvent être utilisés.

L'expression « en solution aqueuse » dans toute la description signifie que la base est dissoute dans un milieu majoritairement constitué d'eau.

Avantageusement, l'expression « en solution aqueuse » désigne exclusivement de l'eau.

L'expression « en l'absence ou en présence de solvant organique » signifie que le milieu réactionnel (qui comprend de l'eau) est respectivement dépourvu ou pourvu de solvants qui sont des composés organiques qui contiennent des atomes de carbone, la base en tant que telle, le formaldéhyde ou la source de formaldéhyde et le phénol n'étant pas non plus considérés comme solvant organique.

L'expression « milieu réactionnel » signifie le mélange des différents composants, comprenant la ou les base(s), le ou les phénol(s) substitué(s) en position 4 de formule (I), la source de formaldéhyde, le tout étant en solution aqueuse ou dans un milieu eau-solvant organique.

Lorsque ledit mélange vient d'être constitué, il est dénommé milieu réactionnel initial, c'est-à-dire un milieu réactionnel dans lequel la réaction de préparation des *p*-(R)calixarènes n'a pas encore été effectuée, en particulier avant tout chauffage dudit mélange des différents composés.

Le milieu réactionnel chauffé correspond au milieu réactionnel initial qui a été chauffé à une température comprise de 100 à 130°C durant environ 30 minutes à 5 heures.

Lorsque la réaction de préparation des *p*-(R)calixarènes géants est effectuée, en particulier après chauffage dudit mélange des différents composés, et traitement thermique, le milieu réactionnel est alors dénommé milieu réactionnel final.

L'expression « et éventuellement soumis ensuite à un traitement thermique en présence de moyens de transmission de chaleur » signifie qu'après ledit chauffage à une température comprise de 100 à 130°C, au cours duquel l'eau est éventuellement éliminée, des moyens de transmission de chaleur sont appliqués au milieu réactionnel, puis ledit milieu réactionnel est chauffé à nouveau.

Les moyens de transmission de chaleur sont en particulier un four ou un solvant, en particulier un solvant chauffé à une température comprise de 120°C à 140°C, par exemple un solvant dont la température d'ébullition est

comprise de 120°C à 140°C.

Par solvant, on entend :

- un solvant, en particulier un solvant organique, dans lequel les composés organiques du milieu réactionnel sont au moins partiellement solubles, par exemple un solvant aromatique, de point d'ébullition supérieur à 50°C, notamment supérieur à 100°C,
  ou
- un liquide dans lequel les composés organiques du milieu réactionnel ne sont pas solubles, par exemple un alcane linéaire ou ramifié, en particulier l'octane, ou une huile de silicone, de point d'ébullition supérieur à 50°C, notamment supérieur à 100°C.

Par « solvant dans lequel les composés organiques du milieu réactionnel sont au moins partiellement solubles », on entend un solvant pouvant éventuellement, selon sa nature, solubiliser certains des constituants dudit milieu réactionnel, les disperser (pour former une suspension colloïdale) ou faire passer tout ou partie d'entre eux à l'état de gel.

Ainsi, lorsque les moyens de transmission de chaleur sont par exemple un four ou un solvant dans lequel les composés organiques du milieu réactionnel ne sont pas solubles, ledit traitement thermique se fait en phase solide, de préférence sans agitation.

Dans un mode de réalisation avantageux, le milieu réactionnel est dépourvu de solvant organique, l'eau est éliminée au cours dudit chauffage à une température comprise de 100 à 130°C, et ledit traitement thermique est réalisé en présence d'un solvant dans lequel les composés organiques du milieu réactionnel sont au moins partiellement solubles.

Dans un autre mode de réalisation avantageux, le milieu réactionnel est dépourvu de solvant organique, l'eau est éliminée au cours dudit chauffage à une température comprise de 100 à 130°C, et ledit traitement thermique est réalisé en présence d'un solvant dans lequel les composés organiques du milieu réactionnel ne sont pas solubles.

Dans un autre mode de réalisation avantageux, le milieu réactionnel est dépourvu de solvant organique, l'eau n'est pas éliminée au cours dudit chauffage à une température comprise de 100 à 130°C, et ledit traitement thermique est réalisé en présence d'un solvant dans lequel les composés organiques du milieu réactionnel sont au moins partiellement solubles.

Dans un autre mode de réalisation avantageux, le milieu réactionnel est dépourvu de solvant organique, l'eau n'est pas éliminée au cours dudit chauffage à une température comprise de 100 à 130°C, et ledit traitement thermique est réalisé en présence d'un solvant dans lequel les composés organiques du milieu réactionnel ne sont pas solubles.

Dans un autre mode de réalisation avantageux, le milieu réactionnel comprend, outre de l'eau, un solvant organique, l'eau n'est pas éliminée au cours du chauffage, et ledit milieu réactionnel n'est pas soumis audit traitement thermique supplémentaire.

Dans un autre mode de réalisation avantageux, le milieu réactionnel comprend, outre de l'eau, un solvant organique, l'eau n'est pas éliminée au cours du chauffage, et ledit milieu réactionnel est soumis audit traitement thermique supplémentaire, l'eau étant en particulier éliminée au cours dudit traitement thermique.

Le système eau-solvant organique est susceptible de favoriser la solubilisation de l'ensemble des composés organiques et inorganiques, au début de la réaction. Cela permet donc de maintenir les constituants du milieu réactionnel plus longtemps en solution. Il en résulte une meilleure consommation des réactifs, d'où 1) un meilleur rendement, et 2) moins de sous-produits (produits de départ, intermédiaires réactionnels...). Ceci rend la purification plus facile.

La présente description concerne également un procédé de préparation d'un mélange de *p*-(R)calixarènes géants dont la taille est supérieure à 20, notamment de 21 à plus de 220, notamment de 21 à environ 220, en particulier de 21 à environ 212, plus particulièrement de 21 à environ 200,
comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I) suivante :

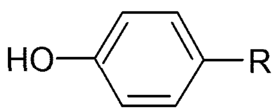
(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle

en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

- un groupe alkyle en $C_1$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),

et une source de formaldéhyde aqueux,
en l'absence de solvant organique, constituant ainsi un milieu réactionnel,
ladite base étant à une concentration totale comprise d'environ 0,01 à 1 équivalent, notamment comprise d'environ 0,1 équivalent à environ 0,4 équivalent, en particulier égale à 0,15 ou 0,4 équivalent, ou 1 équivalent, par rapport audit au moins un phénol substitué en position 4 de formule (I),
ledit milieu réactionnel étant chauffé à une température comprise de 100 à 130°C, pour obtenir un milieu réactionnel chauffé,
et comprenant, éventuellement, après ledit chauffage, une étape de traitement thermique dudit milieu réactionnel chauffé, en présence d'un solvant organique.

L'expression « en l'absence de solvant organique » signifie que le milieu réactionnel est dépourvu de solvants qui sont des composés organiques qui contiennent des atomes de carbone, la base en tant que telle, le formaldéhyde ou la source de formaldéhyde et le phénol n'étant pas non plus considérés comme solvant organique.

L'expression « milieu réactionnel » signifie le mélange des différents composants, comprenant la ou les base(s), le ou les phénol(s) substitué(s) en position 4 de formule (I), la source de formaldéhyde, le tout étant en solution aqueuse.

Dans un mode de réalisation avantageux, la présente invention concerne un procédé de préparation d'un mélange de *p*-(R)calixarènes géants tel que défini ci-dessus, dans lequel R est choisi parmi :

- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire.

Dans un mode de réalisation avantageux, la présente description concerne un procédé de préparation d'un mélange de *p*-(R)calixarènes géants tel que défini ci-dessus, dans lequel R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_5$-$C_{20}$ ramifié ou linéaire, à l'exclusion du groupe *t*-butyle,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_5$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire.

Dans un mode de réalisation avantageux, la présente invention concerne un procédé de préparation d'un mélange de *p*-(R)calixarènes géants tel que défini ci-dessus, dans lequel l'eau produite lors de la réaction est éliminée dudit milieu réactionnel durant ledit chauffage et un précurseur solide sous forme de résine dure cassante, éventuellement isolé, est obtenu.

Dans un mode de réalisation avantageux, la présente invention concerne un procédé de préparation d'un

mélange de *p*-(R)calixarènes géants, l'eau produite lors de la réaction étant éliminée dudit milieu réactionnel durant ledit chauffage et un précurseur solide sous forme de résine dure cassante, éventuellement isolé, étant obtenu, tel que défini ci-dessus,

dans lequel ledit précurseur solide sous forme de résine dure cassante est précédé de la formation d'un dimère phénolique, éventuellement isolé.

Dans ce mode de réalisation, le procédé conduit intermédiairement à l'obtention d'un oligomère de formule (III) puis d'un dimère phénolique de formule (II) qui peut être isolé ou non.

L'obtention dudit oligomère est effectuée au bout d'un chauffage à une température comprise de 100 à 130°C durant 10 à 25 minutes et l'obtention du dimère est effectuée à l'issue d'un chauffage à une température comprise de 100 à 130°C durant un temps compris de 30 minutes à 2h à partir du milieu réactionnel initial.

Si le chauffage à une température comprise de 100 à 130°C est poursuivi durant un temps compris de 1 à 3h sans isoler le dimère obtenu, une résine dure cassante est alors obtenue.

Dans ce cas, la proportion d'eau présente après obtention de la résine est de moins de 5% en poids.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, un précurseur solide sous forme de résine dure cassante, éventuellement isolé, étant obtenu, ledit précurseur solide sous forme de résine dure cassante étant précédé de la formation d'un dimère phénolique, éventuellement isolé, tel que défini ci-dessus,

dans lequel ledit précurseur solide sous forme de résine dure cassante est isolé du susdit milieu réactionnel et dépourvu de traitement thermique.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, un précurseur solide sous forme de résine dure cassante, éventuellement isolé, étant obtenu, ledit précurseur solide sous forme de résine dure cassante étant précédé de la formation d'un dimère phénolique, éventuellement isolé, tel que défini ci-dessus,

dans lequel ledit précurseur solide sous forme de résine dure cassante est isolé du susdit milieu réactionnel et dépourvu de traitement thermique en présence d'un solvant organique.

Le produit obtenu dans ces modes de réalisation est constitué à de 50% à 90% de *p*-(R)calixarènes géants (en fonction de la nature exacte du groupement R , de la base et de la concentration) comprenant éventuellement ledit dimère résiduel et/ou lesdits oligomères résiduels.

En fonction de R, le produit obtenu nécessite une purification ou non.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, un précurseur solide sous forme de résine dure cassante, éventuellement isolé, étant obtenu, ledit précurseur solide sous forme de résine dure cassante étant précédé de la formation d'un dimère phénolique, éventuellement isolé, ledit précurseur solide sous forme de résine dure cassante étant isolé du susdit milieu réactionnel et dépourvu de traitement thermique en présence d'un solvant organique, tel que défini ci-dessus, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I),
avec du formaldéhyde aqueux, pour constituer un milieu réactionnel et chauffage dudit milieu réactionnel durant un temps compris de 20 minutes à 2 heures, en particulier 1 heure, tout en éliminant l'eau formée,
pour obtenir un dimère de formule (II) tel que défini ci-dessus,

b. éventuellement poursuite du chauffage durant 1 à 3 heures dudit milieu réactionnel contenant ledit dimère tout en éliminant l'eau formée pour obtenir un milieu réactionnel contenant un précurseur solide, sous forme de résine dure cassante,

c. éventuellement, après neutralisation de la base, lavage dudit précurseur solide par un solvant polaire, en particulier le méthanol, pour obtenir un mélange de calixarènes géants lavé, dans lequel R est tel que défini ci-dessus, sous forme neutralisée.

d. éventuellement recristallisation dudit mélange de calixarènes géants lavé, dans un solvant à base de DMSO, pour obtenir un mélange de calixarènes géants purifié dans lequel R est tel que défini ci-dessus, et substantiellement dépourvu en *p*-(R)calix[7]arène et/ou en *p*-(R)calix[8]arène.

éventuellement, purification dudit mélange de calixarènes géants purifié dépourvu en *p*-(R)calix[7]arène et/ou en *p*-(R)calix[8]arène par GPC, en séparant différentes fractions en fonction de leur temps d'élution. Dans l'étape a, au bout de 10 à 25 minutes, il y a apparition des oligomères ou polymères de formule (III) qui conduisent ensuite, avec un chauffage plus prolongé, au dimère de formule (II).

Le chauffage est effectué à une température d'environ 100 à 130°C.

Avantageusement, le chauffage est d'environ 110°C, 120°C ou 130°C.

Si le dimère est le produit désiré, il est alors récupéré par filtration du milieu réactionnel.

Dans l'étape b., le chauffage est poursuivi à une température d'environ 100 à 130°C, si le dimère n'est pas le produit désiré, ce qui conduit audit précurseur sous forme de résine dure cassante. Avantageusement, le chauffage est poursuivi durant 1h, 2h ou 3h et à une température comprise de 100°C à 130°C, avantageusement à 110°C, 120°C ou 130°C.

Dans l'étape c., le précurseur solide sous forme de résine dure cassante, obtenu après neutralisation par un acide tel que HCl ou $H_2SO_4$, en particulier HCl dilué ou non, est constitué de 50 à 90% de *p*-(R)calixarènes géants qui peuvent comprendre également d'autres sous produits tels des oligomères résiduels ou du dimère résiduel, mais également du *p*-(R)calix[7]arène et/ou du *p*-(R)calix[8]arène.

Une première étape de purification consiste en un lavage par un solvant polaire, par exemple le méthanol, ce qui permet de purifier partiellement ledit précurseur et notamment d'éliminer certains oligomères linéaires résiduels ou le dimère résiduel.

Dans l'étape d., la recristallisation dans un mélange à base de DMSO permet de compléter la purification, en particulier en éliminant le *p*-(R)calix[7]arène et/ou le *p*-(R)calix[8]arène.

Le mélange de solvants à base de DMSO est notamment un mélange de DMSO avec du toluène ou de l'acétone, le DMSO étant dans une proportion de 5% à 50% en volume dans le mélange de solvant, en particulier de 10 %.

Le DMSO peut être remplacé par un solvant aprotique polaire, de préférence le DMF.

L'étape e. est effectuée pour séparer le mélange de *p*-(R)calixarènes géants purifié dépourvu en *p*-(R)calix[7]arène et/ou en *p*-(R)calix[8]arène en fractions de *p*-(R)calixarènes géants de taille différente.

Dans un mode de réalisation avantageux, la base utilisée dans l'étape a. est $Ba(OH)_2$ et R représente l'octyloxy. Le mélange de *p*-(R)calixarènes géants obtenu est du p-(octyloxy)calix[21-50]arènes et la taille déterminée par la gaussienne centrée en GPC correspond à environ 35 unités phénoliques (taille au pic).

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, un précurseur solide sous forme de résine dure cassante, éventuellement isolé, étant obtenu, ledit précurseur solide sous forme de résine dure cassante étant précédé de la formation d'un dimère phénolique, éventuellement isolé, tel que défini ci-dessus,

dans lequel ledit dimère phénolique ou ledit précurseur solide sous forme de résine dure cassante, contenu dans ledit milieu réactionnel, est mis en présence de moyens de transmission de chaleur sous la forme d'un four ou d'un solvant, en particulier un solvant dans lequel ledit précurseur solide sous forme de résine dure cassante, et ledit dimère phénolique, sont insolubles, notamment dans un alcane linéaire ou ramifié de point d'ébullition supérieur à 50°C, notamment supérieur à 100°C, en particulier l'octane, et est soumis à un traitement thermique.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, un précurseur solide sous forme de résine dure cassante, éventuellement isolé, étant obtenu, ledit précurseur solide sous forme de résine dure cassante étant précédé de la formation d'un dimère phénolique, éventuellement isolé, tel que défini ci-dessus,

dans lequel ledit dimère phénolique ou ledit précurseur solide sous forme de résine dure cassante, contenu dans ledit milieu réactionnel, est mis en présence d'un solvant organique, en particulier un solvant organique dans lequel ledit précurseur solide sous forme de résine dure cassante, et ledit dimère phénolique, sont insolubles, notamment dans un alcane linéaire ou ramifié de point d'ébullition supérieur à 50°C, notamment supérieur à 100°C, en particulier l'octane, et est soumis à un traitement thermique.

Le solvant organique peut être n'importe quel solvant organique à condition que, d'une part, il ne réagisse pas avec le précurseur sous forme de résine et que, d'autre part, ledit précurseur sous forme de résine y soit insoluble.

Le point d'ébullition du solvant organique est notamment supérieur ou égal à 110°C.

Le traitement thermique est effectué au reflux dudit solvant organique, avec ou sans agitation, préférentiellement sans agitation du milieu réactionnel, durant un temps compris en fonction de R et de la base de 3 à 8h et a notamment pour effet d'une part de compléter la formation des macrocycles, notamment ceux compris dans le précurseur solide sous forme de résine, mais également d'augmenter la taille des macrocycles obtenus aussi bien à partir du précurseur solide sous forme de résine que dudit dimère.

Les calixarènes géants obtenus comprennent cependant une proportion de calix[7]arènes et/ou de calix[8]arènes variable en fonction de R, de la concentration en base, de la nature de la base et de la température, accompagnés d'une proportion variable de dimère/oligomères linéaires.

Plus le précurseur solide sous forme de résine ou le dimère est insoluble dans le solvant organique et plus la proportion de calixarènes géants augmente.

La durée du traitement thermique doit également être contrôlée et doit être comprise de 3 à 8h en fonction de

R et de la base utilisée.

Au-delà de 8h, la proportion de petits calixarènes obtenus augmente jusqu'à n'obtenir que des petits calixarènes, les calixarènes géants disparaissant en se dissociant au profit desdits petits calixarènes.

En fonction du solvant utilisé pour le traitement thermique, la taille des macrocycles obtenue peut être modulée.

Plus la température du milieu réactionnel est basse plus la taille des macrocycles obtenus diminue.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, un précurseur solide sous forme de résine dure cassante, éventuellement isolé, étant obtenu, ledit précurseur solide sous forme de résine dure cassante étant précédé de la formation d'un dimère phénolique, éventuellement isolé, ledit dimère phénolique ou ledit précurseur solide sous forme de résine dure cassante, contenu dans ledit milieu réactionnel, étant mis en présence de moyens de transmission de chaleur sous la forme d'un four ou d'un solvant, en particulier un solvant dans lequel ledit précurseur solide sous forme de résine dure cassante, et ledit dimère phénolique, sont insolubles, notamment dans un alcane linéaire ou ramifié de point d'ébullition supérieur à 50°C, notamment supérieur à 100°C, en particulier l'octane, ou une huile de silicone et est soumis à un traitement thermique, tel que défini ci-dessus, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I),
avec du formaldéhyde aqueux, pour constituer un milieu réactionnel et chauffage dudit milieu réactionnel durant un temps compris de 30 minutes à 2 heures, en particulier 1 heures tout en éliminant l'eau formée,
pour obtenir un dimère de formule (II) tel que défini ci-dessus,
b. éventuellement poursuite du chauffage dudit milieu réactionnel contenant ledit dimère tout en éliminant l'eau formée pour obtenir un milieu réactionnel contenant un précurseur solide, sous forme de résine dure cassante, durant 1 à 5 heures, la durée étant variable, en fonction de la concentration en base utilisée, et optionnellement filtration dudit précurseur solide, sous forme de résine dure cassante,
c. chauffage dudit milieu réactionnel à l'aide de moyens de transmission de chaleur sous la forme d'un four, ou addition audit milieu réactionnel contenant ledit précurseur solide sous forme de résine dure cassante d'un solvant dans lequel ledit précurseur solide sous forme de résine dure cassante est insoluble, notamment dans un alcane linéaire ou ramifié de point d'ébullition supérieur à 50°C, notamment supérieur à 100°C, en particulier supérieur ou égal à 110°C, en particulier l'octane ou une huile de silicone, pour obtenir un milieu réactionnel contenant un solide sous forme de résine dure cassante, dans un four ou un solvant et chauffage dudit milieu réactionnel contenant un solide sous forme de résine dure cassante, durant 3 à 8 heures,
pour obtenir un mélange de calixarènes géants dans lequel R est tel que défini ci-dessus, comprenant de plus un *p*-(R)calix[7]arène et/ou un *p*-(R)calix[8]arène,
d. recristallisation, après neutralisation de la base, dudit mélange de calixarènes géants, dans un solvant à base de DMSO, pour obtenir un mélange de calixarènes géants purifié dans lequel R est tel que défini ci-dessus, substantiellement dépourvu en *p*-(R)calix[7]arène et/ou en *p*-(R)calix[8]arène.
e. éventuellement, purification par CPG, en séparant différentes fractions en fonction de leur temps d'élution.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, un précurseur solide sous forme de résine dure cassante, éventuellement isolé, étant obtenu, ledit précurseur solide sous forme de résine dure cassante étant précédé de la formation d'un dimère phénolique, éventuellement isolé, ledit dimère phénolique ou ledit précurseur solide sous forme de résine dure cassante, contenu dans ledit milieu réactionnel, étant mis en présence d'un solvant organique, en particulier un solvant organique dans lequel ledit précurseur solide sous forme de résine dure cassante, et ledit dimère phénolique, sont insolubles, notamment dans un alcane linéaire ou ramifié de point d'ébullition supérieur à 50°C, notamment supérieur à 100°C, en particulier l'octane, ou une huile de silicone et étant soumis à un traitement thermique, tel que défini ci-dessus, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I),
avec du formaldéhyde aqueux, pour constituer un milieu réactionnel et chauffage dudit milieu réactionnel durant un temps compris de 30 minutes à 2 heures, en particulier 1 heures tout en éliminant l'eau formée,
pour obtenir un dimère de formule (II) tel que défini ci-dessus,
b. éventuellement poursuite du chauffage dudit milieu réactionnel contenant ledit dimère tout en éliminant l'eau formée pour obtenir un milieu réactionnel contenant un précurseur solide, sous forme de résine dure cassante,

durant 1 à 5 heures, la durée étant variable, en fonction de la concentration en base utilisée, et optionnellement filtration dudit précurseur solide, sous forme de résine dure cassante,

c. addition audit milieu réactionnel contenant ledit précurseur solide sous forme de résine dure cassante d'un solvant organique dans lequel ledit précurseur solide sous forme de résine dure cassante est insoluble, notamment dans un alcane linéaire ou ramifié de point d'ébullition supérieur à 50°C, notamment supérieur à 100°C, en particulier supérieur à 110°C, en particulier l'octane ou une huile de silicone, pour obtenir un milieu réactionnel contenant un solide sous forme de résine dure cassante, dans un solvant et chauffage dudit milieu réactionnel contenant un solide sous forme de résine dure cassante, durant 3 à 8 heures,

pour obtenir un mélange de calixarènes géants dans lequel R est tel que défini ci-dessus, comprenant de plus un *p*-(R)calix[7]arène et/ou un *p*-(R)calix[8]arène,

d. recristallisation, après neutralisation de la base, dudit mélange de calixarènes géants, dans un solvant à base de DMSO, pour obtenir un mélange de calixarènes géants purifié dans lequel R est tel que défini ci-dessus, substantiellement dépourvu en *p*-(R)calix7]arène et/ou en *p*-(R)calix 8]arène.

e. éventuellement, purification par CPG, en séparant différentes fractions en fonction de leur temps d'élution.

Dans l'étape a., au bout de 10 à 25 minutes, il y a apparition des oligomères ou polymères de formule (III) qui conduisent ensuite, avec un chauffage plus prolongé, au dimère de formule (II).

Le chauffage est effectué à une température d'environ 100 à 130°C durant 30 minutes à 2h à partir du milieu réactionnel initial pour former le dimère de formule (II)

Avantageusement, le chauffage est d'environ 110°C, 120°C ou 130°C.

Si le dimère est le produit désiré, il est alors récupéré par filtration du milieu réactionnel.

Dans l'étape b., le chauffage est poursuivi à une température d'environ 100 à 130°C, si le dimère n'est pas le produit désiré, ce qui conduit audit précurseur sous forme de résine dure cassante. Avantageusement, le chauffage est poursuivi durant 1h, 2h ou 3h et à une température comprise de 100°C à 130°C, avantageusement à 110°C, 120°C ou 130°C.

Le mélange obtenu dans l'étape b. est constitué à environ 50% à 90% de *p*-(R)calixarènes géants, en fonction du groupement R, et peut être isolé par simple filtration.

Dans l'étape c., soit le solvant est introduit directement dans le milieu réactionnel de l'étape précédente b., soit ledit précurseur solide sous forme de résine dure cassante est introduit dans un appareil pour effectuer une réaction et le solvant organique dans lequel ledit précurseur solide sous forme de résine dure cassante et insoluble est introduit, constituant ainsi le milieu réactionnel.

En particulier, le traitement thermique est effectué au reflux dudit solvant organique, avec ou sans agitation, préférentiellement sans agitation du milieu réactionnel.

Le traitement thermique permet de compléter la formation des macrocycles et d'augmenter la taille de *p*-(R)calixarènes géants.

Avantageusement, le traitement thermique est compris d'environ 3 à 4h.

Avantageusement, lorsque le traitement thermique est compris de 3 à 4h, la base utilisée est LiOH, KOH, NaOH, CsOH ou RbOH.

Avantageusement, le traitement thermique est compris d'environ 6 à 8h, en particulier 7h.

Avantageusement, lorsque le traitement thermique est compris d'environ 6 à 8h, en particulier 7h, la base utilisée est $Ba(OH)_2$.

Dans l'étape d, le mélange de solvants à base de DMSO est notamment un mélange de DMSO avec du toluène ou de l'acétone, le DMSO étant dans une proportion de 5% à 50% en volume dans le mélange de solvant, en particulier de 10 %.

Le DMSO peut être remplacé par un solvant aprotique polaire, de préférence le DMF.

L'étape e. est effectuée pour séparer le mélange de *p*-(R)calixarènes géants purifié dépourvu de *p*-(R)calix[7]arène et/ou de *p*-(R)calix[8]arène en fractions de *p*-(R)calixarènes géants de taille différente.

Dans un mode de réalisation avantageux, la base utilisée dans l'étape a. est $Ba(OH)_2$, LiOH, KOH, NaOH, CsOH ou RbOH et R représente le benzyloxy.

Le mélange de p-(R)calixarènes géants obtenu est du p-(benzyloxy)calix[21-60]arènes en fonction de la base utilisée et des différentes expériences, et la taille déterminée par la gaussienne centrée en GPC est en particulier fonction de la base utilisée, de sa concentration, des temps de réaction ; la gaussienne centrée correspond par exemple à environ 30 unités phénoliques, avec 0,4 équivalent de KOH, et l'octane comme solvant lors du traitement thermique.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, un précurseur solide sous forme de résine dure cassante, éventuellement isolé, étant obtenu, ledit précurseur solide sous forme de résine dure cassante étant précédé de la formation d'un dimère phénolique, éventuellement isolé, tel que défini ci-dessus,

dans lequel ledit dimère phénolique ou ledit précurseur solide sous forme de résine dure cassante, contenu dans ledit milieu réactionnel, est mis en présence d'un solvant organique, en particulier un solvant organique dans lequel ledit précurseur solide sous forme de résine dure cassante, et ledit dimère phénolique, sont au moins partiellement solubles, en particulier dans le xylène, le toluène, le DMSO ou le DMF, et est soumis à un traitement thermique. En effet, la dispersion du précurseur solide sous forme de résine dure cassante, et du dimère phénolique est complète, notamment sous forte agitation, mais il y demeure un précipité pouvant être constitué du calix[8]arène et/ou des calix[21-220]arènes.

Lorsque le précurseur solide sous forme de résine ou le dimère est dissout pour l'étape de traitement thermique, les macrocycles obtenus sont de pureté supérieure par rapport à la non dissolution du précurseur solide sous forme de résine ou du dimère dans le solvant organique; cependant, la proportion de petits calixarènes présente et notamment de calix[7]arènes et/ou de calix[8]arènes est supérieure.

Le traitement thermique est effectué au reflux dudit solvant organique, avec ou sans agitation, préférentiellement avec agitation du milieu réactionnel.

La durée du traitement thermique doit également être contrôlée et doit être comprise de 3 à 8h en fonction de R et de la base utilisée.

Au-delà de 8h, la proportion de petits calixarènes obtenus augmente jusqu'à n'obtenir que des petits calixarènes, les calixarènes géants disparaissant en se dissociant au profit desdits petits calixarènes.

En fonction du solvant utilisé pour le traitement thermique avec dissolution, la taille des macrocycles obtenus peut être modulée.

Plus la température du milieu réactionnel est basse plus la taille des macrocycles obtenus diminue.

Par exemple, un mélange de *p*-(R)calixarènes géants obtenu présente une taille déterminée par la gaussienne centrée en GPC d'environ 30 unités phénoliques.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, un précurseur solide sous forme de résine dure cassante, éventuellement isolé, étant obtenu, ledit précurseur solide sous forme de résine dure cassante étant précédé de la formation d'un dimère phénolique, éventuellement isolé, ledit dimère phénolique ou ledit précurseur solide sous forme de résine dure cassante, contenu dans ledit milieu réactionnel, étant mis en présence d'un solvant organique, en particulier un solvant organique dans lequel ledit précurseur solide sous forme de résine dure cassante et ledit dimère phénolique sont au moins partiellement solubles, en particulier dans le xylène, le toluène, le DMSO ou le DMF, et étant soumis à un traitement thermique, tel que défini ci-dessus, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I),
avec du formaldéhyde aqueux, pour constituer un milieu réactionnel et chauffage dudit milieu réactionnel durant un temps compris de 30 minutes à 2 heures, en particulier 1 heure, tout en éliminant l'eau formée,
pour obtenir un dimère de formule (II) tel que défini ci-dessus,
b. éventuellement poursuite du chauffage dudit milieu réactionnel contenant ledit dimère tout en éliminant l'eau formée pour obtenir un milieu réactionnel contenant un précurseur solide, sous forme de résine dure cassante, durant 1 à 3 heures, et optionnellement filtration dudit précurseur solide sous forme de résine dure cassante,
c. addition audit milieu réactionnel contenant ledit précurseur solide sous forme de résine dure cassante d'un solvant organique dans lequel ledit précurseur solide sous forme de résine dure cassante est au moins partiellement soluble, en particulier le xylène ou le toluène, le DMSO ou le DMF, pour obtenir un milieu réactionnel contenant un solide, sous forme de résine dure cassante, introduit dans un solvant et chauffage dudit milieu réactionnel contenant un solide sous forme de résine dissoute, durant 3 à 8 heures,
pour obtenir un mélange de calixarènes géants dans lequel R est tel que défini ci-dessus, comprenant de plus un *p*-(R)calix[7]arène et/ou un *p*-(R)calix[8]arène,
d. recristallisation, après neutralisation de la base, dudit mélange de calixarènes géants, dans un solvant à base de DMSO, pour obtenir un mélange de calixarènes géants purifié dans lequel R est tel que défini ci-dessus, substantiellement dépourvu en *p*-(R)calix[7]arène et/ou en *p*-(R)calix[8]arène.
e. éventuellement purification par GPC, en séparant différentes fractions en fonction de leur temps d'élution.

Dans l'étape a., au bout de 10 à 25 minutes, il y a apparition des oligomères ou polymères de formule (III) qui conduisent ensuite, avec un chauffage plus prolongé, au dimère de formule (II).

Le chauffage est effectué à une température d'environ 100 à 130°C durant 30 minutes à 2h à partir du milieu réactionnel initial pour former le dimère de formule (II)

Avantageusement, le chauffage est d'environ 110°C, 120°C ou 130°C.

Si le dimère est le produit désiré, il est alors récupéré par filtration du milieu réactionnel.

Dans l'étape b., le chauffage est poursuivi à une température d'environ 100 à 130°C, si le dimère n'est pas le produit désiré, ce qui conduit audit précurseur sous forme de résine dure cassante. Avantageusement, le chauffage est poursuivi durant 1h, 2h ou 3h et à une température comprise de 100°C à 130°C, avantageusement à 110°C, 120°C ou 130°C.

Le mélange obtenu dans l'étape b. est constitué à environ 50% à 90% de *p*-(R)calixarènes géants, en fonction de la nature du groupement R, et peut être isolé par simple filtration.

Dans l'étape c., le traitement thermique permet de compléter la formation des macrocycles et d'augmenter la taille de *p*-(R)calixarènes géants.

Avantageusement, le traitement thermique est compris d'environ 3 à 4h.

Avantageusement, lorsque le traitement thermique est compris de 3 à 4h, la base utilisée est LiOH, KOH, NaOH, CsOH ou RbOH.

Avantageusement, le traitement thermique est compris d'environ 6 à 8h, en particulier 7h.

Avantageusement, lorsque le traitement thermique est compris d'environ 6 à 8h, en particulier 7h, la base utilisée est $Ba(OH)_2$.

Dans l'étape d., le mélange de solvants à base de DMSO est notamment un mélange de DMSO avec du toluène ou de l'acétone, le DMSO étant dans une proportion de 5% à 50% en volume dans le mélange de solvant, en particulier de 10 %.

Le DMSO peut être remplacé par un solvant aprotique polaire, de préférence le DMF.

L'étape e. est effectuée pour séparer le mélange de *p*-(R)calixarènes géants purifié dépourvu en *p*-(R)calix[7]arène et/ou en *p*-(R)calix[8]arène en fractions de *p*-(R)calixarènes géants de taille différente. Dans un mode de réalisation avantageux, la base utilisée dans l'étape a. est $Ba(OH)_2$, LiOH, KOH, NaOH, CsOH ou RbOH et R représente le benzyloxy.

Le mélange de p-(R)calixarènes géants obtenu est du p-(benzyloxy)calix[21-50]arènes et la taille déterminée par la gaussienne centrée en GPC est d'environ 30.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, tel que défini ci-dessus,

dans lequel ledit dimère phénolique est isolé, puis soumis à un traitement thermique comme décrit précédemment et à une recristallisation tels que définis dans les étapes c. et d. ci-dessus.

Lorsque le dimère est isolé sous forme neutre, une base, en particulier la base ayant été utilisée pour l'obtention dudit dimère, plus particulièrement à la concentration employée pour l'obtention dudit dimère, est ajoutée préalablement au traitement thermique.

Lorsque le dimère est isolé sous forme de sel, l'ajout d'une base n'est pas nécessaire.

Ledit dimère isolé est donc mis en présence d'un solvant organique, en particulier un solvant organique dans lequel ledit dimère phénolique est soluble ou insoluble, tel que défini ci-dessus, et est soumis au traitement thermique comme ci-dessus.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, tel que défini ci-dessus,

dans lequel ledit précurseur solide sous forme de résine dure cassante précédé de la formation d'un dimère phénolique, est isolé et mis en présence de moyens de transmissions de chaleur sous la forme d'un four ou d'un solvant dans lequel ledit précurseur solide sous forme de résine dure cassante est insoluble, puis soumis à un traitement thermique et à une recristallisation tels que définis dans les étapes c. et d. plus haut.

Ledit précurseur solide sous forme de résine dure cassante isolé est donc mis en présence de moyens de transmissions de chaleur sous la forme d'un four ou d'un solvant dans lequel ledit dimère phénolique est soluble ou insoluble, tel que défini ci-dessus, et est soumis au traitement thermique comme ci-dessus.

Dans un mode de réalisation avantageux, la présente invention concerne un procédé de préparation d'un mélange de *p*-(R)calixarènes géants, l'eau produite lors de la réaction étant éliminée dudit milieu réactionnel durant ledit chauffage, tel que défini ci-dessus,

dans lequel ledit précurseur solide sous forme de résine dure cassante précédé de la formation d'un dimère phénolique, est isolé et mis en présence d'un solvant organique dans lequel ledit précurseur solide sous forme de résine dure cassante est insoluble, puis soumis à un traitement thermique et à une recristallisation tels que définis dans les étapes c. et d. ci-dessus.

Ledit précurseur solide sous forme de résine dure cassante isolé est donc mis en présence d'un solvant organique, en particulier un solvant organique dans lequel ledit dimère phénolique est soluble ou insoluble, tel que défini ci-dessus, et est soumis au traitement thermique comme ci-dessus.

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à

environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), tel que défini ci-dessus, dans lequel l'eau produite lors de la réaction est conservée dans ledit milieu réactionnel durant ledit chauffage à une température comprise de 100 à 130°C et un précurseur solide sous forme de précipité, éventuellement isolé, est obtenu.

Dans ce mode de réalisation, l'eau formée au cours de la réaction n'est pas éliminée mais conservée dans le milieu réactionnel.

Ceci a pour conséquence que le milieu réactionnel ne se solidifie par complètement mais qu'au contraire un précipité est obtenu. Le précipité de la même manière que la résine dure cassante peut être isolé par simple filtration.

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, tel que défini ci-dessus,
dans lequel ledit précurseur solide sous forme de précipité est précédé de la formation d'un dimère phénolique, éventuellement isolé.

Dans ce mode de réalisation, le procédé conduit intermédiairement à l'obtention d'un oligomère de formule (III) puis d'un dimère phénolique de formule (II) qui peut être isolé ou non.

L'obtention dudit oligomère est effectuée au bout d'un chauffage à une température comprise de 100 à 130°C durant 10 à 25 minutes et l'obtention du dimère est effectuée à l'issue d'un chauffage à une température comprise de 100 à 130°C durant un temps compris de 30 minutes à 2h à partir du milieu réactionnel initial.

Si le chauffage à une température comprise de 100 à 130°C est poursuivi durant un temps compris de 1 à 3h sans isoler le dimère obtenu, une résine dure cassante est alors obtenue.

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, tel que défini ci-dessus,
dans lequel ledit précurseur solide sous forme de précipité est isolé du susdit milieu réactionnel et dépourvu de traitement thermique.

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, tel que défini ci-dessus,
dans lequel ledit précurseur solide sous forme de précipité est isolé du susdit milieu réactionnel et dépourvu de traitement thermique en présence d'un solvant organique.

Le produit obtenu dans le précipité est donc constitué essentiellement de *p*-(R)calixarènes géants et ne nécessite pas nécessairement de traitement thermique pour compléter la formation des macrocycles (*p*-(R)calixarènes géants).

Les *p*-(R)calixarènes géants sont de taille inférieure lorsque l'eau est conservée par rapport au mode de réalisation avec élimination de l'eau.

Le produit obtenu dans ce mode de réalisation est constitué de 50 à 90% de *p*-(R)calixarènes géants comprenant éventuellement ledit dimère résiduel et/ou lesdits oligomères résiduels.

En fonction de R, le produit obtenu nécessite une purification ou non.

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde

de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, ledit précurseur solide sous forme de précipité étant isolé du susdit milieu réactionnel et dépourvu de traitement thermique, tel que défini ci-dessus, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier le 4-octyloxyphénol,
avec du formaldéhyde aqueux, pour constituer un milieu réactionnel et chauffage dudit milieu réactionnel durant un temps compris de 30 minutes à 2 heures, en particulier 1 heure, sans élimination de l'eau formée,
pour obtenir un dimère de formule (II) tel que défini ci-dessus,
b. éventuellement poursuite du chauffage dudit milieu réactionnel contenant ledit dimère tout en éliminant l'eau formée pour obtenir un milieu réactionnel contenant un précurseur solide, sous forme de précipité, durant 2 à 3 heures,
c. éventuellement, après neutralisation de la base, lavage du milieu réactionnel contenant un précurseur solide par un solvant polaire, en particulier le méthanol, pour obtenir un mélange de calixarènes géants dans lequel R est tel que défini ci-dessus, sous forme neutralisée,
d. éventuellement recristallisation dudit mélange de calixarènes géants lavé, dans un solvant à base de DMSO, pour obtenir un mélange de calixarènes géants purifié dans lequel R est tel que défini ci-dessus, et substantiellement dépourvu en *p*-(R)calix[7]arène et/ou en *p*-(R)calix[8]arène.
e. éventuellement, purification dudit mélange de calixarènes géants purifié dépourvu en *p*-(R)calix[7]arène et/ou en *p*-(R)calix[8]arène par CPG, en séparant différentes fractions en fonction de leur temps d'élution.

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, ledit précurseur solide sous forme de précipité étant isolé du susdit milieu réactionnel et dépourvu de traitement thermique en présence d'un solvant organique, tel que défini ci-dessus, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), en particulier le 4-octyloxyphénol,
avec du formaldéhyde aqueux, pour constituer un milieu réactionnel et chauffage dudit milieu réactionnel durant un temps compris de 30 minutes à 2 heures, en particulier 1 heure, sans élimination de l'eau formée,
pour obtenir un dimère de formule (II) tel que défini ci-dessus,
b. éventuellement poursuite du chauffage dudit milieu réactionnel contenant ledit dimère tout en éliminant l'eau formée pour obtenir un milieu réactionnel contenant un précurseur solide, sous forme de précipité, durant 2 à 3 heures,
c. éventuellement, après neutralisation de la base, lavage du milieu réactionnel contenant un précurseur solide par un solvant polaire, en particulier le méthanol, pour obtenir un mélange de calixarènes géants dans lequel R est tel que défini ci-dessus, sous forme neutralisée,
d. éventuellement recristallisation dudit mélange de calixarènes géants lavé, dans un solvant à base de DMSO, pour obtenir un mélange de calixarènes géants purifié dans lequel R est tel que défini ci-dessus, et substantiellement dépourvu en *p*-(R)calix[7]arène et/ou en *p*-(R)calix[8]arène.
e. éventuellement, purification dudit mélange de calixarènes géants purifié dépourvu en *p*-(R)calix[7]arène et/ou en *p*-(R)calix[8]arène par CPG, en séparant différentes fractions en fonction de leur temps d'élution.

Dans l'étape a, au bout de 10 à 25 minutes, il y a apparition des oligomères ou polymères de formule (III) qui conduisent ensuite, avec un chauffage plus prolongé, au dimère de formule (II).
Le chauffage est effectué à une température d'environ 100 à 130°C.
Avantageusement, le chauffage est d'environ 110°C, 120°C ou 130°C.
Si le dimère est le produit désiré, il est alors récupéré par filtration du milieu réactionnel.
Dans l'étape b., le chauffage est poursuivi à une température d'environ 100 à 130°C, si le dimère n'est pas le produit désiré, ce qui conduit audit précurseur sous forme de précipité. Avantageusement, le chauffage est poursuivi durant 1h, 2h ou 3h et à une température comprise de 100°C à 130°C, avantageusement à 110°C, 120°C ou 130°C.

Dans l'étape c, le précurseur solide sous forme de précipité, obtenu après neutralisation par un acide tel que HCl ou $H_2SO_4$, en particulier HCl dilué ou non, est constitué à environ 50 à 90% de calixarènes géants qui peuvent comprendre également d'autres sous produits tels que des oligomères linéaires résiduels ou du dimère résiduel, mais également du *p*-(R)calix[7]arène et/ou du *p*-(R)calix[8]arène.

Le lavage par un solvant polaire permet de purifier ledit précurseur et notamment d'éliminer certains oligomères linéaires résiduels ou le dimère résiduel.

Dans l'étape d., la recristallisation dans un mélange à base de DMSO permet d'éliminer le *p*-(R)calix[7]arène et/ou le *p*-(R)calix[8]arène.

Le mélange de solvants à base de DMSO est notamment un mélange de DMSO avec du toluène ou de l'acétone, le DMSO étant dans une proportion de 5% à 50% en volume dans le mélange de solvant, en particulier de 10 %.

Le DMSO peut être remplacé par un solvant aprotique polaire, de préférence le DMF.

L'étape e. est effectuée pour séparer le mélange de *p*-(R)calixarènes géants purifié dépourvu en *p*-(R)calix[7] arène et/ou en *p*-(R)calix[8]arène en fractions de *p*-(R)calixarènes géants de taille différente.

Dans un mode de réalisation avantageux, la base utilisée dans l'étape a. est $Ba(OH)_2$ et R représente l'octyloxy. Le mélange de p-(R)calixarènes géants obtenu contient les p-(octyloxy)calix[21-50]arènes et la taille déterminée par la gaussienne centrée en GPC est d'environ 33.

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, ledit précurseur solide sous forme de précipité étant précédé de la formation d'un dimère phénolique, éventuellement isolé, tel que défini ci-dessus,
dans lequel ledit dimère phénolique ou ledit précurseur solide sous forme de précipité, contenu dans ledit milieu réactionnel, est mis en présence de moyens de transmission de chaleur sous la forme d'un four ou d'un solvant dans lequel ledit précurseur solide sous forme de précipité et ledit dimère phénolique sont insolubles, notamment un alcane linéaire ou ramifié de point d'ébullition supérieur à 50°C, notamment supérieur à 100°C, en particulier l'octane, et est soumis à un traitement thermique.

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, ledit précurseur solide sous forme de précipité étant précédé de la formation d'un dimère phénolique, éventuellement isolé, tel que défini ci-dessus,
dans lequel ledit dimère phénolique ou ledit précurseur solide sous forme de précipité, contenu dans ledit milieu réactionnel, est mis en présence d'un solvant organique dans lequel ledit précurseur solide sous forme de précipité et ledit dimère phénolique sont insolubles, notamment un alcane linéaire ou ramifié de point d'ébullition supérieur à 50°C, notamment supérieur à 100°C, en particulier l'octane, et est soumis à un traitement thermique.

Le solvant organique peut être n'importe quel solvant organique à condition que, d'une part, il ne réagisse pas avec le précurseur sous forme de résine et que, d'autre part, ledit précurseur sous forme de résine soit insoluble.

Le point d'ébullition du solvant organique est notamment supérieur ou égale à 110°C.

Le traitement thermique est effectué au reflux dudit solvant organique, avec ou sans agitation, préférentiellement sans agitation du milieu réactionnel, durant un temps compris, en fonction de R et de la base, de 3 à 8h et a notamment pour effet, d'une part de compléter la formation des macrocycles, notamment ceux compris dans le précurseur solide sous forme de résine, mais également d'augmenter la taille des macrocycles obtenus aussi bien à partir du précurseur solide sous forme de résine que dudit dimère.

Les calixarènes géants obtenus comprennent cependant une proportion de calix[7]arène et/ou de calix[8]arène variable en fonction de R.

Plus le précurseur solide sous forme de résine ou le dimère est insoluble dans le solvant organique et plus la proportion de calixarènes géants augmente.

La durée du traitement thermique doit également être contrôlée et doit être comprise de 3 à 8h en fonction de R et de la base utilisée.

Au-delà de 8h, la proportion de petits calixarènes obtenus augmente jusqu'à n'obtenir que des petits calixarènes,

les calixarènes géants disparaissant en se dissociant au profit desdits petits calixarènes.

En fonction du solvant utilisé pour le traitement thermique, la taille des macrocycles obtenue peut être modulée.

Plus la température du milieu réactionnel est basse plus la taille des macrocycles diminue.

Dans un mode de réalisation avantageux, la présente invention concerne un procédé de préparation d'un mélange de *p*-(R)calixarènes géants dont la taille est supérieure à 20 notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, ledit précurseur solide sous forme de précipité étant précédé de la formation d'un dimère phénolique, éventuellement isolé, ledit dimère phénolique ou ledit précurseur solide sous forme de précipité, contenu dans ledit milieu réactionnel, étant mis en présence de moyens de transmission de chaleur sous la forme d'un four ou d'un solvant dans lequel ledit précurseur solide sous forme de précipité et ledit dimère phénolique sont insolubles, notamment un alcane linéaire ou ramifié de point d'ébullition supérieur à 50°C, notamment supérieur à 100°C, en particulier l'octane et est soumis à un traitement thermique, tel que défini ci-dessus, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I),
avec du formaldéhyde aqueux, pour constituer un milieu réactionnel et chauffage dudit milieu réactionnel durant un temps compris de 30 minutes à 1 heure, en particulier 1 heure, sans élimination de l'eau formée, pour obtenir un dimère de formule (II) tel que défini ci-dessus,
b. éventuellement poursuite du chauffage dudit milieu réactionnel contenant ledit dimère sans élimination de l'eau formée, durant 2 à 3 heures, pour obtenir un milieu réactionnel contenant un précurseur solide sous forme de précipité, et optionnellement filtration dudit précurseur solide sous forme de précipité,
c. chauffage dudit milieu réactionnel à l'aide de moyens de transmission de chaleur sous la forme d'un four, ou addition audit milieu réactionnel contenant ledit précurseur solide sous forme de précipité d'un solvant organique dans lequel ledit précurseur solide sous forme de précipité est insoluble, notamment un alcane linéaire ou ramifié de point d'ébullition supérieur à 50°C, notamment supérieur à 100°C, en particulier l'octane, pour obtenir un milieu réactionnel contenant un solide sous forme de précipité dans un four ou un solvant et chauffage dudit milieu réactionnel contenant un solide sous forme de précipité, durant 3 à 8 heures,
pour obtenir un mélange de calixarènes géants dans lequel R est tel que défini ci-dessus, comprenant de plus un *p*-(R)calix[7]arène et/ou un *p*-(R)calix[8]arène,
d. recristallisation, après neutralisation de la base, dans un solvant à base de DMSO, pour obtenir un mélange de calixarènes géants purifié dans lequel R est tel que défini ci-dessus, substantiellement dépourvu de *p*-(R)calix[7]arène et/ou un *p*-(R)calix[8]arène,
e. éventuellement, purification dudit mélange de calixarènes géants purifié dépourvu en *p*-(R)calix[7]arène et/ou en *p*-(R)calix[8]arène par CPG, en séparant différentes fractions en fonction de leur temps d'élution.

Dans un mode de réalisation avantageux, la présente invention concerne un procédé de préparation d'un mélange de *p*-(R)calixarènes géants dont la taille est supérieure à 20 notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, ledit précurseur solide sous forme de précipité étant précédé de la formation d'un dimère phénolique, éventuellement isolé, ledit dimère phénolique ou ledit précurseur solide sous forme de précipité, contenu dans ledit milieu réactionnel, étant mis en présence d'un solvant organique dans lequel ledit précurseur solide sous forme de précipité et ledit dimère phénolique sont insolubles, notamment un alcane linéaire ou ramifié de point d'ébullition supérieur à 50°C, notamment supérieur à 100°C, en particulier l'octane et est soumis à un traitement thermique, tel que défini ci-dessus, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I),
avec du formaldéhyde aqueux, pour constituer un milieu réactionnel et chauffage dudit milieu réactionnel durant un temps compris de 30 minutes à 1 heure, en particulier 1 heure, sans élimination de l'eau formée, pour obtenir un

dimère de formule (II) tel que défini ci-dessus,

b. éventuellement poursuite du chauffage dudit milieu réactionnel contenant ledit dimère sans élimination de l'eau formée, durant 2 à 3 heures, pour obtenir un milieu réactionnel contenant un précurseur solide sous forme de précipité, et optionnellement filtration dudit précurseur solide sous forme de précipité,

c. addition audit milieu réactionnel contenant ledit précurseur solide sous forme de précipité d'un solvant organique dans lequel ledit précurseur solide sous forme de précipité est insoluble, notamment un alcane linéaire ou ramifié de point d'ébullition supérieur à 50°C, notamment supérieur à 100°C, en particulier l'octane, pour obtenir un milieu réactionnel contenant un solide sous forme de précipité dans un solvant et chauffage dudit milieu réactionnel contenant un solide sous forme de précipité, durant 3 à 8 heures,

pour obtenir un mélange de calixarènes géants dans lequel R est tel que défini ci-dessus, comprenant de plus un *p*-(R)calix[7]arène et/ou un *p*-(R)calix[8]arène,

d. recristallisation, après neutralisation de la base, dans un solvant à base de DMSO, pour obtenir un mélange de calixarènes géants purifié dans lequel R est tel que défini ci-dessus, substantiellement dépourvu de *p*-(R)calix[7]arène et/ou un *p*-(R)calix[8]arène,

éventuellement, purification dudit mélange de calixarènes géants purifié dépourvu en *p*-(R)calix[7]arène et/ou en *p*-(R)calix[8]arène par CPG, en séparant différentes fractions en fonction de leur temps d'élution,
et la taille déterminée par la gaussienne centrée en GPC est d'environ 21 à 50.

Dans l'étape a., au bout de 10 à 25 minutes, il y a apparition des oligomères ou polymères de formule (III) qui conduisent ensuite, avec un chauffage plus prolongé, au dimère de formule (II).

Le chauffage est effectué à une température d'environ 100 à 130°C durant 30 minutes à 2h à partir du milieu réactionnel initial pour former le dimère de formule (II).

Avantageusement, le chauffage est d'environ 110°C, 120°C ou 130°C.

Si le dimère est le produit désiré, il est alors récupéré par filtration du milieu réactionnel.

Dans l'étape b., le chauffage est poursuivi à une température d'environ 100 à 130°C, si le dimère n'est pas le produit désiré, ce qui conduit audit précurseur sous forme de résine dure cassante. Avantageusement, le chauffage est poursuivi durant 1h, 2h ou 3h et à une température comprise de 100°C à 130°C, avantageusement à 110°C, 120°C ou 130°C.

Le mélange obtenu dans l'étape b. est constitué d'environ 50% de *p*-(R)calixarènes géants et peut être isolé par simple filtration.

Dans l'étape c, soit le solvant est introduit directement dans le milieu réactionnel de l'étape précédente b., soit ledit précurseur solide sous forme de résine dure cassante est introduit dans un appareil pour effectuer une réaction et le solvant organique dans lequel ledit précurseur solide sous forme de résine dure cassante est insoluble est introduit constituant, ainsi le milieu réactionnel.

Le traitement thermique est effectué au reflux dudit solvant organique, avec ou sans agitation, préférentiellement sans agitation du milieu réactionnel.

Le traitement thermique permet de compléter la formation des macrocycles et d'augmenter la taille de *p*-(R)calixarènes géants.

Avantageusement, le traitement thermique est compris d'environ 3 à 4h.

Avantageusement, lorsque le traitement thermique est compris de 3 à 4h, la base utilisée est LiOH, KOH, NaOH, CsOH ou RbOH.

Avantageusement, le traitement thermique est compris d'environ 6 à 8h, en particulier 7h.

Avantageusement, lorsque le traitement thermique est compris d'environ 6 à 8h, en particulier 7h, la base utilisée est $Ba(OH)_2$.

Dans l'étape d., le mélange de solvants à base de DMSO est notamment un mélange de DMSO avec du toluène ou de l'acétone, le DMSO étant dans une proportion de 5% à 50% en volume dans le mélange de solvant, en particulier de 10 %.

Le DMSO peut être remplacé par un solvant aprotique polaire, de préférence le DMF.

L'étape e. est effectuée pour séparer le mélange de *p*-(R)calixarènes géants purifié dépourvu de *p*-(R)calix[7]arène et/ou de *p*-(R)calix[8]arène en fractions de *p*-(R)calixarènes géants de taille différente.

Dans un mode de réalisation avantageux, la base utilisée dans l'étape a. est $Ba(OH)_2$, LiOH, KOH ou RbOH et R représente le benzyloxy.

Le mélange de p-(R)calixarènes géants obtenu est du *p*-(benzyloxy)calix[21-50]arènes et la taille déterminée par la gaussienne en GPC centrée est d'environ 30.

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde

de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, ledit précurseur solide sous forme de précipité étant précédé de la formation d'un dimère phénolique, éventuellement isolé, tel que défini ci-dessus,
dans lequel ledit dimère phénolique ou ledit précurseur solide sous forme de précipité, contenu dans ledit milieu réactionnel, est mis en présence d'un solvant organique dans lequel ledit précurseur solide sous forme de précipité et ledit dimère phénolique sont au moins partiellement solubles, en particulier dans le xylène, le toluène, le DMSO ou le DMF, et est soumis à un traitement thermique.

Lorsque le précurseur solide sous forme de précipité ou le dimère sont dissouts pour l'étape de traitement thermique, les macrocycles obtenus sont de pureté supérieure par rapport au procédé consistant à la non dissolution du précurseur solide sous forme de précipité ou du dimère dans le solvant organique, cependant, la proportion de petits calixarènes présente et notamment de calix[7]arène et/ou de calix[8]arène est supérieure.

Le traitement thermique est effectué au reflux dudit solvant organique, avec ou sans agitation, préférentiellement sans agitation du milieu réactionnel.

La durée du traitement thermique doit également être contrôlée et doit être comprise de 3 à 8h en fonction de R et de la base utilisée, de la concentration en base, de la température.

Au-delà de 8h, la proportion de petits calixarènes obtenus augmente jusqu'à n'obtenir que des petits calixarènes, les calixarènes géants disparaissant en se dissociant au profit desdits petits calixarènes.

En fonction du solvant utilisé pour le traitement thermique avec dissolution, la taille des macrocycles obtenue peut être modulée.

Plus la température est basse plus la taille des macrocycles obtenus diminue.

Le mélange de *p*-(R)calixarènes géants obtenu est constitué de *p*-(benzyloxy)calix[n]arènes et la taille déterminée par GPC (gaussienne centrée) est d'environ 22 motifs de répétition.

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, ledit précurseur solide sous forme de précipité étant précédé de la formation d'un dimère phénolique, éventuellement isolé, ledit dimère phénolique ou ledit précurseur solide sous forme de précipité, contenu dans ledit milieu réactionnel, étant mis en présence d'un solvant organique dans lequel ledit précurseur solide sous forme de précipité et ledit dimère phénolique sont au moins partiellement solubles, en particulier dans le xylène, le toluène, le DMSO ou le DMF, et étant soumis à un traitement thermique, tel que défini ci-dessus, comprenant les étapes suivantes :

a. mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I),
avec du formaldéhyde aqueux, pour constituer un milieu réactionnel et chauffage dudit milieu réactionnel durant un temps compris de 30 minutes à 2 heures, en particulier 1 heure, sans élimination de l'eau formée,
pour obtenir un dimère de formule (II) tel que défini ci-dessus,
b. éventuellement poursuite du chauffage dudit milieu réactionnel contenant ledit dimère sans élimination de l'eau formée, durant 2 à 3 heures, pour obtenir un milieu réactionnel contenant un précurseur solide sous forme de précipité, et optionnellement filtration dudit précurseur solide sous forme de précipité,
c. addition audit milieu réactionnel contenant ledit précurseur solide sous forme de précipité d'un solvant organique dans lequel ledit précurseur solide sous forme de précipité est au moins partiellement soluble, en particulier le xylène, le toluène, le DMSO ou le DMF, pour obtenir un milieu réactionnel contenant un solide sous forme de précipité en suspension dans un solvant organique, et chauffage dudit milieu réactionnel contenant un solide sous forme de précipité, dissout, durant 3 à 8 heures,
pour obtenir un mélange de calixarènes géants dans lequel R est tel que défini ci-dessus, comprenant de plus un *p*-(R)calix[7]arène et/ou un *p*-(R)calix[8]arène,
d. recristallisation dudit mélange de calixarènes géants, après neutralisation de la base, dans un solvant à base de DMSO, pour obtenir un mélange de calixarènes géants purifié dans lequel R est tel que défini ci-dessus, substantiellement dépourvu de *p*-(R)calix[7]arène et/ou un *p*-(R)calix[8]arène,
e. éventuellement purification par CPG, en séparant différentes fractions en fonction de leur temps d'élution.

Dans l'étape a., au bout de 10 à 25 minutes, il y a apparition des oligomères ou polymères de formule (III) qui conduisent ensuite, avec un chauffage plus prolongé, au dimère de formule (II).

Le chauffage est effectué à une température d'environ 100 à 130°C durant 30 minutes à 2 heures à partir du milieu réactionnel initial pour former le dimère de formule (II)

Avantageusement, le chauffage est d'environ 110°C, 120°C ou 130°C.

Si le dimère est le produit désiré, il est alors récupéré par filtration du milieu réactionnel.

Dans l'étape b., le chauffage est poursuivi à une température d'environ 100 à 130°C, si le dimère n'est pas le produit désiré, ce qui conduit audit précurseur sous forme de résine dure cassante. Avantageusement, le chauffage est poursuivi durant 1h, 2h ou 3h et à une température comprise de 100°C à 130°C, avantageusement à 110°C, 120°C ou 130°C.

Le mélange obtenu dans l'étape b. est constitué à environ 50 à 90% de *p*-(R)calixarènes géants et peut être isolé par simple filtration.

Dans l'étape c., le traitement thermique permet de compléter la formation des macrocycles et d'augmenter la taille de *p*-(R)calixarènes géants.

Le traitement thermique est effectué au reflux dudit solvant organique, avec ou sans agitation, préférentiellement sans agitation du milieu réactionnel.

Avantageusement, le traitement thermique est compris d'environ 3 à 4h.

Avantageusement, lorsque le traitement thermique est compris de 3 à 4h, la base utilisée est LiOH, KOH, NaOH, CsOH ou RbOH.

Avantageusement, le traitement thermique est compris d'environ 6 à 8h, en particulier 7h.

Avantageusement, lorsque le traitement thermique est compris d'environ 6 à 8h, en particulier 7h, la base utilisée est $Ba(OH)_2$.

Dans l'étape d., le mélange de solvants à base de DMSO est notamment un mélange de DMSO avec du toluène ou de l'acétone, le DMSO étant dans une proportion de 5% à 50% en volume dans le mélange de solvant, en particulier de 10 %.

Le DMSO peut être remplacé par un solvant aprotique polaire, de préférence le DMF.

L'étape e. est effectuée pour séparer le mélange de *p*-(R)calixarènes géants purifié dépourvu en *p*-(R)calix[7] arène et/ou en *p*-(R)calix[8]arène en fractions de *p*-(R)calixarènes géants de taille différente.

Dans un mode de réalisation avantageux, la base utilisée dans l'étape a. est $Ba(OH)_2$, LiOH, KOH, NaOH, CsOH ou RbOH et R représente le benzyloxy.

Le mélange de *p*-(R)calixarènes géants obtenu est du *p*-(benzyloxy)calix[21-30]arènes et la taille déterminée par la gaussienne centrée en GPC est d'environ 22 .

Dans un mode de réalisation avantageux, la présente invention concerne un procédé de préparation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, comprenant une étape de mise en contact d'au moins une base, en particulier choisie parmi l'hydroxyde de baryum, l'hydroxyde de rubidium, l'hydroxyde de lithium, l'hydroxyde de césium, la potasse ou la soude, avec au moins un phénol substitué en position 4 de formule (I), l'eau produite lors de la réaction étant conservée dans ledit milieu réactionnel durant ledit chauffage, un précurseur solide sous forme de précipité, éventuellement isolé, étant obtenu, tel que défini ci-dessus,

dans lequel ledit dimère phénolique est isolé puis soumis à un traitement thermique comme décrit précédemment, et à une recristallisation tels que définis dans les étapes c. et d. ci-dessus.

Lorsque le dimère est isolé sous forme neutre, une base, en particulier la base ayant été utilisée pour l'obtention dudit dimère, plus particulièrement à la concentration employée pour l'obtention dudit dimère, est ajoutée préalablement au traitement thermique.

Lorsque le dimère est isolé sous forme de sel, l'ajout d'une base n'est pas nécessaire.

Ledit dimère isolé est donc mis en présence d'un solvant organique, en particulier un solvant organique dans lequel ledit dimère phénolique est soluble ou insoluble, tel que défini ci-dessus, et est soumis au traitement thermique comme ci-dessus.

Dans un mode de réalisation avantageux, la présente invention concerne un procédé de préparation d'un mélange de *p*-(R)calixarènes géants, l'eau produite lors de la réaction étant conservée dans ledit milieu réactionnel durant ledit chauffage, tel que défini ci-dessus,

dans lequel ledit précurseur solide sous forme de précipité, précédé de la formation d'un dimère phénolique, est isolé et mis en présence d'un solvant organique dans lequel ledit précurseur solide sous forme de précipité est insoluble, puis soumis à un traitement thermique et à une recristallisation tels que définis dans les étapes c. et d. ci-dessus.

Lorsque le précurseur solide est isolé sous forme neutre, une base, en particulier la base ayant été utilisée pour l'obtention dudit dimère, plus particulièrement à la concentration employée pour l'obtention dudit dimère, est ajoutée préalablement au traitement thermique.

Lorsque le précurseur est isolé sous forme de sel, l'ajout d'une base n'est pas nécessaire.

Ledit précurseur solide sous forme de précipité isolé est donc mis en présence d'un solvant organique, en particulier un solvant organique dans lequel ledit précurseur solide sous forme de précipité isolé est soluble ou insoluble, tel que défini ci-dessus, et est soumis au traitement thermique comme ci-dessus.

Il faut noter que le dimère de formule générale (II) ou les précurseurs solides sous forme de précipité ou de résine dure cassante de formule générale (III) peuvent également être préparés par des procédés autres que ceux de l'invention et bien connus de l'homme du métier et être utilisés pour la préparation d'un mélange de *p*-(R)calixarènes géants selon l'invention.

Selon un autre aspect, la présente invention concerne l'utilisation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, encore plus particulièrement de 21 à environ 100, R étant tel que défini ci-dessus, pour la constitution d'un matériau ou dans le cadre du renfort des matériaux.

Les calixarènes de l'invention peuvent également être utilisés pour le piégeage des particules, la synthèse « template » de nanoparticules, la nanofiltration, la filtration de gaz, la complexation d'ions, l'encapsulation de molécules et l'administration de médicaments (« drug delivery ») de par la présence de la cavité importante présente au coeur des calixarènes géants.

La cavité importante présente au coeur des calixarènes géants permet également d'envisager la formation de matériaux réticulés poreux ayant des applications dans les gels ou les mousses.

Ils peuvent aussi servir d'agent de perforation de la couche lipidique en raison de la possibilité offerte de concentrer certains types de fonctions par greffage sur la couronne macrocyclique et de leur configuration cyclique.

Le greffage d'agents hydrophiles sur les hydroxyles disponibles peut conduire à la formation de micelles, compte-tenu du caractère amphiphile marqué des motifs ainsi obtenus et de la structure des calixarènes géants.

Les calixarènes géants amphiphiles contenant notamment des contre-ions cationiques de taille importantes peuvent servir à la vectorisation c'est-à-dire à insérer des principes actifs dans une cellule.

Il est également possible de greffer un ou plusieurs types d'amorceurs de polymérisation sur chaque couronne des calixarènes, de façon sélective, afin de constituer des polymères à architecture complexe permettant d'accroitre leur caractère amphiphile, d'améliorer l'encapsulation de principe actif et leur dispersion en milieu physiologique, de générer des propriétés nouvelles au niveau des matériaux relatifs, par exemple.

Dans un mode de réalisation avantageux, la présente invention concerne l'utilisation d'un mélange de *p*-(R)calixarènes géants dont la taille est supérieure à 20, notamment de 21 à plus de 220, notamment de 21 à 220, en particulier de 21 à 212, plus particulièrement de 21 à environ 200, R étant tel que défini ci-dessus, pour la constitution d'un matériau ou dans le cadre du renfort des matériaux, dans laquelle ledit mélange comprend de plus un *p*-(R)calix[7]arène et/ou un *p*-(R)calix[8]arène.

Selon un autre aspect, la présente invention concerne l'utilisation d'un mélange de calixarènes tel que défini ci-dessus, susceptible d'être obtenu par l'un des procédés définis ci-dessus, pour la constitution d'un matériau ou dans le cadre du renfort des matériaux.

Dans un mode de réalisation avantageux, la présente invention concerne l'utilisation d'un mélange de calixarènes tel que défini ci-dessus, susceptible d'être obtenu par l'un des procédés définis ci-dessus, pour la constitution d'un matériau ou dans le cadre du renfort des matériaux, dans laquelle ledit mélange comprend de plus un *p*-(R)calix[7]arène et/ou un *p*-(R)calix[8]arène.

## DESCRIPTION DES FIGURES

La figure 1 présente la courbe d'étalonnage obtenue avec les échantillons purs de *p*-(benzyloxy)calix[4-16]arènes en RMN diffusionnelle synthétisés selon l'exemple 5. Elle permet de valider la masse molaire des *p*-(benzyloxy)calix[4-16]arènes purs déterminée par chromatographie d'exclusion stérique. Axe des abscisses : ln(M) où M représente le poids moléculaire des *p*-(benzyloxy)calix[4-16]arènes.

Axe des ordonnées : ln(D) où D représente le coefficient d'autodiffusion des molécules qui dépend entre autre de leur volume hydrodynamique déterminé par RMN diffusionnelle PGSE (Pulse Gradient Spin Echo).

Cette courbe permet de relier le coefficient d'autodiffusion à la masse molaire moyenne d'un mélange de calixarènes.

La figure 2 présente la chromatographie d'exclusion stérique des échantillons purs de référence de *p*-(benzyloxy)calix[6]arène, *p*-(benzyloxy)calix[7]arène, *p*-(benzyloxy)calix[8]arène, *p*-(benzyloxy)calix[10]arène, *p*-(benzyloxy)calix[12]arène et *p*-(benzyloxy)calix[16]arène,

Cette technique permet de séparer les molécules en fonction de leur volume hydrodynamique et d'estimer la masse molaire d'échantillons purs ou en mélange à partir d'une gamme d'étalons purs. Les masses molaires représentatives d'un calixarène pur ou d'un mélange de calixarènes sont traduites par des « masses molaires au pic » (Mp) et des

« masses molaires moyennes », renseignant respectivement sur la population majoritaire et sur la moyenne des populations.

En abscisse : Volume d'élution : Ve(ml)

En ordonnée : Signal RI, correspondant à l'indice de réfraction d'échantillons ou d'étalons en solution sortant de la colonne de chromatographie.

La figure 3 présente l'utilisation des calixarènes géants en tant que renfort de matériaux par insertion de polymères dans la cavité de ceux-ci, ce qui permet de mieux répartir une contrainte en son sein et également de ralentir la propagation de fissures (amorces à la rupture).

La figure 4 présente une analyse RMN du solide microcristallin obtenu à l'issue de la recristallisation présentée dans l'exemple 1a).

La figure 5a présente une analyse RMN du solide obtenu après le procédé de reflux sans agitation présenté dans l'exemple 1b).

La figure 5b présente une analyse RMN du solide obtenu après la recristallisation présentée dans l'exemple 1b).

La figure 6 présente une analyse RMN du solide microcristallin obtenu à l'issue de la recristallisation présentée dans l'exemple 1d)A.

La figure 7 présente une mesure de masse molaire par Gel Permeation Chromatography (GPC) du solide microcristallin obtenu à l'issue de la recristallisation présentée dans l'exemple 1d)A.

La figure 8 présente une analyse RMN du précipité microcristallin obtenu à l'issue de la recristallisation présentée dans l'exemple 1d)B.

La figure 9 présente une mesure de masse molaire par GPC du précipité microcristallin obtenu à l'issue de la recristallisation présentée dans l'exemple 1d)B.

La figure 10a présente une analyse RMN du solide majoritairement constitué du dimère, obtenu en tant qu'intermédiaire réactionnel dans l'exemple 1d)C.

La figure 10b présente une analyse RMN du solide obtenu après reflux, dans l'exemple 1d)C.

La figure 10c présente une analyse RMN 1H du calix[80]arène décrit dans l'exemple 1d)C-2.

La figure 11 présente une analyse RMN du solide microcristallin obtenu à l'issue de la recristallisation présentée dans l'exemple 1e)A.

La figure 12 présente une mesure de masse molaire par GPC du solide microcristallin obtenu à l'issue de la recristallisation présentée dans l'exemple 1e)A.

La figure 13 présente une analyse RMN du solide obtenu à l'issue de la recristallisation présentée dans l'exemple 1e)B.

La figure 14 présente une mesure de masse molaire par GPC du solide obtenu à l'issue de la recristallisation présentée dans l'exemple 1e)B.

La figure 15 présente une analyse RMN du solide blanc obtenu à l'issue de la réaction présentée dans l'exemple 1g).

La figure 16 présente une analyse RMN du solide blanc obtenu à l'issue de la réaction présentée dans l'exemple 2a).

La figure 17 présente une analyse RMN du solide obtenu, avant l'ajout de toluène, dans l'exemple 4 (figure 17.1) ainsi que le spectre RMN de référence du *p*-(tBu)calix[8]arène (figure 17.2).

La figure 18 présente une analyse RMN du solide obtenu après reflux et neutralisation, dans l'exemple 4.

La figure 19 présente une analyse RMN de la solution limpide orangée obtenue dans l'exemple 5.

La figure 20 présente le schéma de purification d'un mélange de grands calixarènes obtenu dans l'exemple 5, après l'étape de cristallisation dans le mélange acétone-DMSO (10% en volume de DMSO). Les abréviations PX et FX correspondent respectivement au précipité de la fraction X et au filtrat de la fraction X. Les fractions en gras correspondent à des calixarènes purs isolés.

Chaque lettre en italique correspond à une étape de purification, réalisée par ordre chronologique dans les conditions suivantes :

*A* : Le produit brut contenant le mélange de calixarènes est mis sous agitation dans l'acétate d'éthyle puis filtré pour obtenir les fractions P0 et F0.

*B* : Le précipité P0 est chauffé à reflux 1h sous argon et sous agitation dans 450 mL de chlorobenzène puis filtré pour obtenir les fractions P1 et F1.

*C* : Le précipité P1 est placé vigoureusement sous agitation dans 40 mL de THF pendant une nuit, filtré et abondamment rincé au THF pour obtenir les fractions P8 (640 mg, Mp correspond à 20 motifs) et F8.

*D* : F1 est chauffé à reflux sous argon et sous agitation dans 75 mL de chlorobenzène puis refroidit et agité 1h à température ambiante. La solution est placée une nuit à -20°C et filtrée pour obtenir les fractions P2 et F2.

*E* : P2 est placé une nuit sous agitation dans 20mL de dichlorométhane à température ambiante pour obtenir les fractions F3et P3 (40 mg, mélange de calixarènes dont les Mp correspondent à 22, 11 et 8 motifs).

*F* : F3 est dissout dans 40 mL de toluène à reflux puis refroidit à température ambiante. Après 4h sous agitation à température ambiante, la suspension est filtrée pour obtenir F5 (428 mg, mélange de calixarène dont les Mp

correspondent à 11 et 17 motifs) et P5 (15 mg).

*G* : F2 est dissout à chaud dans du toluène et déposé sur une colonne de silice. Le mélange de calixarène est élué dans un premier temps dans du dichlorométhane pour obtenir les fractions F2a et F2b, puis dans du tetrahydrofurane pour obtenir la fraction Fc.

*H* : On ajoute 15 mL de DCM sur la fraction F2a et un précipité blanc apparait immédiatement. On laisse reposer 1h sans agitation et on filtre la suspension pour obtenir P4 (40 mg, calix[16]arène pur) et F4.

*I* : F4 est chauffé brièvement à reflux du toluène (20 mL) jusqu'à dissolution totale puis placé 24h à température ambiante sans agitation. On obtient une suspension qui est filtrée pour obtenir les fractions F6 et P6 (30 mg, mélange de grands calixarènes).

*J :* F6 est dissout dans le toluène puis déposé sur une colonne de silice. La chromatographie est effectuée avec un gradient d'éluant 70/30 à 100/0 de dichlorométhane/toluène. On isole, par ordre d'élution, du calix[7]arène (30 mg), du calix[9]arène et un mélange de calix[10+12]arènes (fraction F6c).

*K* : F6c est chauffé brièvement à reflux du toluène (6 mL) jusqu'à dissolution totale puis placée 48h à température ambiante sans agitation. On obtient une suspension que l'on dilue dans 20 mL de toluène. La suspension est filtrée pour obtenir P7 (44 mg, calix[10]arène pur) et F7.

*L* : F2b est dissoute dans 10 mL de toluène. Un précipité blanc apparait rapidement et la suspension est stockée 5 jours sans agitation. On rajoute 25 mL de toluène et, après 2 semaines sans agitation, la suspension est filtrée pour obtenir P9 (88 mg, calix[10]arène pur) et F9.

*M* : F7 et F9 sont rassemblés, dispersés dans 150 mL d'acétone puis chauffé 1h à reflux du solvant. La suspension est filtrée pour obtenir P10 (688 mg, calix[12]arène pur) et F5 (75 mg, mélange de calix[10]arène et d'autres grands calixarènes).

*N :* F2c est placé 24h sous agitation dans l'acétone à température ambiante puis filtrée pour obtenir un précipité. Ce précipité est soumis à trois cycles de lavages/filtrations dans l'acétone. On obtient P11 (512 mg) et, après assemblage des filtrats, F11 (1.43g).

*O* : P11 est placé sous agitation dans 25 mL de dichlorométhane pendant un weekend à température ambiante. On obtient P12 (50 mg, mélange de calixarènes dont Mp correspond à 21 motifs) et F12 (547 mg, deux populations dont les Mp correspondent à 19 et 30 motifs).

*P* : F8 et P13 sont assemblés et placés 24h sous agitation dans l'acétone pour obtenir P14 (790 mg, deux populations dont les Mp correspondent à 19 et 12 motifs) et F14 (92 mg, deux populations dont les Mp correspondent à 17 et 11 motifs).

*Q* : F11 est mélangé à 50 mL d'acétonitrile. Après chauffage pendant 15 min à reflux du solvant, une sorte d'huile noire visqueuse se dépose sur les parois du ballon et le surnageant est immédiatement séparé. Cette opération est répétée et on obtient P15 (696.5 mg, mélange de calixarènes dont Mp correspond à 34 motifs) et, après assemblage des filtrats, F15 (425,6 mg).

*R* : F15 est placé sous agitation pendant 24h dans 50 mL d'acétonitrile à température ambiante. On obtient F16 (30 mg, calix[13]arène pur) et P16 (401 mg, deux populations dont les Mp correspondent à 18 et 12 motifs).

La figure 21 présente l'ensemble des chromatographies d'exclusion stérique réalisées sur les mélanges de grands calixarènes issus du schéma de purification présenté en figure 3. Chaque chromatogramme inclue le calix[16]arène en référence.

La figure 22 présente les spectres de masse Maldi-Tof des échantillons purs de *p*-(benzyloxy)calix[9]arène, *p*-(benzyloxy)calix[10]arène, *p*-(benzyloxy)calix[12]arène et *p*-(benzyloxy)calix[16]arène. Cette technique permet de déterminer avec précision la masse molaire d'un composé afin de remonter à sa composition chimique.

La figure 23 présente les spectres RMN des échantillons purs de *p*-(benzyloxy)calix[9]arène, *p*-(benzyloxy)calix[10] arène, *p*-(benzyloxy)calix[12]arène, *p*-(benzyloxy)calix[13]arène et *p*-(benzyloxy)calix[16]arène.

La figure 24 présente le chromatogramme GPC du macroamorceur obtenu à partir du calix[50]arène (exemple C).

La figure 25 présente le chromatogramme GPC des polymères en étoile obtenus à partir du calix[50]arène, lesdits polymères différant par la durée du procédé ATRP .

La figure 26 présente la caractérisation des structures des polymères en étoile, en exprimant log Rh où Rh correspond au rayon hydrodynamique du polymère étudié, en fonction de log Mw où Mw est la masse molaire du polymère.

La figure 27 présente une analyse RMN 1H du solide obtenu à l'exemple 1d)D.

La figure 28 présente une analyse par spectrométrie de masse « MALDI » des calixarènes géants obtenus en utilisant LiOH 0,4 équivalents par rapport au phénol (exemple 1d)D). Les valeurs indiquées correspondent au nombre d'unités phénoliques. Les calixarènes apparaissent majoritairement cationisés par du potassium (cationisation par le sodium observée minoritairement). Les pics à M-91 correspondent à des réactions de débenzylation des calixarènes induites par le faisceau laser (pendant l'analyse).

La figure 29a présente une analyse RMN 1H du mélange de calixarènes géants purifié obtenu en utilisant CsOH 0,4 équivalents (exemple 1d)E).

La figure 29b présente une analyse RMN 13C (63 MHz) du mélange de calixarènes géants purifié obtenu en utilisant CsOH 0,4 équivalents (exemple 1d)E).

**EXEMPLES**

**A) synthèse des calixarènes géants**

**Exemple 1 : Préparation de *p*-(benzyloxy)calixarènes géants**

**a) par réaction du benzyloxyphénol avec une base et du formaldéhyde dans l'eau et élimination de l'eau. Précurseur solide sous forme de résine cassante isolée.**

Un ballon tricol de 2 litres équipé d'un agitateur mécanique, d'un collecteur de Dean-Stark et d'un réfrigérant ainsi que d'un chauffage par bain d'huile, est chargé avec 104g de 4-(benzyloxy)phénol (0,519 mol) et 135 ml de formaldéhyde en solution aqueuse à 30%.

La suspension résultante est chauffée à 140°C affichés sous protection d'argon.

A 90°C, on injecte rapidement une solution de 12g de KOH (0,214 mol ; 0,41 équivalent) dans 10ml d'eau, tout en poursuivant le chauffage. La suspension blanche se transforme en une solution limpide jaune vif. Cette solution est maintenue à reflux pendant 1h.

L'un des rodages du ballon est alors équipé d'un col de cygne relié à un bulleur, et la solution est alors soumise à un fort balayage d'argon (≈20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout d'1h.

La résine solide dure et cassante obtenue est lavée avec un mélange de 750ml de méthanol, acidifiée avec 50 ml d'HCl en solution aqueuse à 37%. Le précurseur est dispersé sous forte agitation mécanique.

La suspension résultante est filtrée.

Une analyse RMN montre que ce précurseur ainsi isolé apparaît être constitué de 50% de calixarènes géants. L'impureté principale est le dimère, plus un faible pourcentage d'oligomères linéaires.

Ce précurseur est dissout dans 1l d'un mélange acétone/DMSO (90 : 10 en volume) et mis à cristalliser au réfrigérateur (1°C/4 jours). On récupère 20g d'un solide microcristallin, dont l'analyse RMN est présentée à la figure 4. Ce solide comporte environ 80% de calixarènes géants.

Mesure de masse molaire par GPC : 4600g/mol, nombre de motifs de répétition = 22.

Mesure du rayon hydrodynamique des calixarènes formés par diffusion dynamique de la lumière (DDL) : 4nm.

**b) par réaction du benzyloxyphénol avec une base et du formaldéhyde dans l'eau et élimination de l'eau puis traitement thermique du précurseur solide sous forme de résine cassante non isolé dans l'octane au reflux**

Un ballon tricol de 2 litres équipé d'un agitateur mécanique, d'un collecteur de Dean-Stark et d'un réfrigérant ainsi que d'un chauffage par bain d'huile, est chargé avec 104g de 4-(benzyloxy)phénol (0,519 mol) et 135 ml de formaldéhyde en solution aqueuse à 30%.

La suspension résultante est chauffée à 140°C affichés sous protection d'argon.

A 90°C, on injecte rapidement une solution de 12g de KOH (0,214 mol ; 0,41 équivalent) dans 10ml d'eau, tout en poursuivant le chauffage. La suspension blanche se transforme en une solution limpide jaune vif. Cette solution est maintenue à reflux pendant 1h.

L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout d'1h.

Cette résine solide est ensuite additionnée de 300 ml d'octane et le système est mis au reflux sans agitation pendant 3h30. L'analyse RMN du solide obtenu est présentée à la figure 5a.

Ce solide apparaît être constitué à 60% de calixarènes géants.

Ce solide est dissout dans 2l d'un mélange d'un mélange acétone/DMSO (90 : 10 en volume) et mis à cristalliser au réfrigérateur (1°C/4 jours). On récupère 32g de calixarènes géants. La RMN (DMSO)-d6 de ce produit est présentée en figure 5b. Rendement : 30%

Mesure du rayon hydrodynamique des calixarènes formés par diffusion DDL : 4,5 nm. L'estimation du nombre de motifs de répétition est n = 30.

**c) par réaction du benzyloxyphénol avec une base et du formaldéhyde dans l'eau et élimination de l'eau puis traitement thermique du précurseur solide sous forme de résine cassante non isolé dans une huile de silicone (140°C).**

Dans un ballon de Schlenk de 250ml, un échantillon de 10 g du précurseur solide (non neutralisé) obtenu à l'exemple 1b (KOH 0,4 équivalent) est plongé dans 100ml d'huile de silicone, et chauffé 8h à 140°C. Une analyse RMN proton montre que le solide résultant est constitué à 68% de calixarènes géants.

**c-2) par réaction du benzyloxyphénol avec une base et du formaldéhyde dans l'eau et élimination de l'eau puis traitement thermique du précurseur solide sous forme de résine cassante non isolé dans un four (140°C).**

Un échantillon de 10 g du précurseur solide (non neutralisé) obtenu à l'exemple 1.d)C (KOH 1 équivalent) est placé dans un tube de Schlenk et dégazé sous vide primaire pendant 3h, pour éliminer les résidus volatils (eau, formaldéhyde résiduel).
Ce produit est ensuite placé dans un four à 140°C pendant 22h. Une analyse RMN proton montre que le solide résultant est constitué à 60% de calixarènes géants.

**d) par réaction du benzyloxyphénol avec une base et du formaldéhyde dans l'eau et élimination de l'eau puis traitement thermique du précurseur solide sous forme de résine cassante non isolé dans le xylène ou toluène au reflux avec agitation.**

**Exemple A : KOH (0,4 équivalent par rapport au phénol)**

Un ballon tricol de 2 litres équipé d'un agitateur mécanique, d'un collecteur de Dean-Stark et d'un réfrigérant ainsi que d'un chauffage par bain d'huile, est chargé avec 104g de 4-(benzyloxy)phénol (0,519 mol) et 135 ml de formaldéhyde en solution aqueuse à 30%.
La suspension résultante est chauffée à 140°C affichés sous protection d'argon.
A 90°C, on injecte rapidement une solution de 12g de KOH (0,214 mol ; 0,41 équivalent) dans 10ml d'eau, tout en poursuivant le chauffage. La suspension blanche se transforme en une solution limpide jaune vif. Cette solution est maintenue à reflux pendant 1h.
L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.
Au bout d'environ 40min de balayage, la solution initialement limpide et fluide devient de plus en plus visqueuse, pour finalement prendre en masse au bout d'1h.
On ajoute ensuite à ce solide 600ml de xylène, et la suspension résultante est portée au reflux sous forte agitation mécanique, de façon à bien redisperser le précurseur solide pendant cette durée.
Après neutralisation par un mélange de 500ml de THF/50 ml d'HCl 37% sous forte agitation.
Une filtration de la suspension obtenue permet de récupérer 40g de *p*-(benzyloxy)calix[8]arène.
Le filtrat est évaporé à sec. Le solide obtenu est dissout dans 11 d'un mélange acétone/DMSO (90 : 10 en volume) et cristallisé au frigo (1°C/4 jours). On récupère 17g d'un solide microcristallin, dont l'analyse RMN (DMSO-d6) est présentée à la figure 6.
Ce solide apparaît être constitué à 95% de calixarènes géants.
La mesure de masse molaire par GPC est présentée à la figure 7 : M=6000g/mol, nombre de motifs phénoliques (n)=28.
Mesure du rayon hydrodynamique des calixarènes formés par DDL : D (diamètre) = 4,5nm.

**Exemple B : $Ba(OH)_2$ (0,4 équivalent par rapport au phénol).**

A) Dans un erlenmeyer de 250ml, on prépare une solution de 60g de $Ba(OH)_2.8H_2O$ (0,19 mol ; 0,4 équivalent) dans 135 ml de formaldéhyde aqueux à 30% sous forte agitation magnétique.
On obtient une suspension blanche, dénommée par la suite « solution A ».
B) Un ballon tricol de 2 litres équipé d'un agitateur mécanique, d'un collecteur de Dean-Stark et d'un réfrigérant ainsi que d'un chauffage par bain d'huile, est chargé avec 104g de 4-(benzyloxy)phénol (0,519 mol). On y ajoute la solution A. On commence alors à chauffer la suspension résultante (140°C affichés), sous protection d'argon.

A 50°C mesurés, on ajoute ensuite 40ml d'éthanol, tout en poursuivant le chauffage. La suspension blanche

se transforme alors instantanément en une solution limpide jaune vif. Cette solution est maintenue à reflux pendant 1h.

L'un des rodages du ballon est alors équipé d'un col de cygne relié à un bulleur, et la solution limpide jaune vif est alors soumise à un fort balayage d'argon (20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout d'1h de balayage d'argon.

On ajoute ensuite à ce solide 300ml de xylène, et la suspension résultante est portée à reflux sous agitation pendant 8h.

Après retour à température ambiante, la suspension est filtrée, puis le précipité est neutralisé par mise en suspension dans un mélange de 500 ml de methanol/50 ml d'HCl 37%, sous forte agitation.

Après filtration, on récupère un solide brun, qui est dissout dans 1l d'un mélange acétone/DMSO (90 : 10 en volume). Cette solution est immédiatement filtrée pour isoler le *p*-(benzyloxy)calix[8]arène.

Après stockage au réfrigérateur (1°C) pendant 4 jours, on récupère 63g d'un précipité microcristallin, dont l'analyse RMN (DMSO-d6) est présentée à la figure 8.

Le solide ainsi obtenu apparaît être constitué à 90% de calixarènes géants.

La mesure de masse molaire par GPC est présentée à la figure 9 : masse molaire au pic (gaussienne centrée)=10400, soit n=50.

Mesure du rayon hydrodynamique des calixarènes formés par DDL : D (diamètre) = 7nm.

**Exemple C : KOH (1 équivalent par rapport au phénol)**

Un ballon tricol de 2 litres équipé d'un agitateur mécanique, d'un collecteur de Dean-Stark et d'un réfrigérant ainsi que d'un chauffage par bain d'huile, est chargé avec 104g de 4-(benzyloxy)phénol (0,519 mol) et 135 ml de formaldéhyde en solution aqueuse à 30%.

La suspension résultante est chauffée à 140°C affichés sous protection d'argon.

A 90°C, on injecte rapidement une solution de 28 g de KOH (0,519 mol ; 1 équivalent) dans 20ml d'eau, tout en poursuivant le chauffage. La suspension blanche se transforme en une solution limpide jaune vif. Cette solution est maintenue à reflux pendant 1h.

L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout de 1h.

L'analyse RMN (DMSO-d6) de ce solide ainsi obtenu est présentée à la figure 10a.

Ce solide apparaît majoritairement être constitué du dimère, accompagné d'une plus faible proportion d'oligomères linéaires.

On ajoute ensuite à ce solide 400ml de xylène, et la suspension résultante est portée à reflux sous agitation pendant 3h.

Après neutralisation par un mélange de 500ml de THF/50 ml d'HCl 37% sous forte agitation, une filtration de la suspension obtenue permet de récupérer 15g de *p*-(benzyloxy)calix[8]arène.

Le filtrat est évaporé à sec. Le solide obtenu est dissout dans 1l d'un mélange acétone/DMSO (90 : 10 en volume) et cristallisé au frigo (1°C/4 jours). On récupère un solide microcristallin.

Mesure du rayon hydrodynamique des calixarènes formés par DDL : D (diamètre) = 6 nm, pour un nombre de motifs phénoliques estimé de 50 (gaussienne centrée en GPC).

L'analyse RMN de ce solide est présentée en figure 10b.

**Exemple C-2 : KOH (1 équivalent par rapport au phénol), obtention de calix[80]arènes**

Un ballon tricol de deux litres équipé d'un agitateur mécanique et d'un collecteur d'eau de type « Dean-Stark » est chargé d'une suspension de 104 g de 4-(benzyloxy)phénol (0,52 mol), 28g de KOH (0,5 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.

Après retour à température ambiante, on ajoute alors 400ml de xylène. La suspension ainsi obtenue est alors portée à reflux sous agitation. Un suivi de la réaction montre alors une évolution de la composition vers la formation de calixarènes géants.

Au bout de 4h de reflux, on ajoute 50ml d'acide chlorhydrique à 37%, ainsi qu'un litre de méthanol, sous forte agitation mécanique. La suspension résultante est filtrée. Après séchage, le solide blanc ainsi obtenu est dissout dans un mélange de 100ml de DMSO et 1000ml d'acétone, puis filtré. Le solide blanc ainsi obtenu est séché à l'air, puis mis

en suspension dans 300ml de THF. Le filtrat récupéré après filtration est évaporé à sec, puis lavé avec 50ml d'acétone. Après élimination du surnageant, on obtient 2g de calixarènes géants (taille estimée en GPC (gaussienne centrée) : 80 motifs phénoliques).

L'analyse RMN 1H de ce produit est présentée sur la figure 10c.

**Exemple D : LiOH (0,4 équivalent par rapport au phénol)**

Un ballon tricol de deux litres équipé d'un agitateur mécanique et d'un collecteur d'eau de type « Dean-Stark » est chargé d'une suspension de 100 g de 4-(benzyloxy)phénol (0,5 mol), 8,4g de $LiOH.H_2O$ (0,2 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.

Une analyse RMN 1H de ce solide montre qu'il est constitué presque exclusivement du dimère phénolique (figure 27).

Après retour à température ambiante, on ajoute alors 400ml de xylène. La suspension ainsi obtenue est alors portée à reflux sous forte agitation. Un suivi de la réaction montre une évolution de la composition vers la formation de calixarènes géants.

Au bout de 2h de reflux, on ajoute 50ml d'acide chlorhydrique à 37%, ainsi qu'un litre de méthanol, sous forte agitation mécanique. La suspension résultante est filtrée, et le précipité ainsi obtenu est séché à l'air. Une analyse RMN montre que le milieu réactionnel contient 40% de calixarènes géants.

Une analyse par spectrométrie de masse « MALDI » confirme la présence de ces calixarènes (figure 28).

**Exemple E : CsOH (0,4 équivalent par rapport au phénol)**

Un ballon tricol de deux litres équipé d'un agitateur mécanique et d'un collecteur d'eau de type « Dean-Stark » est chargé d'une suspension de 104 g de 4-(benzyloxy)phénol (0,52 mol), 36 ml d'une solution de CsOH à 50% (0,208 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.

Après retour à température ambiante, on ajoute alors 200ml de xylène. La suspension ainsi obtenue est alors portée à reflux sous agitation. Un suivi de la réaction montre alors une évolution de la composition vers la formation de calixarènes géants.

Au bout de 2h de reflux, on ajoute 50ml d'acide chlorhydrique à 37%, ainsi que 500ml de tétrahydrofurane, sous forte agitation mécanique. La suspension résultante est évaporée à sec. Le solide résultant est lavé avec 2L de méthanol, puis filtré. Après séchage, le solide blanc ainsi obtenu est recristallisé dans un mélange de 100ml de DMSO et 1000ml d'acétone.

Après filtration, le filtrat est abandonné au réfrigérateur (1°C) pendant 3 jours.

Après filtration du solide ayant spontanément précipité, on obtient 30g de calixarènes géants (taille estimée en GPC : 20 motifs phénoliques). Rendement : 28%.

Une analyse RMN de ce produit est présentée en figure 29a.

Une analyse 13C de ce même produit est présentée en figure 29b.

**e) par réaction du benzyloxyphénol avec une base et du formaldéhyde dans l'eau et élimination de l'eau puis traitement thermique du précurseur solide sous forme de résine cassante non isolé dans le xylène ou toluène au reflux sans agitation.**

**Exemple A : RbOH (0,4 équivalent par rapport au phénol).**

Un ballon tricol de 2 litres équipé d'un agitateur mécanique, d'un collecteur de Dean-Stark et d'un réfrigérant ainsi que d'un chauffage par bain d'huile, est chargé avec 104g de 4-(benzyloxy)phénol (0,519 mol) et 135 ml de formaldéhyde en solution aqueuse à 30%.

La suspension résultante est chauffée à 140°C affichés sous protection d'argon.

A 90°C, on injecte rapidement 25,2ml d'une solution de RbOH à 50% en poids dans l'eau (0,4 équivalent). La suspension blanche se transforme en une solution limpide jaune vif. Cette solution est maintenue à reflux pendant 1h.

L'un des rodages du ballon est alors équipé d'un col de cygne relié à un bulleur, et la solution est alors soumise

à un fort balayage d'argon (20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout d'1h.

On ajoute ensuite à ce solide 170ml de xylène, et la suspension résultante est portée au reflux SANS agitation pendant 2h.

Le milieu réactionnel est ensuite neutralisé par ajout d'un mélange de 500ml de méthanol et de 70ml d'HCl à 37% dans l'eau sous forte agitation.

Après filtration, le solide et séchage sous vide, le solide est dissout dans un mélange de 2l acétone/DMSO (90 : 10 en volume).

Une filtration de la suspension obtenue permet de récupérer 10g de *p*-(benzyloxy)calix[8]arène.

Le filtrat est ensuite placé au réfrigérateur (1°C/4 jours).

On récupère 25g d'un solide microcristallin, dont l'analyse RMN est présentée à la figure 11.

Ce solide apparaît être constitué à 90% de calixarènes géants.

La mesure de masse molaire par GPC de ce solide est présentée à la figure 12 : M=6000g/mol, nombre de motifs phénoliques (n)=29 (gaussienne centrée).

Mesure du rayon hydrodynamique des calixarènes formés par DDL : D (diamètre) = 4,6nm.

**Exemple B : KOH (0,4 équivalent par rapport au phénol)**

Un ballon tricol de 2 litres équipé d'un agitateur mécanique, d'un collecteur de Dean-Stark et d'un réfrigérant ainsi que d'un chauffage par bain d'huile, est chargé avec 104g de 4-(benzyloxy)phénol (0,519 mol) et 135 ml de formaldéhyde en solution aqueuse à 30%.

La suspension résultante est chauffée à 140°C affichés sous protection d'argon.

A 90°C, on injecte rapidement 12g de KOH dans 10ml d'eau (0,4 équivalent). La suspension blanche se transforme en une solution limpide jaune vif. Cette solution est maintenue à reflux pendant 1h.

L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout d'1h.

On ajoute ensuite à ce solide 170ml de xylène, et la suspension résultante est portée au reflux SANS agitation pendant 2h.

Le milieu réactionnel est ensuite neutralisé par ajout d'un mélange de 500ml de méthanol et de 70ml d'HCl à 37% dans l'eau sous forte agitation.

Après filtration et séchage sous vide, le solide est dissout dans un mélange de 2l acétone/DMSO (90 : 10 en volume).

Une filtration de la suspension obtenue permet de récupérer 10g de *p*-(benzyloxy)calix[8]arène. Le filtrat est ensuite placé au réfrigérateur (1°C/4 jours).

On récupère 35g d'un solide microcristallin, dont l'analyse RMN (DMSO-d6) montre qu'il apparaît être constitué à 90% de *p*-(benzyloxy)calixarènes géants.

Une recristallisation de ce solide dans le dichlorobenzène permet de récupérer 10g d'un solide brun de pureté améliorée, dont l'analyse RMN (DMSO-d6) est présentée à la figure 13.

Ce solide apparaît être constitué de calixarènes géants à plus de 95%.

Une mesure de masse molaire par GPC est présentée à la figure 14 : M=12700g/mol, nombre de motifs phénoliques (n)=60 (gaussienne centrée).

Mesure du rayon hydrodynamique des calixarènes formés par DDL: D (diamètre) = 8nm.

**Exemple C : KOH (0,13 équivalent par rapport au phénol)**

Un ballon tricol de 2 litres équipé d'un agitateur mécanique, d'un collecteur de Dean-Stark et d'un réfrigérant ainsi que d'un chauffage par bain d'huile, est chargé avec 104g de 4-(benzyloxy)phénol (0,519 mol) et 135 ml de formaldéhyde en solution aqueuse à 30%.

La suspension résultante est chauffée à 140°C affichés sous protection d'argon.

A 90°C, on injecte rapidement une solution de 3,7g de KOH (0,066 mol ; 0,13 équivalent) dans 5ml d'eau, tout en poursuivant le chauffage. La suspension blanche se transforme en une solution limpide jaune vif. Cette solution est maintenue à reflux pendant 1h.

L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 40min de balayage, la solution initialement limpide et fluide devient de plus en plus visqueuse,

pour finalement prendre en masse au bout de 3h.

On ajoute ensuite à ce solide 200ml de xylène, et la suspension résultante est portée au reflux sans agitation pendant 2h.

Après neutralisation par un mélange de 500ml de THF/50 ml d'HCl 37% sous forte agitation.

Une filtration de la suspension obtenue permet de récupérer 15g de *p*-(benzyloxy)calix[8]arène.

Le filtrat est évaporé à sec. Le solide obtenu est dissout dans 1l d'un mélange acétone/DMSO (90 : 10 en volume) et cristallisé au frigo (1°C/4 jours). On récupère 50g d'un solide microcristallin, constitué à 90% de calixarènes géants.

DDL : diamètre moyen mesuré = 4,7 nm, ce qui correspond à un nombre de motifs phénoliques estimé de 35.

**f) par réaction du benzyloxyphénol avec une base et du formaldéhyde dans l'eau et conservation de l'eau puis traitement thermique du précurseur solide sous forme de résine cassante non isolé dans l'octane au reflux.**

Un ballon tricol de 250 ml équipé d'un agitateur mécanique est chargé avec 22,6g (double espace) (0,113 mol) de 4-(benzyloxy)phénol, 30ml de formaldéhyde en solution aqueuse à 37% et 2,53g (0,045 mol, 0,4 équivalent par rapport au phénol) de KOH en pastilles.

La solution limpide jaune vif ainsi obtenue est mise au reflux sous agitation mécanique et sous argon. Au bout d'environ 1h, on observe la formation d'un précipité au sein de la phase aqueuse.

On ajoute alors 40ml de d'octane, et la solution est portée au reflux. Au bout de 1h, le milieu réactionnel prend en masse. Le chauffage et l'agitation sont arrêtés.

Le solide ainsi obtenu est lavé avec un mélange de 200ml de méthanol et de 10ml d'HCl à 37%, sous forte agitation.

Après filtration, on récupère quantitativement un solide blanc, dont l'analyse RMN montre qu'il est constitué à 60% de calixarènes géants.

**g) par réaction du benzyloxyphénol avec une base et du formaldéhyde dans l'eau et conservation de l'eau puis traitement thermique du précurseur solide sous forme de résine cassante non isolé dans le xylène ou toluène au reflux.**

Un ballon tricol de 250 ml équipé d'un agitateur mécanique est chargé avec 22,6g (0,113 mol) de 4-(benzyloxy)phénol, 30ml de formaldéhyde en solution aqueuse à 37% et 2,53g (0,045 mol, 0,4 équivalent par rapport au phénol) de KOH en pastilles.

La solution limpide jaune vif ainsi obtenue est mise au reflux sous agitation mécanique et sous argon. Au bout d'environ 1h, on observe la formation d'un précipité au sein de la phase aqueuse.

On ajoute alors 40ml de xylène, et la solution est portée au reflux. Au bout de 2h, le milieu réactionnel prend en masse. Le chauffage et l'agitation sont arrêtés.

Le solide ainsi obtenu est lavé avec un mélange de 200ml de méthanol et de 10ml d'HCl à 37%, sous forte agitation.

Après filtration, on récupère quantitativement un solide blanc, dont l'analyse RMN est présentée à la figure 15.

Ce solide apparaît être constitué à 80% de calixarènes géants.

Mesure de masse molaire par GPC : M=4600g/mol, nombre de motifs phénoliques (n)=22.

Mesure du rayon hydrodynamique des calixarènes formés par DDL : D (diamètre) = 3,2nm.

**h) par traitement thermique du dimère du benzyloxyphénol, isolé, dans l'octane au reflux.**

Un ballon tricol de deux litres équipé d'un agitateur mécanique et d'un collecteur d'eau de type « Dean-Stark » est chargé d'une suspension de 100 g de 4-(benzyloxy)phénol (0,5 mol), 8,4g de $LiOH.H_2O$ (0,2 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.

Une analyse RMN 1H de ce solide montre qu'il est constitué presque exclusivement du dimère phénolique (figure 27).

Le milieu réactionnel est alors neutralisé avec un mélange HCl/méthanol, sous forte agitation.

Après filtration et lavage à l'eau, on récupère quantitativement un solide blanc.

La totalité du solide ainsi récupéré est placée dans un ballon bicol d'lL. Une solution de KOH (11g) dans 150ml de méthanol est ajoutée sous agitation mécanique, sous argon. Au bout de 8h, le méthanol est évaporé sous pompe à palette. 400ml d'octane sont alors ajoutés au solide jaune ainsi obtenu. Après fixation d'un collecteur d'eau de type

« Dean-Stark » sur l'un des rodages, le milieu est porté à reflux pendant 8h. Un suivi RMN de la réaction montre l'évolution de la composition du milieu réactionnel vers la formation de calixarènes géants. Proportion finale : 60%.

**i) par traitement thermique du dimère du benzyloxyphénol, isolé, dans le xylène ou toluène au reflux.**

Un ballon tricol de deux litres équipé d'un agitateur mécanique et d'un collecteur d'eau de type « Dean-Stark » est chargé d'une suspension de 100 g de 4-(benzyloxy)phénol (0,5 mol), 8,4g de $LiOH.H_2O$ (0,2 mol) et 100ml d'une solution de formaldehyde à 37% (1,34 mol). Après reflux pendant 20 minutes, on obtient une solution limpide jaune vif.
Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.
Une analyse RMN 1H de ce solide montre qu'il est constitué presque exclusivement du dimère phénolique (figure 27).
Le milieu réactionnel est alors neutralisé avec un mélange HCl/méthanol, sous forte agitation.
Après filtration et lavage à l'eau, on récupère quantitativement un solide blanc.
La totalité du solide ainsi récupéré est placée dans un ballon bicol d'1L. Une solution de KOH (11g) dans 150ml de méthanol est ajoutée sous agitation mécanique, sous argon. Au bout de 8h, le méthanol est évaporé sous pompe à palette. 400ml de xylène sont alors ajoutés au solide jaune ainsi obtenu. Après fixation d'un collecteur d'eau de type « Dean-Stark » sur l'un des rodages, le milieu est porté à reflux pendant 4h. Un suivi RMN de la réaction montre l'évolution de la composition du milieu réactionnel vers la formation de calixarènes géants. Proportion finale : 40%.

**j) par réaction du benzyloxyphénol avec une base et du formaldéhyde dans l'eau, et un solvant organique (sans élimination d'eau)**

Un ballon tricol de deux litres équipé d'un agitateur mécanique et d'un collecteur d'eau de type « Dean-Stark » est chargé d'une suspension de 100 g de 4-(benzyloxy)phénol (0,5 mol), 13g de KOH (0,214 mol), 100ml d'une solution de formaldehyde à 37% (1,34 mol) et 800ml de xylène. L'émulsion jaune ainsi obtenue est portée au reflux pendant 5h.
Une analyse RMN montre que le milieu réactionnel contient 60% de calixarènes géants.

**Exemple 2 : Préparation de *p*-(octyloxy)calixarènes :**

**a) par réaction de octyloxyphénol avec une base et du formaldéhyde dans l'eau et élimination de l'eau. Précurseur solide sous forme de résine cassante isolé.**

Dans un ballon bicol de 250ml, on prépare une suspension de 25 g de 4-(octyloxy)phénol (0,113 mol), 30ml de formaldéhyde à 37% et 2,60g de KOH (0,046mol, 0,41 équivalent) sous protection d'argon. Au bout d' 1h de reflux, on obtient une solution limpide jaune vif.
L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.
Au bout d'environ 40min de balayage, la solution initialement limpide et fluide devient de plus en plus visqueuse, pour finalement prendre en masse au bout de deux heures.
On obtient quantitativement un solide jaune vif.
L'analyse RMN ($CDCl_3$/DMSO) montre que ce produit brut apparaît être constitué à 85% de calixarènes géants.
Le solide jaune dur et cassant précédemment obtenu est dispersé sous forte agitation dans une solution de 10ml d'HCl à 37% dans 200ml de méthanol.
Après filtration, on récupère quantitativement un solide blanc, dont l'analyse RMN ($CDCl_3$/DMSO) est présentée à la figure 16.
Ce solide apparaît être constitué à 95 % de calixarènes géants.
Mesure de masse molaire par Gel Permeation Chromatography (GPC) :
M=7700g/mol, nombre de motifs phénoliques (n)=35 ; masse molaire moyenne correspondant à 57 unités phénoliques.
Mesure du rayon hydrodynamique des calixarènes formés par DDL : D (diamètre) = 4,2nm.

**b) par réaction de l'octyloxyphénol avec une base et du formaldéhyde dans l'eau et élimination de l'eau puis traitement thermique du précurseur solide sous forme de résine cassante non isolé dans l'octane au reflux.**

Dans un ballon bicol de 250ml, on prépare une suspension de 25 g de 4-(octyloxy)phénol (0,113 mol), 30ml de formaldéhyde à 37% et 2,60g de KOH (0,046mol, 0,41 équivalent) sous protection d'argon. Au bout d' 1h de reflux, on obtient une solution limpide jaune vif.

L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout de deux heures.

On obtient quantitativement un solide jaune vif.

Ce solide est ensuite additionné de 100ml d'octane, et porté à reflux pendant 8h. Après neutralisation par un mélange HCl/méthanol, on obtient quantitativement une solide jaune pâle.

Une analyse GPC montre une augmentation de la taille des macrocycles obtenus par rapport au cas précédent (40 unités phénoliques, gaussienne centrée).

**c) par réaction de l'octyloxyphénol avec une base et du formaldéhyde dans l'eau et élimination de l'eau puis traitement thermique du précurseur solide sous forme de résine cassante non isolé dans le xylène ou toluène au reflux.**

Dans un ballon bicol de 250ml, on prépare une suspension de 25 g de 4-(octyloxy)phénol (0,113 mol), 30ml de formaldéhyde à 37% et 2,60g de KOH (0,046mol, 0,41 équivalent) sous protection d'argon. Au bout d' 1h de reflux, on obtient une solution limpide jaune vif.

L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout de deux heures.

On obtient quantitativement un solide jaune vif.

Ce solide est ensuite additionné de 100ml de xylène, et porté à reflux pendant 4h. Une analyse RMN montre une diminution de la proportion de calixarènes géants (60%). Une étude GPC montre que la taille des macrocycles obtenus est de 40 unités phénoliques (gaussienne centrée).

**d) par réaction de l'octyloxyphénol avec une base et du formaldéhyde dans l'eau et conservation de l'eau puis traitement thermique du précurseur solide sous forme de résine cassante non isolée dans l'octane au reflux.**

Dans un ballon bicol de 250ml, on prépare une suspension de 25 g de 4-(octyloxy)phénol (0,113 mol), 30ml de formaldéhyde à 37% et 2,60g de KOH (0,046mol, 0,41 équivalent) sous protection d'argon. Au bout d'1h de reflux, on obtient une solution limpide jaune vif. Au bout d' 1h de reflux supplémentaire, un précipité brun clair apparait dans le milieu réactionnel. Après ajout de 50ml d'octane, la suspension fluide et homogène ainsi obtenue est mise au reflux pendant 2h, durée au bout de laquelle un volumineux précipité apparaît.

Après neutralisation par une solution de HCl dans le méthanol et filtration, on obtient un solide jaune pâle.

Une analyse RMN montre une diminution de la proportion de calixarènes géants (60%). Une étude GPC montre que la taille des macrocycles obtenus est de 40 unités phénoliques (gaussienne centrée).

**e) par réaction de l'octyloxyphénol avec une base et du formaldéhyde dans l'eau et conservation de l'eau puis traitement thermique du précurseur solide sous forme de résine cassante non isolée dans le xylène ou toluène au reflux.**

Dans un ballon bicol de 250ml, on prépare une suspension de 25 g de 4-(octyloxy)phénol (0,113 mol), 30ml de formaldéhyde à 37% et 2,60g de KOH (0,046mol, 0,41 équivalent) sous protection d'argon. Au bout d'1h de reflux, on obtient une solution limpide jaune vif. Au bout d'1h de reflux supplémentaire, un précipité brun clair apparait dans le milieu réactionnel. Après ajout de 50ml de xylène, la suspension fluide et homogène ainsi obtenue est mise au reflux pendant 2h, durée au bout de laquelle un volumineux précipité apparaît.

Une analyse RMN montre une diminution de la proportion de calixarènes géants (50%). Une étude GPC montre que la taille des macrocycles obtenus est de 45 unités phénoliques (gaussienne centrée).

**f) par traitement thermique du dimère de l'octyloxyphénol, isolé, dans l'octane au reflux.**

Dans un ballon bicol de 250ml, on prépare une suspension de 25 g de 4-(octyloxy)phénol (0,113 mol), 30ml de formaldéhyde à 37% et 1,93g de LiOH.H2O (0,046mol, 0,41 équivalent) sous protection d'argon. Au bout d' 1h de reflux, on obtient une solution limpide jaune vif.

L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 20min de balayage, la solution initialement limpide et fluide devient de plus en plus visqueuse,

pour finalement prendre en masse au bout d'une heure.

On obtient quantitativement un solide jaune vif, essentiellement constitué du dimère de l'octyloxyphénol.

Ce solide est neutralisé sous forte agitation par HCl dans le méthanol, puis filtré.

Sous argon, on ajoute alors 2,60g de KOH (0,046mol, 0,41 équivalent) en solution dans 50ml de méthanol, sous agitation mécanique. Au bout de 4 heures, le méthanol est évaporé à la pompe à palettes.

On ajoute alors 100ml d'octane, et le milieu réactionnel est remis au reflux 4h.

Une analyse RMN du solide jaune obtenu montre qu'il est constitué d'une proportion de 80% de calixarènes géants. Une étude GPC montre que la taille des macrocycles obtenus est de 35 unités phénoliques (gaussienne centrée).

**g) par traitement thermique du dimère de l'octyloxyphénol, isolé, dans le xylène ou toluène au reflux.**

Dans un ballon bicol de 250ml, on prépare une suspension de 25 g de 4-(octyloxy)phénol (0,113 mol), 30ml de formaldéhyde à 37% et 1,93g de LiOH.H2O (0,046mol, 0,41 équivalent) sous protection d'argon. Au bout d' 1h de reflux, on obtient une solution limpide jaune vif.

L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 20min de balayage, la solution initialement limpide et fluide devient de plus en plus visqueuse, pour finalement prendre en masse au bout d'une heure.

On obtient quantitativement un solide jaune vif, essentiellement constitué du dimère de l'octyloxyphénol.

Ce solide est neutralisé sous forte agitation par HCl dans le méthanol, puis filtré.

Sous argon, on ajoute alors 2,60g de KOH (0,046mol, 0,41 équivalent) en solution dans 50ml de méthanol, sous agitation mécanique. Au bout de 4 heures, le méthanol est évaporé à la pompe à palettes.

On ajoute alors 100ml de xylène, et le milieu réactionnel est remis au reflux 4h.

Une analyse RMN du solide jaune obtenu montre qu'il est constitué d'une proportion de 80% de calixarènes géants. Une étude GPC montre que la taille des macrocycles obtenus est de 33 unités phénoliques (gaussienne centrée).

**h) par réaction de l'octyloxyphénol avec une base et du formaldéhyde dans l'eau et un solvant organique (sans élimination d'eau)**

Dans un ballon bicol de 250ml, on prépare une suspension de 25 g de 4-(octyloxy)phénol (0,113 mol), 30ml de formaldéhyde à 37% et 2,60g de KOH (0,046mol, 0,41 équivalent) et 200ml de xylène sous protection d'argon.

L'émulsion jaune ainsi obtenue est portée au reflux pendant 5h.

Une analyse RMN montre que le milieu réactionnel contient 80% de calixarènes géants.

**Exemple 2.1 : Préparation de *p*-(méthoxy)calixarènes :**

**a) par réaction du 4-(méthoxy)phénol avec une base et du formaldéhyde dans l'eau et élimination de l'eau puis. Précurseur solide sous forme de résine cassante isolé.**

Un ballon tricol de deux litres équipé d'un agitateur mécanique et d'un collecteur d'eau de type « Dean-Stark » est chargé d'une suspension de 65g de 4-(méthoxy)phénol (0,523 mol), 12g de KOH (0,214 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune. Une analyse RMN 1H de ce solide montre qu'il est constitué à 40% de calixarènes géants.

**b) par réaction du 4-(méthoxy)phénol avec une base et du formaldéhyde dans l'eau et élimination de l'eau puis traitement thermique du précurseur solide sous forme de résine cassante non isolé dans l'octane au reflux.**

Un ballon tricol de deux litres équipé d'un agitateur mécanique et d'un collecteur d'eau de type « Dean-Stark » est chargé d'une suspension de 65g de 4-(méthoxy)phénol (0,523 mol), 12g de KOH (0,214 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.

Après retour à température ambiante, on ajoute alors 400ml d'octane. Le milieu réactionnel hétérogène ainsi obtenu est alors porté à reflux sans agitation. Un suivi de la réaction montre alors une évolution de la composition vers

la formation de calixarènes géants.

Au bout de 6h de reflux, on ajoute 50ml d'acide chlorhydrique à 37%, ainsi qu'un litre de méthanol, sous forte agitation mécanique. La suspension résultante est filtrée, et le précipité ainsi obtenu est séché à l'air. On récupère 60g d'un solide poudreux. Rendement : 92%. Une analyse GPC montre que ce solide est constitué de calixarènes géants (taille estimée en GPC : 50 motifs phénoliques).

**c) par réaction du 4-(méthoxy)phénol avec une base et du formaldéhyde dans l'eau et élimination de l'eau puis traitement thermique du précurseur solide sous forme de résine cassante non isolé dans le xylène ou toluène au reflux.**

Un ballon tricol de deux litres équipé d'un agitateur mécanique et d'un collecteur d'eau de type « Dean-Stark » est chargé d'une suspension de 65g de 4-(méthoxy)phénol (0,523 mol), 12g de KOH (0,214 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.

Après retour à température ambiante, on ajoute alors 400ml de xylène. La suspension ainsi obtenu est alors porté à reflux sans agitation pendant 4h. Le milieu réactionnel est neutralisé par 50ml d'acide chlorhydrique à 37% en solution dans IL de méthanol, sous forte agitation mécanique. La suspension résultante est filtrée, et le précipité ainsi obtenu est séché à l'air. On récupère 60g d'un solide brun. Rendement : 92%. Une analyse par RMN 1H de ce solide montre qu'il est constitué à 60% de calixarènes géants.

**d) par réaction du 4-(méthoxy)phénol avec une base et du formaldéhyde dans l'eau et conservation de l'eau puis traitement thermique du précurseur solide sous forme de résine cassante non isolé dans l'octane au reflux.**

Un ballon tricol de deux litres équipé d'un agitateur mécanique et d'un collecteur d'eau de type « Dean-Stark » est chargé d'une suspension de 65g de 4-(méthoxy)phénol (0,523 mol), 12g de KOH (0,214 mol) et 100ml d'une solution de formaldehyde à 37% (1,34 mol). Après reflux pendant 20 minutes, on obtient une solution limpide jaune vif. Le reflux est poursuivi pendant 2h, durée au bout de laquelle un précipité jaune apparaît. On ajoute alors 400ml d'octane, et le reflux est poursuivi sous agitation pendant 1h, durée au bout de laquelle le milieu réactionnel prends en masse. Le milieu réactionnel est neutralisé par 50ml d'acide chlorhydrique à 37% en solution dans IL de méthanol, sous forte agitation mécanique. La suspension résultante est filtrée, et le précipité ainsi obtenu est séché à l'air. On récupère 60g d'un solide brun. Rendement : 92%. Une analyse par RMN 1H de ce solide montre qu'il est constitué à 80% de calixarènes géants.

**e) par réaction du 4-(méthoxy)phénol avec une base et du formaldéhyde dans l'eau et conservation de l'eau puis traitement thermique du précurseur solide sous forme de résine cassante non isolé dans le xylène ou toluène au reflux.**

Un ballon tricol de deux litres équipé d'un agitateur mécanique et d'un collecteur d'eau de type « Dean-Stark » est chargé d'une suspension de 65g de 4-(méthoxy)phénol (0,523 mol), 12g de KOH (0,214 mol) et 100ml d'une solution de formaldehyde à 37% (1,34 mol). Après reflux pendant 20 minutes, on obtient une solution limpide jaune vif. Le reflux est poursuivi pendant 2h, durée au bout de laquelle un précipité jaune apparaît. On ajoute alors 400ml de xylène, et le reflux est poursuivi sous agitation pendant 1h, durée au bout de laquelle le milieu réactionnel prends en masse. Le milieu réactionnel est neutralisé par 50ml d'acide chlorhydrique à 37% en solution dans IL de méthanol, sous forte agitation mécanique. La suspension résultante est filtrée, et le précipité ainsi obtenu est séché à l'air. On récupère 60g d'un solide brun. Rendement : 92%. Une analyse par RMN 1H de ce solide montre qu'il est constitué à 80% de calixarènes géants.

**f) par traitement thermique du dimère du 4-(méthoxy)phénol, isolé, dans l'octane au reflux.**

Un ballon tricol de deux litres équipé d'un agitateur mécanique et d'un collecteur d'eau de type « Dean-Stark » est chargé d'une suspension de 65g de 4-(méthoxy)phénol (0,523 mol), 10,08g de $LiOH.H_2O$ (0,214 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.

Une analyse RMN 1H de ce solide montre qu'il est constitué presque exclusivement du dimère phénolique.

Le milieu réactionnel est alors neutralisé avec un mélange HCl/méthanol, sous forte agitation.

Après filtration et lavage à l'eau, on récupère quantitativement un solide blanc.

La totalité du solide ainsi récupéré est placée dans un ballon bicol d'IL. Une solution de KOH (13,2g) dans 150ml de méthanol est ajoutée sous agitation mécanique, sous argon. Au bout de 8h, le méthanol est évaporé sous pompe à palette. 400ml d'octane sont alors ajoutés au solide jaune ainsi obtenu. Après fixation d'un collecteur d'eau de type « Dean-Stark » sur l'un des rodages, le milieu est porté à reflux pendant 4h. Un suivi RMN de la réaction montre l'évolution de la composition du milieu réactionnel vers la formation de calixarènes géants. Proportion finale : 80%.

**g) par traitement thermique du dimère du 4-(méthoxy)phénol, isolé, dans le xylène ou toluène au reflux.**

Un ballon tricol de deux litres équipé d'un agitateur mécanique et d'un collecteur d'eau de type « Dean-Stark » est chargé d'une suspension de 65g de 4-(méthoxy)phénol (0,523 mol), 10,08g de $LiOH.H_2O$ (0,214 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.

Une analyse RMN 1H de ce solide montre qu'il est constitué presque exclusivement du dimère phénolique.

Le milieu réactionnel est alors neutralisé avec un mélange HCl/méthanol, sous forte agitation.

Après filtration et lavage à l'eau, on récupère quantitativement un solide blanc.

La totalité du solide ainsi récupéré est placée dans un ballon bicol d'IL. Une solution de KOH (13,2g) dans 150ml de méthanol est ajoutée sous agitation mécanique, sous argon. Au bout de 8h, le méthanol est évaporé sous pompe à palette. 400ml de xylène sont alors ajoutés au solide jaune ainsi obtenu. Après fixation d'un collecteur d'eau de type « Dean-Stark » sur l'un des rodages, le milieu est porté à reflux pendant 4h. Un suivi RMN de la réaction montre l'évolution de la composition du milieu réactionnel vers la formation de calixarènes géants. Proportion finale : 50%.

**h) Par réaction du 4-(méthoxy)phénol avec une base et du formaldéhyde dans l'eau et un solvant organique (sans élimination d'eau).**

Un ballon tricol de deux litres équipé d'un agitateur mécanique et d'un collecteur d'eau de type « Dean-Stark » est chargé d'une suspension de 65g de 4-(méthoxy)phénol (0,523 mol), 12g de KOH (0,214 mol), 100ml d'une solution de formaldehyde à 37% (1,34 mol) et 800ml de xylène. L'émulsion jaune ainsi obtenue est portée au reflux pendant 5h.

Une analyse RMN montre que le milieu réactionnel contient 70% de calixarènes géants.

**Exemple 2.2 : Préparation de *p*-(méthyl)calixarènes :**

**a) par réaction du 4-(méthyl)phénol avec une base et du formaldéhyde dans l'eau et élimination. Précurseur solide sous forme de résine cassante isolé.**

Un ballon tricol de deux litres équipé d'un agitateur mécanique et d'un collecteur d'eau de type « Dean-Stark » est chargé d'une solution de 55g de *p*-crésol (0,5 mol), 12g de KOH (0,214 mol) et 100ml d'une solution de formaldehyde à 37% (1,34 mol). Après reflux pendant 20 minutes, on obtient une solution limpide jaune pâle.

Tout en maintenant le reflux, on établit alors un fort courant d'argon l'intérieur du ballon, dans le but d'éliminer l'eau. (double espace) Le milieu réactionnel initialement très fluide devient de plus en plus visqueux, jusqu'à se solidifier complètement en un solide cassant de couleur jaune pâle.

Une analyse RMN proton montre que ce solide est constitué à 30% de calixarènes géants

**b) par réaction du 4-(méthyl)phénol avec une base et du formaldéhyde dans l'eau et élimination de l'eau puis traitement thermique du précurseur solide sous forme de résine cassante non isolé dans l'octane au reflux.**

Un ballon tricol de deux litres équipé d'un agitateur mécanique et d'un collecteur d'eau de type « Dean-Stark » est chargé d'une solution de 55g de *p*-crésol (0,5 mol), 12g de KOH (0,214 mol) et 100ml d'une solution de formaldehyde à 37% (1,34 mol). Après reflux pendant 20 minutes, on obtient une solution limpide jaune pâle.

Tout en maintenant le reflux, on établit alors un fort courant d'argon l'intérieur du ballon, dans le but d'éliminer l'eau. Le milieu réactionnel initialement très fluide devient de plus en plus visqueux, jusqu'à se solidifier complètement en un solide cassant de couleur jaune pâle.

Après retour à température ambiante, on ajoute alors 400ml d'octane. Le milieu réactionnel hétérogène ainsi obtenu est alors porté à reflux sans agitation. Un suivi de la réaction montre alors une évolution de la composition vers la formation de calixarènes géants.

Au bout de 6h de reflux, on ajoute 50ml d'acide chlorhydrique à 37%, ainsi qu'un litre de méthanol, sous forte agitation mécanique. La suspension résultante est filtrée, et le précipité ainsi obtenu est séché à l'air. On récupère 40g d'un solide poudreux. Rendement : 72%.

**c) par réaction du 4-(méthyl)phénol avec une base et du formaldéhyde dans l'eau et élimination de l'eau puis traitement thermique du précurseur solide sous forme de résine cassante non isolé dans le xylène ou toluène au reflux.**

Un ballon tricol de deux litres équipé d'un agitateur mécanique et d'un collecteur d'eau de type « Dean-Stark » est chargé d'une solution de 55g de *p*-crésol (0,51 mol), 12g de KOH (0,214 mol) et 100ml d'une solution de formaldehyde à 37% (1,34 mol). Après reflux pendant 20 minutes, on obtient une solution limpide jaune pâle.

Tout en maintenant le reflux, on établit alors un fort courant d'argon l'intérieur du ballon, dans le but d'éliminer l'eau. Le milieu réactionnel initialement très fluide devient de plus en plus visqueux, jusqu'à se solidifier complètement en un solide cassant de couleur jaune pâle.

Après retour à température ambiante, on ajoute alors 400ml de xylène. Le milieu réactionnel est alors porté à reflux sous agitation.

Au bout de 6h de reflux, on ajoute 50ml d'acide chlorhydrique à 37%, ainsi qu'un litre de méthanol, sous forte agitation mécanique. La suspension résultante est filtrée, et le précipité ainsi obtenu est séché à l'air. Une analyse du milieu réactionnel par RMN proton montre qu'il est constitué à 30% de calixarènes géants.

**d) par réaction du 4-(méthyl)phénol avec une base et du formaldéhyde dans l'eau et conservation de l'eau puis traitement thermique du précurseur solide sous forme de résine cassante non isolé dans l'octane au reflux.**

Un ballon tricol de deux litres équipé d'un agitateur mécanique et d'un collecteur d'eau de type « Dean-Stark » est chargé d'une suspension de 55g dep-crésol (0,5 mol) (0,51 mol), 12g de KOH (0,214 mol) et 100ml d'une solution de formaldehyde à 37% (1,34 mol). Après reflux pendant 20 minutes, on obtient une solution limpide jaune vif. Le reflux est poursuivi pendant 2h, durée au bout de laquelle un précipité jaune apparaît. On ajoute alors 400ml d'octane, et le reflux est poursuivi sous agitation pendant 1h, durée au bout de laquelle le milieu réactionnel prends en masse. Le milieu réactionnel est neutralisé par 50ml d'acide chlorhydrique à 37% en solution dans IL de méthanol, sous forte agitation mécanique. La suspension résultante est filtrée, le précipité est lavé à l'eau, et séché à l'air. Une analyse par RMN 1H de ce solide montre qu'il est constitué à 80% de calixarènes géants.

**e) par réaction du 4-(méthyl)phénol avec une base et du formaldéhyde dans l'eau et conservation de l'eau puis traitement thermique du précurseur solide sous forme de résine cassante non isolé dans le xylène ou toluène au reflux.**

Un ballon tricol de deux litres équipé d'un agitateur mécanique et d'un collecteur d'eau de type «Dean-Stark» est chargé d'une suspension de 55g de *p*-crésol (0,5 mol), 12g de KOH (0,214 mol) et 100ml d'une solution de formaldehyde à 37% (1,34 mol). Après reflux pendant 20 minutes, on obtient une solution limpide jaune vif. Le reflux est poursuivi pendant 2h, durée au bout de laquelle un précipité jaune apparaît. On ajoute alors 400ml de xylène, et le reflux est poursuivi sous agitation pendant 1h, durée au bout de laquelle le milieu réactionnel prends en masse. Le milieu réactionnel est neutralisé par 50ml d'acide chlorhydrique à 37% en solution dans IL de méthanol, sous forte agitation mécanique. La suspension résultante est filtrée, le précipité est lavé à l'eau, et séché à l'air. Une analyse par RMN 1H de ce solide montre qu'il est constitué à 80% de calixarènes géants.

**f) par traitement thermique du dimère du 4-(méthyl)phénol, isolé, dans l'octane au reflux.**

Un ballon tricol de deux litres équipé d'un agitateur mécanique et d'un collecteur d'eau de type « Dean-Stark » est chargé d'une suspension de 55g de 4-(métyl)phénol (0,5 mol), 10,08g de $LiOH.H_2O$ (0,214 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.

Une analyse RMN 1H de ce solide montre qu'il est constitué presque exclusivement du dimère phénolique.

Le milieu réactionnel est alors neutralisé avec un mélange HCl/méthanol, sous forte agitation. Après filtration et lavage à l'eau, on récupère quantitativement un solide blanc.

La totalité du solide ainsi récupéré est placée dans un ballon bicol d'IL. Une solution de KOH (12g) dans 150ml de méthanol est ajoutée sous agitation mécanique, sous argon. Au bout de 8h, le méthanol est évaporé sous pompe à

palette. 400ml d'octane sont alors ajoutés au solide jaune ainsi obtenu. Après fixation d'un collecteur d'eau de type « Dean-Stark » sur l'un des rodages, le milieu est porté à reflux pendant 4h. Un suivi RMN de la réaction montre l'évolution de la composition du milieu réactionnel vers la formation de calixarènes géants. Proportion finale : 50%.

**g) par traitement thermique du dimère du 4-(méthyl)phénol, isolé, dans le xylène ou toluène au reflux.**

Un ballon tricol de deux litres équipé d'un agitateur mécanique et d'un collecteur d'eau de type « Dean-Stark » est chargé d'une suspension de 55g de 4-(métyl)phénol (0,5 mol), 10,08g de LiOH.$H_2O$ (0,214 mol) et 100ml d'une solution de formaldehyde à 37% (1,34 mol). Après reflux pendant 20 minutes, on obtient une solution limpide jaune vif.
Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.
Une analyse RMN 1H de ce solide montre qu'il est constitué presque exclusivement du dimère phénolique.
Le milieu réactionnel est alors neutralisé avec un mélange HCl/méthanol, sous forte agitation.
Après filtration et lavage à l'eau, on récupère quantitativement un solide blanc.
La totalité du solide ainsi récupéré est placée dans un ballon bicol d'IL. Une solution de KOH (12g) dans 150ml de méthanol est ajoutée sous agitation mécanique, sous argon. Au bout de 8h, le méthanol est évaporé sous pompe à palette. 400ml de xylène sont alors ajoutés au solide jaune ainsi obtenu. Après fixation d'un collecteur d'eau de type « Dean-Stark » sur l'un des rodages, le milieu est porté à reflux pendant 4h. Un suivi RMN de la réaction montre l'évolution de la composition du milieu réactionnel vers la formation de calixarènes géants. Proportion finale : 50%.

**h) Par réaction du 4-(méthyl)phénol avec une base et du formaldéhyde dans l'eau et un solvant organique (sans élimination d'eau).**

Un ballon tricol de deux litres équipé d'un agitateur mécanique et d'un collecteur d'eau de type « Dean-Stark » est chargé d'une suspension de 55 g de p-crésol (0,5 mol), 12g de KOH (0,214 mol), 100ml d'une solution de formaldehyde à 37% (1,34 mol) et 800ml de xylène. L'émulsion jaune ainsi obtenue est portée au reflux pendant 5h.
Une analyse RMN montre que le milieu réactionnel contient 70% de calixarènes géants.

**Exemple 2.3 : Préparation de *p*-(éthyl)calixarènes :**

Un ballon tricol de 2 litres équipé d'un agitateur mécanique, d'un collecteur de Dean-Stark et d'un réfrigérant ainsi que d'un chauffage par bain d'huile, est chargé avec 61,04g de 4-(ethyl)phénol (0,5 mol) et 135 ml de formaldéhyde en solution aqueuse à 30%.
La suspension résultante est chauffée à 140°C affichés sous protection d'argon.
A 90°C, on injecte rapidement une solution de 12g de KOH (0,214 mol ; 0,43 équivalents) dans 10ml d'eau, tout en poursuivant le chauffage. La suspension blanche se transforme en une solution limpide jaune vif. Cette solution est maintenue à reflux pendant 1h.
L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.
Au bout d'environ 40min de balayage, la solution initialement limpide et fluide devient de plus en plus visqueuse, pour finalement prendre en masse au bout d'1h.
On ajoute ensuite à ce solide 600ml de xylène, et la suspension résultante est portée au reflux sous forte agitation mécanique, de façon à bien redisperser le précurseur solide pendant cette durée.
Après neutralisation par un mélange de 500ml de THF/50 ml d'HCl 37% sous forte agitation et filtration, on récupère un solide blanc constitué à 60% de calixarènes géants (analye RMN 1H).

**Exemple 2.4** : **Préparation de *p*-(benzyl)calixarènes :**

Un ballon tricol de 2 litres équipé d'un agitateur mécanique, d'un collecteur de Dean-Stark et d'un réfrigérant ainsi que d'un chauffage par bain d'huile, est chargé avec 92,12g de 4-(benzyl)phénol (0,5 mol) et 135 ml de formaldéhyde en solution aqueuse à 30%.
La suspension résultante est chauffée à 140°C affichés sous protection d'argon.
A 90°C, on injecte rapidement une solution de 12g de KOH (0,214 mol ; 0,43 équivalents) dans 10ml d'eau, tout en poursuivant le chauffage. La suspension blanche se transforme en une solution limpide jaune vif. Cette solution est maintenue à reflux pendant 1h.
L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout d'1h.

On ajoute ensuite à ce solide 600ml de xylène, et la suspension résultante est portée au reflux sous forte agitation mécanique, de façon à bien redisperser le précurseur solide pendant cette durée.

Après neutralisation par un mélange de 500ml de THF/50 ml d'HCl 37% sous forte agitation et filtration, on récupère un solide blanc constitué à 65% de calixarènes géants (analye RMN 1H).

**Exemple 2.5 : Préparation de *p*-(dibenzylamino)calixarènes :**

Un ballon tricol de 2 litres équipé d'un agitateur mécanique, d'un collecteur de Dean-Stark et d'un réfrigérant ainsi que d'un chauffage par bain d'huile, est chargé avec 150,66g de 4-(dibenzylamino)phénol (0,5 mol) et 135 ml de formaldéhyde en solution aqueuse à 30%.

La suspension résultante est chauffée à 140°C affichés sous protection d'argon.

A 90°C, on injecte rapidement une solution de 12g de KOH (0,214 mol ; 0,43 équivalents) dans 10ml d'eau, tout en poursuivant le chauffage. La suspension blanche se transforme en une solution limpide jaune vif. Cette solution est maintenue à reflux pendant 1h.

L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout d'1h.

On ajoute ensuite à ce solide 600ml de xylène, et la suspension résultante est portée au reflux sous forte agitation mécanique, de façon à bien redisperser le précurseur solide pendant cette durée.

Après neutralisation par un mélange de 500ml de THF/50 ml d'HCl 37% sous forte agitation et filtration, on récupère un solide blanc constitué à 55% de calixarènes géants (analye RMN 1H).

**Exemple 2.5 : Préparation de *p*-(méthyl)calixarènes en présence de CsOH (0,4 équivalent):**

Un ballon tricol de deux litres équipé d'un agitateur mécanique et d'un collecteur d'eau de type « Dean-Stark » est chargé d'une suspension de 54,03g (0,52 mol) de 4-(methyl)phénol (p-crésol), 36 ml d'une solution de CsOH à 50% (0,208 mol) et 100ml d'une solution de formaldehyde à 37% (1,34 mol). Après reflux pendant 20 minutes, on obtient une solution limpide jaune vif.

Tout en maintenant le reflux, on établit alors un fort courant d'argon à l'intérieur du ballon, dans le but d'éliminer l'eau. Le milieu réactionnel initialement très fluide devient de plus en plus visqueux, jusqu'à se solidifier complètement en un solide cassant de couleur jaune.

Après retour à température ambiante, on ajoute alors 200ml de xylène. La suspension ainsi obtenue est alors portée à reflux sous agitation. Un suivi de la réaction montre alors une évolution de la composition vers la formation de calixarènes géants.

Au bout de 2h de reflux, on ajoute 50ml d'acide chlorhydrique à 37%, ainsi que 500ml de tétrahydrofurane, sous forte agitation mécanique. La suspension résultante est évaporée à sec.

Une analyse RMN 1H montre que ce solide est constitué à 50% de calixarènes géants.

**B) Exemples de fonctionnalisation des calixarènes géants**

**Pegylation (PEG2) de la couronne basse d'un calixarène géant (KOH 0,4 équivalent sans agitation, nombre de motifs phénoliques au pic=60)**

Dans un tricol de 25 mL, on introduit 1 g de calixarènes géants et 5 mL de 2-(2-ethoxyethoxy)ethyl bromide sous argon. On chauffe à 60 °C et on introduit sous forte agitation, lentement, une suspension de 377.4 mg d'hydrure de sodium (60 % dans la paraffine, 9.43 mmol, 2 équivalents par phénol) dans 5 mL de DMF. Après une nuit à 60°C, le milieu réactionnel est refroidi à température ambiante et on ajoute 50 mL de méthanol, on filtre et on rince le solide avec de l'eau puis du méthanol. Le solide est placé sous agitation dans 50 mL d'acétonitrile pendant 2h puis filtré et séché sous vide. On obtient 1.41 g ($\eta$=91%) d'un solide de couleur orangée qui se solubilise à chaud dans le DMSO. $^{1}$H RMN (DMSO-$d_6$, 50°C, $\delta$, ppm) : 7.22 (s, 5H, aromatiques) ; 6,54 (s, 2H, hydroquinones) ; 4,83 (s, 2H, méthylènes benzyliques) ; 4.05 (s, 2H, PEG) ; 3.85 (s, 2H, méthylènes pontant) ; 3.69 (s, 2H, PEG) ; 3.54 (s, 2H, PEG) ; 3.44 (s, 2H, PEG),; 3.36 (m, 2H, PEG), 1.01 (s, 3H, PEG).

**Acétylation de la couronne basse d'un calixarène géant (nombre de motifs phénoliques au pic=60)**

Dans un tricol de 10 mL, on introduit 0.6 g de calixarène, 1.6 mL de bromoacétate d'éthyle, 3.91 g de carbonate de potassium (2.61 mmol, 80 équivalents par phénol), 100 mg de sulfate de tetrabutylammonium (0.29 mmol) et 5 mL d'acétonitrile. On chauffe une nuit à 75°C et on ajoute 50 mL de méthanol. On filtre, on rince à l'eau puis au méthanol. Le solide obtenu est séché sous vide (787 mg, $\eta$=91%). $^1$H RMN (DMSO-$d_6$, $\delta$, ppm) : 7.22 (s, 5H, aromatiques) ; 6,52 (s, 2H, hydroquinones) ; 4,80 (s, 2H, méthylène benzylique) ; 4.29 (s, 2H, méthylène en alpha du carbonyle) ; 4.05 (m, 2H, méthylène fonction éthyle) ; 3.96 (s, 2H, méthylène pontant) ; 1.11 (s, 3H, méthyle).

**Exemple C) Utilisation de calixarènes géants pour la synthèse de nanomatériaux : polymères en étoile**

La procédure utilisée est en deux étapes. Au cours de la première, les groupements phénoliques du calixarène sont acylés par le bromure de bromopropionyle. Ces groupements constituent des amorceurs de polymérisation, utilisés au cours d'une deuxième étape pour la croissance de chaînes polystyrène.

**Synthèse de l'amorceur pour le calix[50]arène.**

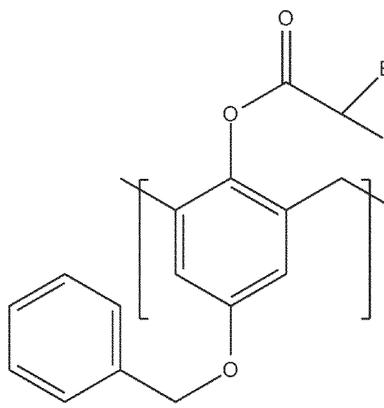

n=50.

Dans un tube de Schlenk, on introduit le calixarène géant (500mg, 0,0002mol). Sous argon, on ajoute alors 5 mL de DMF et 1,5 mL de $Et_3N$. Le système est plongé dans un bain de glace. On introduit ensuite 300 $\mu$L de bromure de bromopropionyle. La solution est agitée pendant 1h. Une précipitation est réalisée dans 50 mL MeOH. On filtre sous vide. L'amorceur est solubilisé dans quelques millilitres de THF. Puis on réalise une deuxième précipitation avec 50mL de MeOH. On filtre sous vide. Le produit est séché au dessiccateur.
Rendement = 60%.
RMN $^1$H (350MHz, $CDCl_3$) $\delta_{ppm}$ : 7,1-7,6 (m, 60H, -$OCH_2$-*Ar*), 6,8-6,4 (m, 24H, Ar), 5,1-4,7 (m, 24H, -O*$CH_2$*-Ar-), 4-3,5 (m, 24H, -Ar(phenol)-*$CH_2$*-), 3-1,5 (m, 24H, $CH_3$-*CH*(COR)-Br), 2-1,5 (m, 36H, *$CH_3$*-CH(COR)-Br).

Ce macroamorceur est également caractérisé par chromatographie d'exclusion stérique (figure 24).

**Synthèse du polymère en étoile par polymérisation radicalaire par transfert d'atomes (ATRP)**

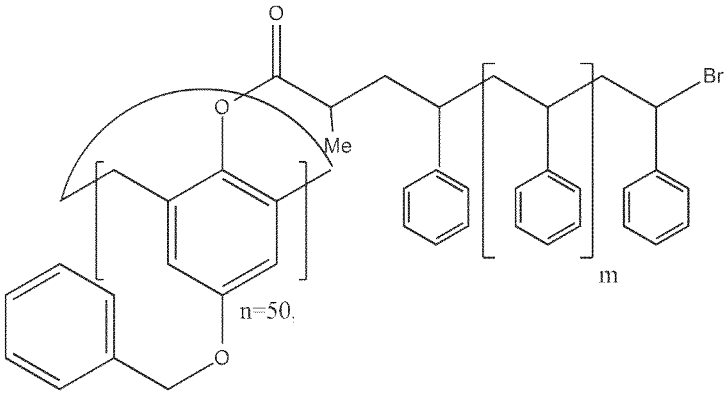

Dans un shlenk sous argon, on introduit CuBr (25mg, 0,00017mol), la bipyridine (80mg, 0,00052mol), l'amorceur précédemment synthétisé (60mg, 0,00017mol) et 2mL de styrène. Le milieu réactionnel est dégazé par 3 cycles de congélation-décongélation. Le tube de schlenk est plongé dans un bain d'huile chauffé à 100°C pendant un temps donné. Le mélange est solubilisé dans du THF. Le mélange est filtré sur une colonne d'$Al_2O_3$ basique. Le polymère est précipité dans du MeOH (avec un rapport $\frac{THF}{MeOH} = 10$), puis filtré sous vide et rincé avec du MeOH. Le produit est séché au dessiccateur.
Rendement = 50%.
RMN $^1H$ (360MHz, $CDCl_3$) $\delta_{ppm}$ : 7,1-7,6 (m, 5H, -$OCH_2$-*Ar*), 7,2-6,9 (m, 5H, $Ar_{styrène}$), 7,2-6,9 (m, 1H, -$CH_2$-*CH*(Ar)-$CH_2$-), 6,8-6,4 (m, 2H, Ar), 2,1-1,6 (m, 2H, -CH-$CH_2$-CH-), 2,1-1,6 (m, 1H, $HO_2C$-*CH*($CH_3$)-$CH_2$-), 1,6-1,3 (m, 3H, $CH_3$-CH-).

Les polymères en étoile obtenus, qui diffèrent par la durée de l'ATRP, sont également caractérisés par chromatographie d'exclusion stérique (figure 25).

**Etude de la structure des polymères en solution.**

Pour étudier le comportement desdits polymères en étoile en solution, on peut utiliser une loi d'échelle :

$$Rh = KM^{\alpha}$$

où Rh correspond au rayon hydrodynamique, M correspond à la masse molaire ; K et $\alpha$ sont des coefficients. Les termes K et $\alpha$ sont des constantes qui varient avec le polymère.

$$\log Rh = \log K + \alpha \log M$$

Le terme $\alpha$ nous donne des informations sur le comportement du polymère en solution:
Si $\alpha$ tend vers 1 alors le polymère se comporte comme un polymère linéaire.

Si $\alpha$ tend vers 0,5 alors le polymère se comporte comme une pelote statistique.

Si $\alpha$ tend vers 0 alors le polymère se comporte comme une sphère dure.

Le tracé du rayon hydrodynamique (mesuré par diffusion de la lumière) en fonction de la masse molaire (obtenue en mesurant la masse molaire des branches de polystyrène (après « décrochage » du coeur calixarène central par saponification) permet donc d'apporter des informations sur la structure de ces nanoobjets en solution (figure 26).

Dans le cas desdits polymères en étoile dérivé du calix[50]arène, l'exposant alpha tend vers 0 (comportement en solution type « sphère dure »). Les calixarènes géants présentent donc un comportement hydrodynamique spécifique type « sphère dure », et forment donc en solution (THF) des nanoobjets bien définis, potentiellement intéressants pour l'encapsulation et la vectorisation.

**Exemple 3 : Exemples comparatifs : Obtention de petits *p*-(benzyloxy)calixarène avec CsOH <0,5 eq et du xylène.**

**Exemple A : Obtention d'un composé consistant en du *p*-(benzyloxy)calix[6]arène ou du *p*-(benzyloxy)calix[7]arène sous forme de sel de césium ou neutralisée ou d'un mélange comprenant du *p*-(benzyloxy)calix[6]arène et du *p*-(benzyloxy)calix[7]arène avec de l'hydroxyde de césium.**

**Exemple A.1 : concentration en hydroxyde de césium de 0,15 équivalent**

Une suspension de 50,6g de *p*-(benzyloxy)phénol (0,254 mol), 20g (0,667 mol) de paraformaldéhyde (point de fusion : 135°C) dans 700 ml de xylène est mise sous argon dans un ballon tricol de 2 litres équipé d'un agitateur mécanique et d'un récupérateur d'eau type « Dean-Stark ».
La suspension est mise à chauffer sous agitation. Lorsque la température atteint 90°C, on ajoute rapidement à l'aide d'une seringue (et sous balayage d'argon) 7,4 ml (0,0425 mol) d'une solution de CsOH à 50% (en poids) dans l'eau.
La suspension est laissée à reflux pendant 6h, période au cours de laquelle on observe la formation d'un volumineux précipité blanc.
Ce précipité est filtré, lavé au xylène, puis au pentane. m=35g, rendement en *p*-(benzyloxy)calix[7]arène (sous forme de monosel de césium) : 58%
Les caractéristiques spectroscopiques de ce précipité correspondent à celle d'un monoanion du *p*-(benzyloxy)calix[7]arène.
RMN $^1H$ (DMSO-$d_6$) : (déplacements chimiques, ppm) 7,60-7,20 (multiplet, aromatiques) ; 6,72 (singulet fin, hydroquinone) ; 4,93 (singulet fin, protons benzyliques) ; 3,71 (singulet fin, méthylènes intracycliques).
Spectrométrie de masse (MALDI, matrice DHB) : m/z=1617,41 $(M+Cs)^+$.
Un échantillon de 1g de ce précipité est mis en suspension dans 5ml de dichlorométhane, puis neutralisé avec une solution aqueuse concentrée d'HCl, sous forte agitation pendant 24h.
La phase organique est récupérée, puis évaporée à sec au rotovapor.
On récupère 0,91g d'un solide blanc, dont les caractéristiques spectroscopiques sont en parfait accord avec le *p*-(benzyloxy)calix[7]arène neutre.
RMN $^1H$ (DMSO-$d_6$) : (déplacements chimiques, ppm) 7,30 (multiplet large, aromatiques) ; 6,62 (singulet fin, hydroquinone) ; 4,84 (singulet fin, protons benzyliques) ; 3,74 (singulet fin, méthylènes intracycliques).
Spectrométrie de masse (MALDI, matrice DHB) : m/z=1507,65 $(M+Na)^+$.
La perte de masse observée (90mg) est tout à fait en accord avec celle attendue pour la neutralisation d'un monosel de césium : *p*-(benzyloxy)calix[7]arène$^-$.$Cs^+$ → *p*-(benzyloxy)calix[7]arène.

**Exemple A.2 : concentration en hydroxyde de césium de 0,3 équivalent.**

Une suspension de 50,6g de *p*-(benzyloxy)phénol (0,254 mol), 20g (0,667 mol) de paraformaldehyde (point de fusion : 135°C) dans 700 ml de xylène est mise sous argon dans un ballon tricol de 2 litres équipé d'un agitateur mécanique et d'un récupérateur d'eau type « Dean-Stark ».
La suspension est mise à chauffer sous agitation. Lorsque la température atteint 90°C, on ajoute rapidement à l'aide d'une seringue (et sous balayage d'argon) 14,8 ml (0,085 mol) d'une solution de CsOH à 50% (en poids) dans l'eau.
La suspension est laissée à reflux pendant 5h30. On obtient alors une solution parfaitement limpide orange vif.
On ajoute alors 100ml d'une solution de HCl à 37% dans l'eau, et l'on observe la formation d'un précipité. La suspension est laissée sous forte agitation pendant deux jours, puis évaporée à sec à l'évaporateur rotatif.
Après lavage avec 500ml d'eau (élimination des sels et de l'excès d'HCl), le solide est dissout à chaud (130°C) dans 200ml de DMSO. On obtient alors une solution limpide noire, à laquelle sont ajoutés à chaud 2 1 d'acétone.
Après retour à température ambiante, cette solution limpide est abandonnée une semaine, pendant laquelle un précipité cristallin de *p*-(benzyloxy)calix[6]arène (18 g) se dépose sur les parois du ballon.
Le filtrat est évaporé au rotovapor, puis au pistolet chauffant jusqu'à obtention d'un solide noir. Ce solide est lavé à l'acétone, ce qui conduit à la récupération d'un deuxième lot de *p*-(benzyloxy)calix[6]arène (10 g). Total : 28g, rendement 51%.
RMN $^1H$ (DMSO-$d_6$) : (déplacements chimiques, ppm) 7,30 (multiplet large, aromatiques) ; 6,62 (singulet fin, hydroquinones) ; 4,79 (singulet fin, protons benzyliques) ; 3,72 (singulet fin, méthylènes intracycliques).
Spectrométrie de masse (MALDI, matrice DHB) : m/z=1295,49 $(M+Na)^+$.
**Obtention d'un composé consistant en du *p*-(benzyloxy)calix[6]arène ou du *p*-(benzyloxy)calix[7]arène sous forme neutralisée ou du *p*-(benzyloxy)calix[8]arène ou d'un mélange comprenant du *p*-(benzyloxy)calix[6]arène, du *p*-(benzyloxy)calix[7]arène et du p-(benzyloxy)calix[8]arène avec de l'hydroxyde de sodium ou de potassium.**

**Exemple B.1 : concentration en hydroxyde de sodium ou de potassium de 0,15 équivalent.**

Une suspension de 34,5g de *p*-(benzyloxy)phénol (0,173 mol), 20g (0,667 mol) de paraformaldehyde (point de fusion : 135°C) dans 450ml de xylène est mise sous argon dans un ballon tricol de 1 l équipé d'un agitateur mécanique et d'un récupérateur d'eau type « Dean-Stark ».
La suspension est mise à chauffer sous agitation. Lorsque la température atteint 95°C, on ajoute rapidement à l'aide d'une seringue (et sous balayage d'argon) une solution de 1,43g (0,03 mol) de KOH dans 6ml d'eau Millipore. On observe l'apparition immédiate d'une coloration jaune.
Le milieu réactionnel est porté au reflux pendant 3h30, période pendant laquelle on observe la formation d'un abondant précipité blanc et d'une solution orange vif.
Après retour à température ambiante, le précipité est récupéré par filtration, lavé avec 100ml de xylène et 300ml de pentane.
L'analyse de ce précipité indique qu'il est constitué de *p*-(benzyloxy)calix[7]arène pur. M = 20g, rendement 54%.

Caractérisations :

RMN $^1H$ (DMSO-$d_6$) : (déplacements chimiques, ppm) 7,30 (multiplet large, aromatiques) ; 6,62 (singulet fin, hydroquinone) ; 4,84 (singulet fin, protons benzyliques) ; 3,74 (singulet fin, méthylènes intracycliques).
Spectrométrie de masse (MALDI, matrice DHB) : m/z=1524,62 $(M+K)^+$.

**Exemple B.2 : concentration en hydroxyde de sodium ou de potassium de 0,3 équivalent.**

Une suspension de 51,5g de *p*-(benzyloxy)phénol (0,258 mol), 20g (0,667 mol) de paraformaldehyde (point de fusion : 135°C) dans 700 ml de xylène est mise sous argon dans un ballon tricol de 2 l équipé d'un agitateur mécanique et d'un récupérateur d'eau type « Dean-Stark ».
La suspension est mise à chauffer sous agitation. Lorsque la température atteint 90°C, on ajoute rapidement à l'aide d'une seringue (et sous balayage d'argon) 3,056g de NaOH (0,0764 mol) dans 10 ml d'eau.
La suspension est laissée à reflux pendant 4h30, période au bout de laquelle le milieu réactionnel prend en masse.
Après retour à température ambiante, le milieu réactionnel est neutralisé par 500ml d'une solution d'HCl 2M, sous très forte agitation.
L'émulsion résultante est évaporée à sec et lavée avec 500ml d'eau (élimination des sels de sodium).
Le solide orangé résultant est lavé avec 500ml de THF et le précipité blanc résultant est filtré, ce qui conduit à la récupération de 20g de *p*-(benzyloxy)calix[8]arène pur. Rendement : 36,6%.
RMN$^1H$ (DMSO-$d_6$) (déplacements chimiques, ppm) 7,30 (multiplet large, aromatiques) ; 6,58 (singulet fin, hydroquinones) ; 4,80 (singulet fin, protons benzyliques) ; 3,77 (singulet fin, méthylènes intracycliques).
Spectrométrie de masse (MALDI, matrice DHB) : m/z=1719,62 $(M+Na)^+$.
Le filtrat correspondant est évaporé à sec, et dissout à chaud dans 45 ml de DMSO, ce qui conduit à la formation d'une solution homogène noire. 1l de toluène y est ajouté, et la solution limpide orange foncée est placée au congélateur (-23°C) pendant 1 semaine, ce qui conduit à la formation d'un précipité microcristallin. Ce précipité est filtré, et son analyse par RMN $^1H$ montre qu'il s'agit de *p*-(benzyloxy)calix[6]arène pur ; m=6,3g, 11%.
RMN $^1H$ (DMSO-$d_6$) . (déplacements chimiques, ppm) 7,30 (multiplet large, aromatiques) ; 6,62 (singulet fin, hydroquinone) ; 4,79 (singulet fin, protons benzyliques) ; 3,72 (singulet fin, méthylènes intracycliques).
Spectrométrie de masse (MALDI, matrice DHB) : m/z=1295,49 $(M+Na)^+$.
Le filtrat DMSO/toluène correspondant est évaporé jusqu'à obtention d'un liquide orangé. Après ajout de 1l de méthanol et filtration, on récupère 28,5g de *p*-(benzyloxy)calix[7]arène pur. Rendement : 52%.
RMN $^1H$ (DMSO-d6): (déplacements chimiques, ppm) 7,30 (multiplet large, aromatiques) ; 6,62 (singulet fin, hydroquinones) ; 4,84 (singulet fin, protons benzyliques) ; 3,74 (singulet fin, méthylènes intracycliques).
Spectrométrie de masse (MALDI, matrice DHB) : m/z=1524,62 $(M+K)^+$.

**Exemple 4: Exemple comparatif : Réaction de *p*-(*t*-butyl)phénol avec une base à <0,5 eq dans l'eau sans solvant organique.**

Un ballon tricol de 2 litres équipé d'un agitateur mécanique, d'un collecteur de Dean-Stark et d'un réfrigérant ainsi que d'un chauffage par bain d'huile, est chargé avec 100g de 4-(tBu)phénol (0,666 mol) et 135 ml de formaldéhyde en solution aqueuse à 37%.
La suspension résultante est chauffée à 140°C affichés sous protection d'argon.
A 90°C, on injecte rapidement une solution de 15g de KOH (0,266 mol ; 0,4 équivalent) dans 10ml d'eau, tout

en poursuivant le chauffage. La suspension blanche se transforme en une solution limpide jaune vif. Cette solution est maintenue à reflux pendant 1h.

L'un des rodages du ballon est alors équipé d'un col de cygne relié à un bulleur, et la solution est alors soumise à un fort balayage d'argon (20 bulles/s), l'entrée d'argon se faisant par le sommet du réfrigérant.

Au bout d'environ 40min de balayage, la solution initialement limpide et fluide devient de plus en plus visqueuse, pour finalement prendre en masse au bout de 1h.

L'analyse RMN de ce solide (DMSO-d6) est présentée à la figure 17.1.

Ce solide semble essentiellement constitué de *p*-(tBu)calix[8]arène, par comparaison avec l'échantillon de référence de ce composé dans le DMSO-d6 (figure 17.2).

On ajoute ensuite à ce solide 400ml de toluène, et la suspension résultante est portée au reflux sans agitation pendant 2h.

Après neutralisation par un mélange de 500ml de THF/50 ml d'HCl 37% sous forte agitation, l'analyse RMN de ce solide ($CDCl_3$, figure 18) montre que le produit obtenu comporte une proportion non négligeable de *p*-(tBu)calix[4,6,8]arènes (comparaison avec des échantillons authentiques), ainsi qu'une forte proportion de polymères (signaux larges).

L'évolution caractéristique de la signature spectroscopique des méthylènes pontants des calixarènes de grande taille (9 à 16 motifs de répétition) dans le $CDCl_3$ n'est pas observée (C. D. Gutsche et collaborateurs, JACS 1999).

Par ailleurs, la présence de signaux intenses vers 5ppm indique une forte proportion d'oligomères linéaires.

### Exemple 5: Exemple comparatif : préparation d'un mélange de *p*-benzyloxycalix[9-20]arènes avec CsOH ou RbOH (0,6 eq)

Dans un ballon bicol de 2l équipé d'un condenseur de Dean-Stark et d'un réfrigérant, on prépare sous argon une suspension de 50g (0,25 mol) de 4-(benzyloxy)phénol et de 18g (0,6 mol) de paraformaldéhyde dans 700ml de xylène.

La suspension est mise en chauffe sous argon et forte agitation magnétique, et à 90°C on ajoute rapidement 26 ml d'une solution de CsOH à 50% dans l'eau.

Le milieu réactionnel est porté au reflux pendant 6h, période au cours de laquelle on obtient une solution limpide orangée.

Une analyse RMN (DMSO-d6, figure 19) de cette solution montre qu'elle comporte environ 60% de grands calixarènes (identifiables par des signaux caractéristiques vers 6,52 ppm).

Cette solution est neutralisée par ajout de 200ml de THF contenant 20ml d'HCl à 37% sous forte agitation.

La suspension résultante est évaporée, et lavée avec 200ml d'acétonitrile (élimination des petits calixarènes).

Le solide résultant est dissout dans 1l d'un mélange acétone/DMSO (90/10 en volume), filtré (élimination du calix[8]arène) et abandonné 4 jours au réfrigérateur (1°C).

On récupère 15g d'un solide microcristallin.

Ce solide est constitué d'un mélange de grands calixarènes.

Ses différents constituants peuvent être séparés par une succession de recristallisations/chromatographies dans différents solvants. Le schéma de purification correspondant est donné à la figure 20.

## Revendications

1. Utilisation d'au moins une base en solution aqueuse, avec au moins un phénol substitué en position 4 de formule (I) suivante :

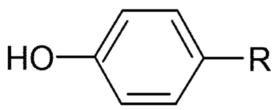

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ linéaire,

- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther -S-$CH_2$-Ph éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),
- un groupe -$NR_aR_b$, $R_a$ et $R_b$ représentant indépendamment l'un de l'autre un alkyle $C_1$-$C_{20}$ ramifié ou linéaire,
- un groupe dibenzylamine de formule -N(benzyle)$_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

et une source de formaldéhyde aqueux,
en l'absence ou en présence de solvant organique, constituant ainsi un milieu réactionnel, ladite base étant à une concentration totale comprise de 0,01 à 1 équivalent, par rapport audit au moins un phénol substitué en position 4 de formule (I),
ledit milieu réactionnel étant chauffé à une température comprise de 100 à 130°C, et, ledit milieu réactionnel étant éventuellement soumis ensuite à un traitement thermique en présence de moyens de transmission de chaleur,
pour la mise en oeuvre d'une réaction de préparation d'un mélange de *p*-(R)calixarènes géants dont la taille est supérieure à 20.

**2.** Utilisation selon la revendication 1, dans laquelle de l'eau est produite lors de la réaction, la dite eau étant éliminée du milieu réactionnel durant ledit chauffage à une température comprise de 100 à 130°C et un précurseur solide sous forme de résine dure cassante, éventuellement isolé, est obtenu.

**3.** Utilisation selon l'une des revendications 1 à 2, dans laquelle ledit précurseur solide sous forme de résine dure cassante est précédé de la formation d'un dimère phénolique, éventuellement isolé.

**4.** Utilisation selon la revendication 2 ou 3, dans laquelle ledit précurseur solide sous forme de résine dure cassante ou ledit dimère phénolique, éventuellement isolé du milieu réactionnel, est mis en présence de moyens de transmission de chaleur sous la forme d'un four ou d'un solvant dans lequel ledit précurseur solide sous forme de résine dure cassante et ledit dimère phénolique sont insolubles, et est soumis audit traitement thermique,
pour la préparation d'un mélange de *p*-(R)calixarènes géants dont la taille est comprise de 21 à 50 unités phénoliques.

**5.** Utilisation selon la revendication 1, dans laquelle de l'eau est produite lors de la réaction, ladite eau étant conservée dans le milieu réactionnel durant ledit chauffage et un précurseur solide, sous forme de précipité, éventuellement isolé, est obtenu.

**6.** Mélange de calixarènes de formule (IV) suivante :

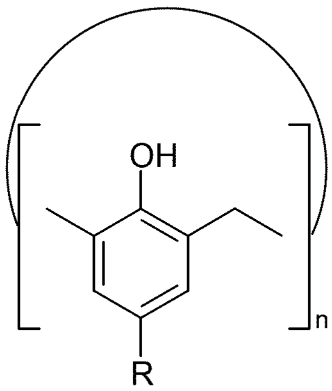

(IV)

dans lequel n est un entier compris de 21 à plus de 220, R étant tel que défini dans la revendication 1.

**7.** Procédé de préparation d'un mélange de *p*-(R)calixarènes géants dont la taille est supérieure à 20, comprenant une étape de mise en contact d'au moins une base, avec au moins un phénol substitué en position 4 de formule (I) suivante :

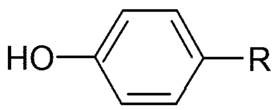

(I)

dans laquelle R est choisi parmi :

- un groupe benzyle éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyle en $C_1$-$C_{20}$ linéaire,
- un groupe benzyloxy éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkyloxy en $C_1$-$C_{20}$ ramifié ou linéaire, un PEG-1 à 10 ramifié ou linéaire, dont le groupe terminal -OH est alkylé par un alkyle $C_1$-$C_{20}$ ramifié ou linéaire,
- un groupe benzylthioéther $-S-CH_2-Ph$ éventuellement substitué en ortho et/ou para et/ou méta par un ou plusieurs substituants choisis parmi un halogène, SR' et OR', $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,
- un groupe alkylthioéther en $C_1$-$C_{20}$ ramifié ou linéaire, de formule -S-($C_1$-$C_{20}$-alkyle),
- un groupe $-NR_aR_b$, $R_a$ et $R_b$ représentant indépendamment l'un de l'autre un alkyle $C_1$-$C_{20}$ ramifié ou linéaire,
- un groupe dibenzylamine de formule $-N(benzyle)_2$, dans lequel les deux groupes benzyle sont, indépendamment l'un de l'autre, éventuellement substitué en ortho et/ou para et/ou méta par un ou plusieurs substituants choisis parmi un halogène, SR' et OR', R' représentant une chaine alkyle en $C_1$-$C_{20}$ linéaire ou ramifiée, un groupe cycloalkyle en $C_3$ à $C_{20}$, $NR_aR_b$, $PR_aR_b$, $P(O)R_aR_b$, $P(O)OR_aOR_b$, $R_a$ et $R_b$ représentant indépendamment l'un de l'autre un alkyle $C_1$-$C_{20}$ ramifié ou linéaire,

et une source de formaldéhyde aqueux,
en l'absence ou en présence de solvant organique, constituant ainsi un milieu réactionnel, ladite base étant à une concentration totale comprise de 0,01 à 1 équivalent, par rapport audit au moins un phénol substitué en position 4 de formule (I),
ledit milieu réactionnel étant chauffé à une température comprise de 100 à 130°C, pour obtenir un milieu réactionnel chauffé,
et comprenant, éventuellement, après ledit chauffage, une étape de traitement thermique dudit milieu réactionnel

chauffé, en présence de moyens de transmission de chaleur.

**8.** Procédé selon la revendication 7, dans lequel l'eau produite lors de la réaction est éliminée dudit milieu réactionnel durant ledit chauffage et un précurseur solide sous forme de résine dure cassante, éventuellement isolé, est obtenu.

**9.** Procédé selon la revendication 7, dans lequel l'eau produite lors de la réaction est éliminée dudit milieu réactionnel durant ledit chauffage et un précurseur solide sous forme de résine dure cassante, éventuellement isolé, est obtenu, dans lequel ledit précurseur solide sous forme de résine dure cassante est précédé de la formation d'un dimère phénolique, éventuellement isolé,
comprenant les étapes suivantes :

a. mise en contact d'au moins une base, avec au moins un phénol substitué en position 4 de formule (I), avec du formaldéhyde aqueux, pour constituer un milieu réactionnel et chauffage dudit milieu réactionnel durant un temps compris de 30 minutes à 2 heures, tout en éliminant l'eau formée,
pour obtenir un dimère de formule (II) suivante :

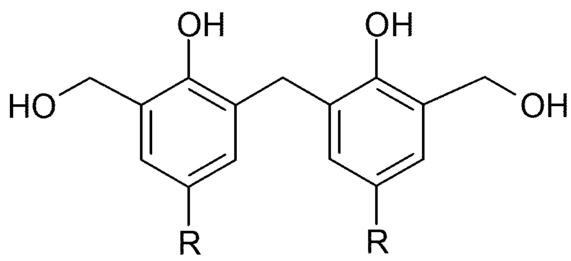
(II)

b. éventuellement poursuite du chauffage dudit milieu réactionnel contenant ledit dimère tout en éliminant l'eau formée pour obtenir un milieu réactionnel contenant un précurseur solide, sous forme de résine dure cassante, durant 1 à 3 heures, et optionnellement filtration dudit précurseur solide, sous forme de résine dure cassante,
c. chauffage dudit milieu réactionnel à l'aide de moyens de transmission de chaleur sous la forme d'un four, ou addition audit milieu réactionnel contenant ledit précurseur solide sous forme de résine dure cassante d'un solvant organique dans lequel ledit précurseur solide sous forme de résine dure cassante est insoluble, pour obtenir un milieu réactionnel contenant un solide sous forme de résine dure cassante, dans un four ou un solvant et chauffage dudit milieu réactionnel contenant un solide sous forme de résine dure cassante, durant 3 à 8 heures, pour obtenir un mélange de calixarènes géants dans lequel R est tel que défini dans la revendication 1, comprenant de plus un *p*-(R)calix[7]arène et/ou un *p*-(R)calix[8]arène,
d. recristallisation, après neutralisation de la base, dudit mélange de calixarènes géants, dans un solvant à base de DMSO, pour obtenir un mélange de calixarènes géants purifié dans lequel R est tel que défini dans la revendication 1, substantiellement dépourvu en *p*-(R)calix[7]arène et/ou en *p*-(R)calix[8]arène.
e. éventuellement, purification par GPC, en séparant différentes fractions en fonction de leur temps d'élution.

**10.** Procédé selon la revendication 7, dans lequel l'eau produite lors de la réaction est conservée dans ledit milieu réactionnel durant ledit chauffage et un précurseur solide sous forme de précipité, éventuellement isolé, est obtenu.

**11.** Procédé selon la revendication 10, dans lequel ledit précurseur solide sous forme de précipité est isolé du susdit milieu réactionnel, comprenant les étapes suivantes :

a. mise en contact d'au moins une base, avec au moins un phénol substitué en position 4 de formule (I), avec du formaldéhyde aqueux, pour constituer un milieu réactionnel et chauffage dudit milieu réactionnel durant un temps compris de 30 minutes à 1 heure, sans élimination de l'eau formée, pour obtenir un dimère de formule (II) tel que défini dans la revendication 9,
b. éventuellement poursuite du chauffage dudit milieu réactionnel contenant ledit dimère sans élimination de l'eau formée, durant 2 à 3 heures, pour obtenir un milieu réactionnel contenant un précurseur solide sous forme de précipité, et optionnellement filtration dudit précurseur solide sous forme de précipité,
c. chauffage dudit milieu réactionnel à l'aide de moyens de transmission de chaleur sous la forme d'un four, ou addition audit milieu réactionnel contenant ledit précurseur solide sous forme de précipité d'un solvant organique dans lequel ledit précurseur solide sous forme de précipité est insoluble, pour obtenir un milieu réactionnel contenant un solide sous forme de précipité dans un four ou un solvant et chauffage dudit milieu réactionnel

contenant un solide sous forme de précipité, durant 3 à 8 heures,
pour obtenir un mélange de calixarènes géants dans lequel R est tel que défini dans la revendication 1, comprenant de plus un *p*-(R)calix[7]arène et/ou un *p*-(R)calix[8]arène,
d. recristallisation, après neutralisation de la base, dans un solvant à base de DMSO, pour obtenir un mélange de calixarènes géants purifié dans lequel R est tel que défini dans la revendication 1, substantiellement dépourvu de *p*-(R)calix[7]arène et/ou un *p*-(R)calix[8]arène,
e. éventuellement, purification dudit mélange de calixarènes géants purifié dépourvu en *p*-(R)calix[7]arène et/ou en *p*-(R)calix[8]arène par GPC, en séparant différentes fractions en fonction de leur temps d'élution.

**12.** Procédé selon l'une quelconque des revendications 7 à 11, dans lequel ladite base est choisie parmi l'hydroxyde de baryum, l'hydroxyde de rubidium, l'hydroxyde de lithium, l'hydroxyde de césium, la potasse ou la soude.

**13.** Utilisation d'un mélange de *p*-(R)calixarènes géants dont la taille est supérieure à 20, R étant tel que défini dans la revendication 1, pour la constitution d'un matériau ou dans le cadre du renfort des matériaux.

**14.** Utilisation d'un mélange de calixarènes selon la revendication 6, susceptible d'être obtenu par le procédé défini dans l'une des revendications 7 à 12, pour la constitution d'un matériau ou dans le cadre du renfort des matériaux.

## Patentansprüche

**1.** Verwendung mindestens einer Base in wässriger Lösung, mit mindestens einem Phenol, substituiert in Position 4, mit der folgenden Formel (I):

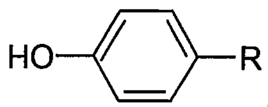
(I)

wobei R ausgewählt ist aus:

- einer Benzylgruppe, gegebenenfalls substituiert in ortho und/oder para und/oder meta durch einen oder mehrere Substituenten, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,
- einer linearen $C_1$-$C_{20}$-Alkylgruppe,
- einer Benzyloxygruppe, gegebenenfalls substituiert in ortho und/oder para und/oder meta durch einen oder mehrere Substituenten, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,
- einer verzweigten oder linearen $C_1$-$C_{20}$-Alkyloxygruppe, einem verzweigten oder linearen PEG-1 bis 10, dessen Endgruppe -OH durch ein verzweigtes oder lineares $C_1$-$C_{20}$-Alkyl alkyliert ist,
- einer Benzylthioethergruppe $-S-CH_2-Ph$, gegebenenfalls substituiert in ortho und/oder para und/oder meta durch einen oder mehrere Substituenten, ausgewählt aus einem Halogen, SR' und OR', $NR_aR_b$, $PR_aR_b$, $P(O)R_aR_b$, $P(O)OR_aOR_b$, wobei $R_a$ und $R_b$ unabhängig voneinander ein verzweigtes oder lineares $C_1$-$C_{20}$-Alkyl darstellen,
- einer verzweigten oder linearen $C_1$-$C_{20}$-Alkylthioethergruppe mit der Formel -S-($C_1$-$C_{20}$-Alkyl),
- einer Gruppe $NR_aR_b$, wobei $R_a$ und $R_b$ unabhängig voneinander ein verzweigtes oder lineares $C_1$-$C_{20}$-Alkyl darstellen,
- einer Dibenzylamingruppe mit der Formel $-N(Benzyl)_2$, wobei die beiden Benzylgruppen unabhängig voneinander gegebenenfalls in ortho und/oder para und/oder meta durch einen oder mehrere Substituenten substituiert sind, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,

und einer wässrigen Formaldehydquelle,
in Abwesenheit oder in Anwesenheit eines organischen Lösungsmittels, wobei somit ein Reaktionsmedium gebildet

wird,
wobei die Base eine Gesamtkonzentration von 0,01 bis Äquivalent, im Verhältnis zu dem mindestens einen Phenol, substituiert in Position 4, mit der Formel (I), aufweist,
wobei das Reaktionsmedium auf eine Temperatur von 100 bis 130°C erhitzt wird, wobei das Reaktionsmedium gegebenenfalls anschließend einer Wärmebehandlung in Anwesenheit von Wärmeübertragungsmitteln unterzogen wird,
zur Durchführung einer Reaktion zur Herstellung einer Mischung von Riesen p-(R) Calixarenen, deren Größe mehr als 20 beträgt.

2. Verwendung nach Anspruch 1, wobei das Wasser bei der Reaktion erzeugt wird, wobei das Wasser aus dem Reaktionsmedium während des Erhitzens auf eine Temperatur von 100 bis 130°C eliminiert wird, und ein fester Vorläufer in der Form eines spröden harten Harzes, gegebenenfalls isoliert, erhalten wird.

3. Verwendung nach einem der Ansprüche 1 bis 2, wobei dem festen Vorläufer in der Form eines spröden harten Harzes die Bildung eines Phenoldimers, gegebenenfalls isoliert, vorausgeht.

4. Verwendung nach Anspruch 2 oder 3, wobei der feste Vorläufer in der Form eines spröden harten Harzes oder das Phenoldimer, gegebenenfalls isoliert von dem Reaktionsmedium, Wärmeübertragungsmitteln in der Form eines Ofens oder eines Lösungsmittels ausgesetzt wird, in dem der feste Vorläufer in der Form eines spröden harten Harzes und das Phenoldimer unlöslich sind, und der genannten Wärmebehandlung unterzogen wird,
zur Herstellung einer Mischung von Riesen p-(R) Calixarenen, deren Größe von 21 bis 50 Phenoleinheiten beträgt.

5. Verwendung nach Anspruch 1, wobei das Wasser bei der Reaktion erzeugt wird, wobei das Wasser in dem Reaktionsmedium während des Erhitzens gehalten wird, und ein fester Vorläufer, in gefällter Form, gegebenenfalls isoliert, erhalten wird.

6. Mischung von Calixarenen mit der folgenden Formel (IV) :

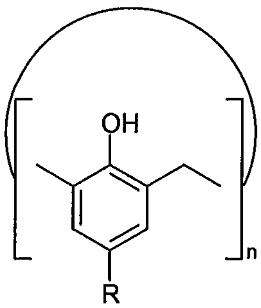
(IV)

wobei n eine ganze Zahl von 21 bis mehr als 220 ist, wobei R wie in Anspruch 1 definiert ist.

7. Verfahren zur Herstellung einer Mischung von Riesen p-(R) Calixarenen, deren Größe mehr als 20 beträgt, umfassend einen Schritt des Inberührungbringens mindestens einer Base, mit mindestens einem Phenol, substituiert in Position 4, mit der folgenden Formel (I) :

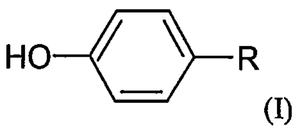
(I)

wobei R ausgewählt wird aus:

- einer Benzylgruppe, gegebenenfalls substituiert in ortho und/oder para und/oder meta durch einen oder mehrere Substituenten, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,

- einer linearen $C_1$-$C_{20}$-Alkylgruppe,
- einer Benzyloxygruppe, gegebenenfalls substituiert in ortho und/oder para und/oder meta durch einen oder mehrere Substituenten, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,
- einer verzweigten oder linearen $C_1$-$C_{20}$-Alkyloxygruppe, einem verzweigten oder linearen PEG-1 bis 10, dessen Endgruppe -OH durch ein verzweigtes oder lineares $C_1$-$C_{20}$-Alkyl alkyliert ist,
- einer Benzylthioethergruppe -S-$CH_2$-Ph, gegebenenfalls substituiert in ortho und/oder para und/oder meta durch einen oder mehrere Substituenten, ausgewählt aus einem Halogen, SR' und OR', $NR_aR_b$, $PR_aR_b$, $P(O)R_aR_b$, $P(O)OR_aOR_b$, wobei $R_a$ und $R_b$ unabhängig voneinander ein verzweigtes oder lineares $C_1$-$C_{20}$-Alkyl darstellen,
- einer verzweigten oder linearen $C_1$-$C_{20}$-Alkylthioethergruppe mit der Formel -S-($C_1$-$C_{20}$-Alkyl),
- einer Gruppe $NR_aR_b$, wobei $R_a$ und $R_b$ unabhängig voneinander ein verzweigtes oder lineares $C_1$-$C_{20}$-Alkyl darstellen,
- einer Dibenzylamingruppe mit der Formel -N(Benzyl)$_2$, wobei die beiden Benzylgruppen unabhängig voneinander gegebenenfalls in ortho und/oder para und/oder meta durch einen oder mehrere Substituenten substituiert sind, ausgewählt aus einem Halogen, SR' und OR', wobei R' eine lineare oder verzweigte $C_1$-$C_{20}$-Alkylkette, eine $C_3$-$C_{20}$-Cycloalkylgruppe, $NR_aR_b$, $PR_aR_b$, $P(O)R_aR_b$, $P(O)OR_aOR_b$ darstellt, wobei $R_a$ und $R_b$ unabhängig voneinander ein verzweigtes oder lineares $C_1$-$C_{20}$-Alkyl darstellen,

und einer wässrigen Formaldehydquelle,
in Abwesenheit oder in Anwesenheit eines organischen Lösungsmittels, wobei somit ein Reaktionsmedium gebildet wird,
wobei die Base eine Gesamtkonzentration von 0,01 bis Äquivalent, im Verhältnis zu dem mindestens einen Phenol, substituiert in Position 4, mit der Formel (I), aufweist,
wobei das Reaktionsmedium auf eine Temperatur von 100 bis 130°C erhitzt wird, um ein erhitztes Reaktionsmedium zu erhalten,
und umfassend gegebenenfalls, nach dem Erhitzen, einen Schritt einer Wärmebehandlung des erhitzten Reaktionsmediums, in Anwesenheit von Wärmeübertragungsmitteln.

**8.** Verfahren nach Anspruch 7, wobei das bei der Reaktion erzeugte Wasser aus dem Reaktionsmedium während des Erhitzens eliminiert wird, und ein fester Vorläufer in der Form eines spröden harten Harzes, gegebenenfalls isoliert, erhalten wird.

**9.** Verfahren nach Anspruch 7, wobei das bei der Reaktion erzeugte Wasser aus dem Reaktionsmedium während des Erhitzens eliminiert wird, und ein fester Vorläufer in der Form eines spröden harten Harzes, gegebenenfalls isoliert, erhalten wird,
wobei dem festen Vorläufer in der Form eines spröden harten Harzes die Bildung eines Phenoldimers, gegebenenfalls isoliert, vorausgeht,
umfassend die folgenden Schritte:

a. Inberührungbringen mindestens einer Base, mit mindestens einem Phenol, substituiert in Position 4, mit der Formel (I),
mit wässrigem Formaldehyd, um ein Reaktionsmedium zu bilden, und Erhitzen des Reaktionsmediums während einer Zeit von 30 Minuten bis 2 Stunden, wobei gleichzeitig das gebildete Wasser eliminiert wird,
um ein Dimer mit der folgenden Formel (II) zu erhalten:

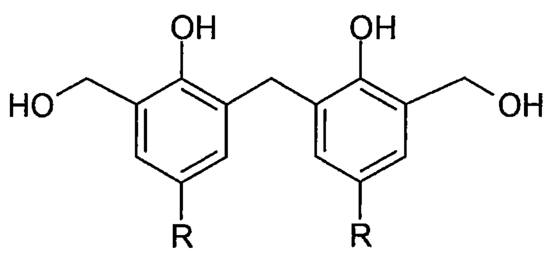

(II)

b. gegebenenfalls Weiterführen des Erhitzens des Reaktionsmediums, welches das Dimer enthält, wobei gleichzeitig das gebildete Wasser eliminiert wird, um ein Reaktionsmedium zu erhalten, das einen festen Vorläufer in der Form eines spröden harten Harzes enthält, während 1 bis 3 Stunden, und gegebenenfalls Filtrieren des festen Vorläufers in der Form eines spröden harten Harzes,

c. Erhitzen des Reaktionsmediums mit Hilfe von Wärmeübertragungsmitteln in der Form eines Ofens, oder Zusatz, zu dem Reaktionsmedium, das den festen Vorläufer in der Form eines spröden harten Harzes enthält, eines organischen Lösungsmittels, in dem der feste Vorläufer in der Form eines spröden harten Harzes unlöslich ist, um ein Reaktionsmedium zu erhalten, das einen Feststoff in der Form eines spröden harten Harzes enthält, in einem Ofen oder einem Lösungsmittel, und Erhitzen des Reaktionsmediums, das einen Feststoff in der Form eines spröden harten Harzes enthält, während 3 bis 8 Stunden,

um eine Mischung von Riesen Calixarenen zu erhalten, wobei R wie in Anspruch 1 definiert ist, umfassend außerdem ein p-(R) Calix[7]aren und/oder ein p-(R) Calix[8]aren,

d. Umkristallisieren, nach Neutralisieren der Base, der Mischung von Riesen Calixarenen in einem Lösungsmittel auf der Basis von DMSO, um eine gereinigte Mischung von Riesen Calixarenen zu erhalten, wobei R wie in Anspruch 1 definiert ist, im Wesentlichen ohne ein p-(R) Calix[7]aren und/oder ein p-(R) Calix[8]aren,

e. gegebenenfalls Reinigen durch GPC, wobei verschiedene Fraktionen als Funktion ihrer Elutionszeit getrennt werden.

**10.** Verfahren nach Anspruch 7, wobei das bei der Reaktion erzeugte Wasser in dem Reaktionsmedium während des Erhitzens gehalten wird, und ein fester Vorläufer, in gefällter Form, gegebenenfalls isoliert, erhalten wird.

**11.** Verfahren nach Anspruch 10, wobei der feste Vorläufer in gefällter Form von dem Reaktionsmedium isoliert wird, umfassend die folgenden Schritte:

a. Inberührungbringen mindestens einer Base, mit mindestens einem Phenol, substituiert in Position 4 mit der Formel (I),

mit wässrigem Formaldehyd, um ein Reaktionsmedium zu bilden, und Erhitzen des Reaktionsmediums während einer Zeit von 30 Minuten bis 1 Stunde, ohne Eliminieren des gebildeten Wassers, um ein Dimer mit der Formel (II) zu erhalten, wie in Anspruch 9 definiert,

b. gegebenenfalls gefolgt vom Erhitzen des Reaktionsmediums, welches das Dimer enthält, ohne Eliminieren des gebildeten Wassers, während 2 bis 3 Stunden, um ein Reaktionsmedium zu erhalten, das einen festen Vorläufer in gefällter Form enthält, und gegebenenfalls Filtrieren des festen Vorläufers in gefällter Form,

c. Erhitzen des Reaktionsmediums mit Hilfe von Wärmeübertragungsmitteln in der Form eines Ofens, oder Zusatz, zu dem Reaktionsmedium, das den festen Vorläufer in gefällter Form enthält, eines organischen Lösungsmittels, in dem der feste Vorläufer in gefällter Form unlöslich ist, um ein Reaktionsmedium zu erhalten, das einen Feststoff in gefällter Form enthält, in einem Ofen oder einem Lösungsmittel, und Erhitzen des Reaktionsmediums, das einen Feststoff in gefällter Form enthält, während 3 bis 8 Stunden, um eine Mischung von Riesen Calixarenen zu erhalten, wobei R wie in Anspruch 1 definiert ist, umfassend außerdem ein p-(R) Calix[7]aren und/oder ein p-(R) Calix[8]aren,

d. Umkristallisieren, nach Neutralisieren der Base, in einem Lösungsmittel auf der Basis von DMSO, um eine gereinigte Mischung von Riesen Calixarenen zu erhalten, wobei R wie in Anspruch 1 definiert ist, im Wesentlichen ohne ein p-(R) Calix[7]aren und/oder ein p-(R) Calix[8]aren,

e. gegebenenfalls Reinigen der gereinigten Mischung von Riesen Calixarenen ohne ein p-(R) Calix[7]aren und/oder ein p-(R) Calix[8]aren durch GPC, wobei verschiedene Fraktionen als Funktion ihrer Elutionszeit getrennt werden.

**12.** Verfahren nach einem der Ansprüche 7 bis 11, wobei die Base ausgewählt wird aus Bariumhydroxid, Rubidiumhydroxid, Lithiumhydroxid, Cesiumhydroxid, Kaliumchlorid oder Natriumhydroxid.

**13.** Verwendung einer Mischung von Riesen p-(R) Calixarenen, deren Größe mehr als 20 beträgt, wobei R wie in Anspruch 1 definiert ist, zur Bildung eines Materials oder im Rahmen der Verstärkung von Materialien.

**14.** Verwendung einer Mischung von Calixarenen nach Anspruch 6, die durch das in einem der Ansprüche 7 bis 12 definierte Verfahren erhalten werden kann, zur Bildung eines Materials oder im Rahmen der Verstärkung von Materialien.

## Claims

1. Use of at least one base in an aqueous solution, with at least one phenol substituted in position 4 of the following formula (I):

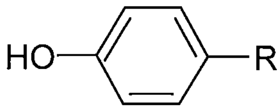

(I)

in which R is selected from:

- a benzyl group optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear $C_1$-$C_{20}$ alkyl group,
- a benzyloxy group optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear or branched $C_1$-$C_{20}$ alkyloxy group, a linear or branched PEG-1 to 10, the -OH end group of which is alkylated with a linear or branched $C_1$-$C_{20}$ alkyl,
- a benzyl thioether group $-S-CH_2-Ph$ optionally substituted in the ortho and/or para and/or meta position with one or more substituents selected from a halogen, SR' and OR', $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear or branched $C_1$-$C_{20}$ alkyl thioether group, of formula -S-($C_1$-$C_{20}$-alkyl),
- an $-NR_aR_b$ group, $R_a$ and $R_b$ representing, independently of one another, a linear or branched $C_1$-$C_{20}$ alkyl,
- a dibenzylamine group of formula $-N(benzyl)_2$, in which the two benzyl groups are, independently of one another, optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,

and a source of aqueous formaldehyde,
in the absence or in the presence of an organic solvent, thus constituting a reaction medium,
said base being at a total concentration comprised from 0.01 to 1 equivalent, with respect to said at least one phenol substituted in position 4 of formula (I),
said reaction medium being heated to a temperature comprised from 100 to 130°C, and said reaction medium optionally then being subjected to a heat treatment in the presence of heat transfer means,
for carrying out a reaction for the preparation of a mixture of giant *p*-(R)calixarenes the size of which is greater than 20.

2. Use according to claim 1, in which water is produced during the reaction, said water being removed from the reaction medium during said heating at a temperature comprised from 100 to 130°C and a solid precursor in the form of an optionally isolated, hard brittle resin is obtained.

3. Use according to one of claims 1 to 2, in which said solid precursor in the form of a hard brittle resin is preceded by the formation of an optionally isolated phenolic dimer.

4. Use according to claim 2 or 3, in which said solid precursor in the form of a hard brittle resin or said phenolic dimer, optionally isolated from the reaction medium, is placed in the presence of heat transfer means in the form of an oven or of a solvent in which said solid precursor in the form of a hard brittle resin and said phenolic dimer are insoluble, and is subjected to said heat treatment,
for the preparation of a mixture of giant p-(R)calixarenes the size of which is comprised from 21 to 50 phenolic units.

5. Use according to claim 1, in which water is produced during the reaction, said water being retained in the reaction medium during said heating and a solid precursor, in the form of a precipitate, optionally isolated, is obtained.

**6.** Mixture of calixarenes of the following formula (IV):

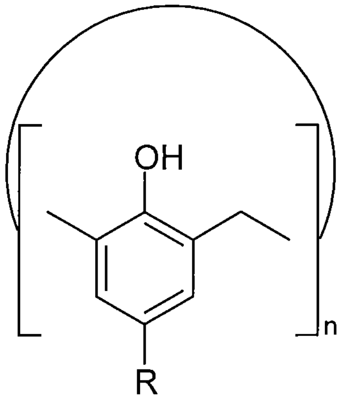

(IV)

in which n is an integer comprised from 21 to at most 220, R being as defined in claim 1.

**7.** Process for the preparation of a mixture of giant p-(R)calixarenes the size of which is greater than 20, comprising a stage of bringing at least one base, into contact with at least one phenol substituted in position 4 of the following formula (I):

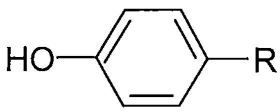

(I)

in which R is selected from:

- a benzyl group optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear $C_1$-$C_{20}$ alkyl group,
- a benzyloxy group optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear or branched $C_1$-$C_{20}$ alkyloxy group, a linear or branched PEG-1 to 10, the -OH end group of which is alkylated with a linear or branched $C_1$-$C_{20}$ alkyl,
- a benzyl thioether group $-S-CH_2-Ph$ optionally substituted in the ortho and/or para and/or meta position with one or more substituents selected from a halogen, SR' and OR', $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,
- a linear or branched $C_1$-$C_{20}$ alkyl thioether group, of formula -S-($C_1$-$C_{20}$-alkyl),
- an $-NR_aR_b$ group, $R_a$ and $R_b$ representing, independently of one another, a linear or branched $C_1$-$C_{20}$ alkyl,
- a dibenzylamine group of formula $-N(benzyl)_2$, in which the two benzyl groups are, independently of one another, optionally substituted in the ortho and/or para and/or meta position with one or more substituents selected from a halogen, SR' and OR', R' representing a linear or branched $C_1$-$C_{20}$ alkyl chain, a $C_3$ to $C_{20}$ cycloalkyl group, $NR_aR_b$, $PR_aR_b$, $P(O)R_aR_b$, $P(O)OR_aOR_b$, $R_a$ and $R_b$ representing, independently of one another, a linear or branched $C_1$-$C_{20}$ alkyl,

and a source of aqueous formaldehyde,
in the absence or in the presence of an organic solvent, thus constituting a reaction medium,
said base being at a total concentration comprised from 0.01 to 1 equivalent, with respect to said at least one phenol substituted in position 4 of formula (I),
said reaction medium being heated to a temperature comprised from 100 to 130°C, in order to obtain a heated

reaction medium,
and comprising, optionally, after said heating, a stage of heat treatment of said heated reaction medium, in the presence of heat transfer means.

**8.** Process according to claim 7, in which the water produced during the reaction is removed from said reaction medium during said heating and a solid precursor in the form of an optionally isolated, hard brittle resin is obtained.

**9.** Process according to claim 7, in which the water produced during the reaction is removed from said reaction medium during said heating and a solid precursor in the form of an optionally isolated, hard brittle resin is obtained,
in which said solid precursor in the form of a hard brittle resin is preceded by the formation of an optionally isolated phenolic dimer,
comprising the following stages:

a. bringing at least one base, into contact with at least one phenol substituted in position 4 of formula (I), with aqueous formaldehyde, in order to constitute a reaction medium and heating said reaction medium for a period of time comprised from 30 minutes to 2 hours, while removing the water formed,
in order to obtain a dimer of the following formula (II):

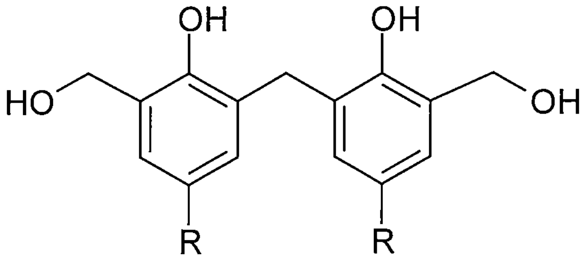

(II)

b. optionally continuing the heating of said reaction medium containing said dimer while removing the water formed in order to obtain a reaction medium containing a solid precursor, in the form of a hard brittle resin, for 1 to 3 hours, and optionally filtration of said solid precursor, in the form of a hard brittle resin,
c. heating said reaction medium using heat transfer means in the form of an oven, or addition to said reaction medium containing said solid precursor in the form of a hard brittle resin of an organic solvent in which said solid precursor in the form of a hard brittle resin is insoluble, in order to obtain a reaction medium containing a solid in the form of a hard brittle resin, in an oven or a solvent and heating said reaction medium containing a solid in the form of a hard brittle resin, for 3 to 8 hours,
in order to obtain a mixture of giant calixarenes in which R is as defined in claim 1, further comprising, a *p*-(R)calix[7]arene and/or ap-(R)calix[8]arene,
d. recrystallization, after neutralization of the base, of said mixture of giant calixarenes, from a solvent based on DMSO, in order to obtain a purified mixture of giant calixarenes in which R is as defined in claim 1, substantially devoid of *p*-(R)calix[7]arene and/or of *p*-(R)calix[8]arene.
e. optionally, purification using GPC, by separating different fractions depending on their elution time.

**10.** Process according to claim 7, in which the water produced during the reaction is retained in said reaction medium during said heating and a solid precursor in the form of an optionally isolated precipitate is obtained.

**11.** Process according to claim 10, in which said solid precursor in the form of a precipitate is isolated from the aforesaid reaction medium, comprising the following stages:

a. bringing at least one base, into contact with at least one phenol substituted in position 4 of formula (I), with aqueous formaldehyde, in order to constitute a reaction medium and heating said reaction medium for a period of time comprised from 30 minutes to 1 hour, without removal of the water formed, in order to obtain a dimer of formula (II) as defined in claim 9,
b. optionally continuing the heating of said reaction medium containing said dimer without removal of the water formed, for 2 to 3 hours, in order to obtain a reaction medium containing a solid precursor in the form of a precipitate, and optionally filtration of said solid precursor in the form of a precipitate,
c. heating said reaction medium using heat transfer means in the form of an oven, or the addition to said reaction medium containing said solid precursor in the form of a precipitate of an organic solvent in which said solid

precursor in the form of a precipitate is insoluble, in order to obtain a reaction medium containing a solid in the form of a precipitate in an oven or a solvent and heating said reaction medium containing a solid in the form of a precipitate, for 3 to 8 hours,
in order to obtain a mixture of giant calixarenes in which R is as defined in claim 1, further comprising, a *p*-(R)calix[7]arene and/or a *p*-(R)calix[8]arene,
d. recrystallization, after neutralization of the base, from a solvent based on DMSO, in order to obtain a purified mixture of giant calixarenes in which R is as defined in claim 1, substantially devoid ofp-(R)calix[7]arene and/or a *p*-(R)calix[8]arene,
e. optionally, purification of said purified mixture of giant calixarenes devoid of *p*-(R)calix[7]arene and/or of *p*-(R)calix[8]arene using GPC, by separating different fractions depending on their elution time.

**12.** Process according to anyone of claims 7 to 11, in which said base is chosen from barium hydroxide, rubidium hydroxide, lithium hydroxide, cesium hydroxide, potassium hydroxide or sodium hydroxide.

**13.** Use of a mixture of giant p-(R)calixarenes the size of which is greater than 20, R being as defined in claim 1, for the constitution of a material or in the context of reinforcement of materials.

**14.** Use of a mixture of calixarenes according to claim 6, capable of being obtained by the process defined in one of claims 7 to 12, for the constitution of a material or in the context of reinforcement of materials.

**FIGURE 1**

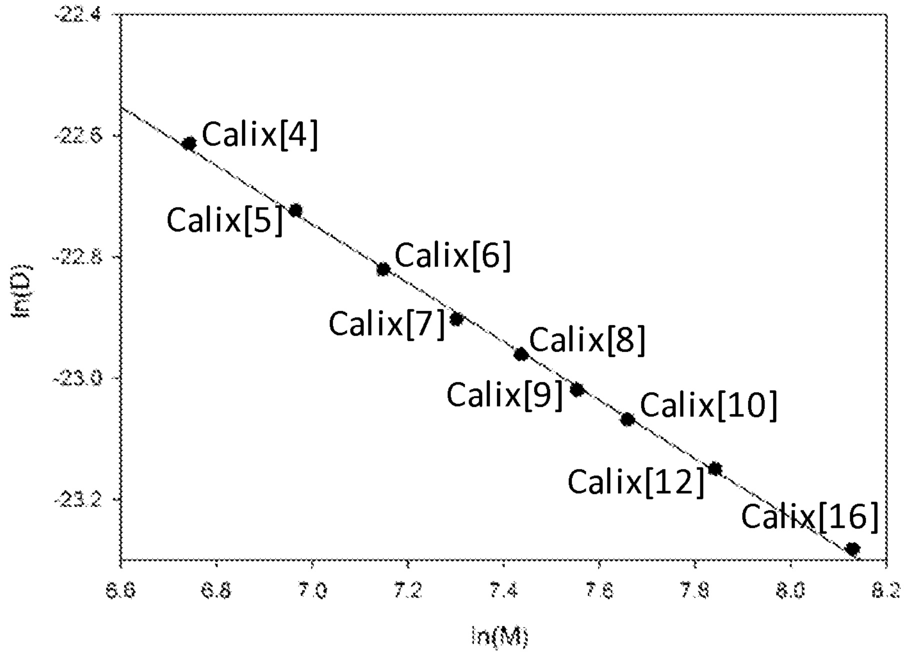


**FIGURE 2**

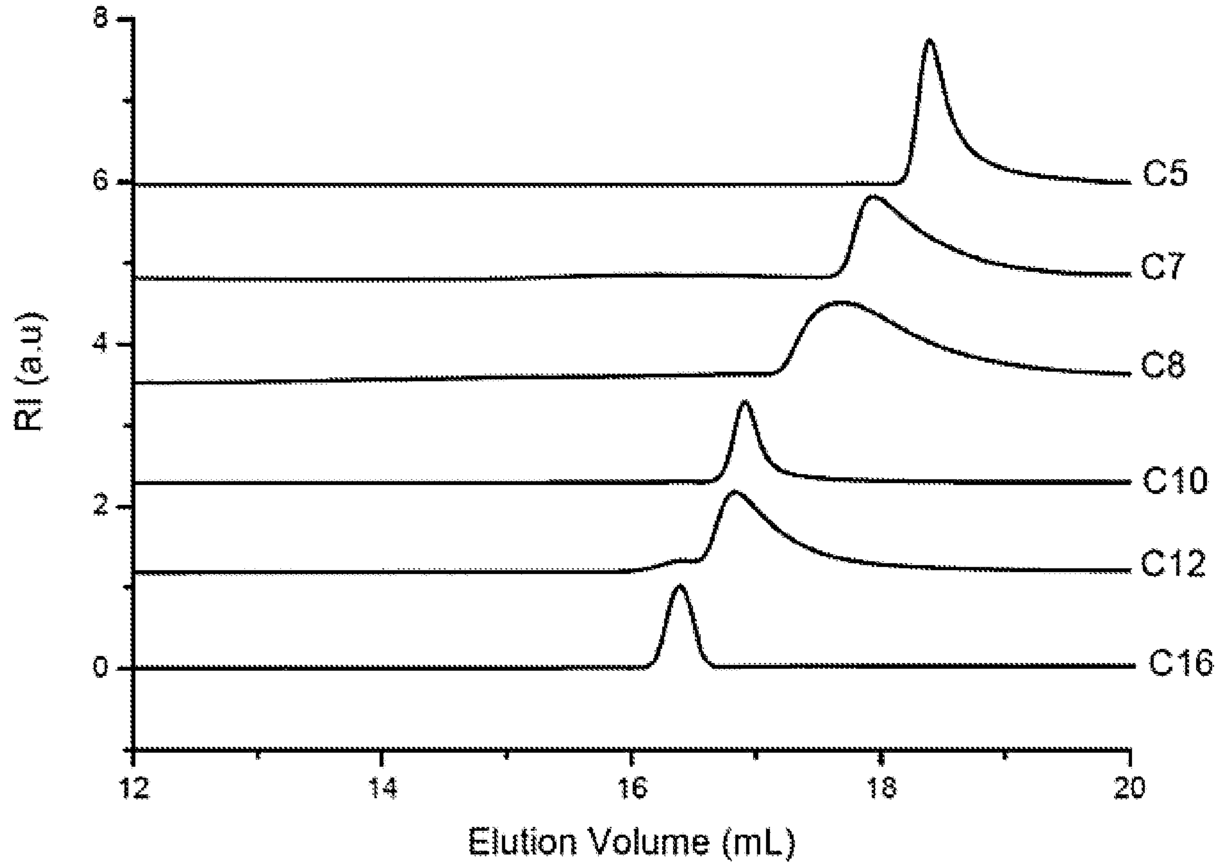

FIGURE 3

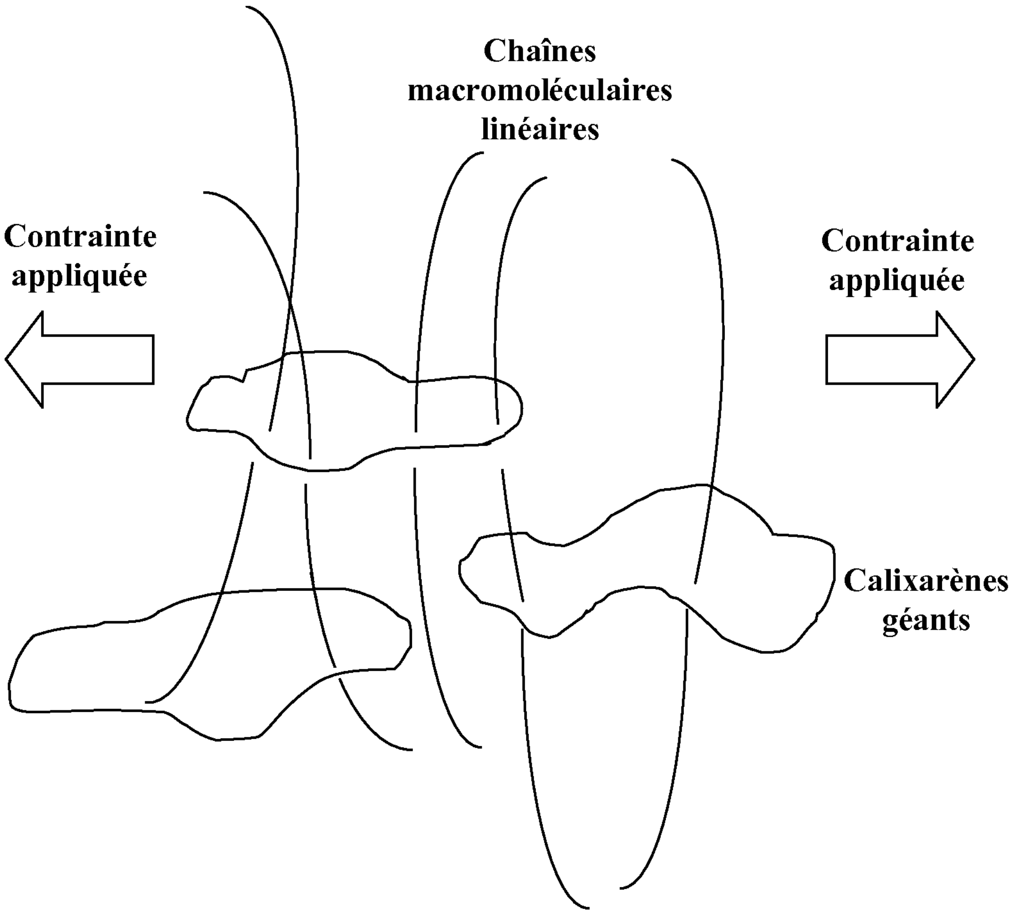
Chaînes macromoléculaires linéaires
Contrainte appliquée
Contrainte appliquée
Calixarènes géants

FIGURE 4

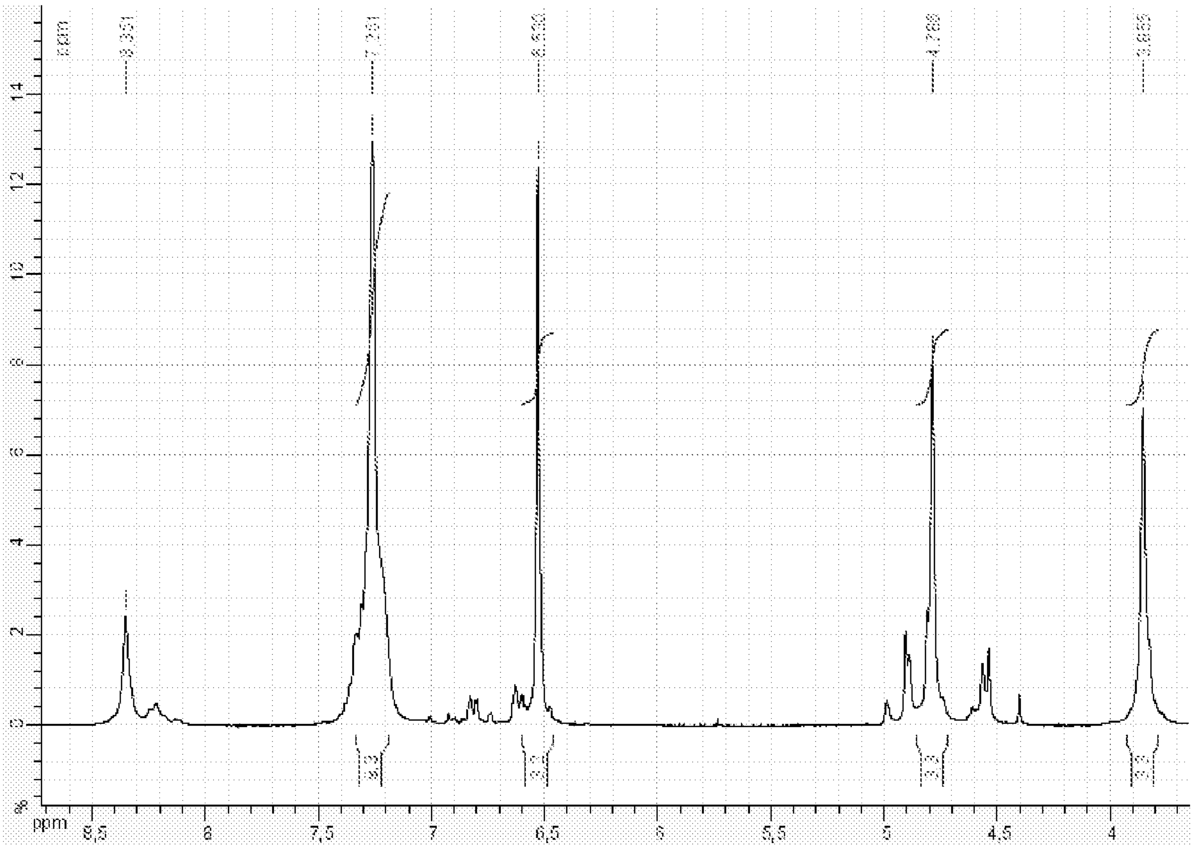

**FIGURE 5a**

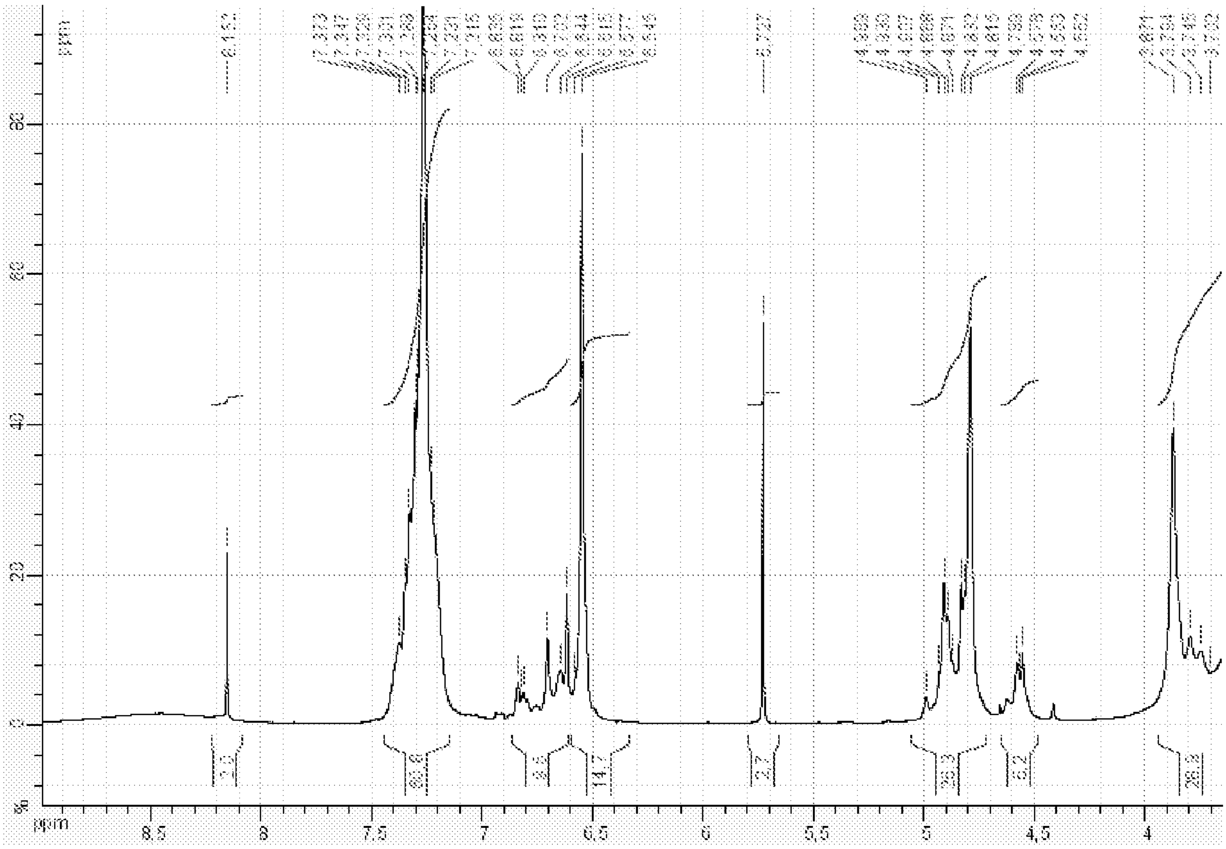

**FIGURE 5b**

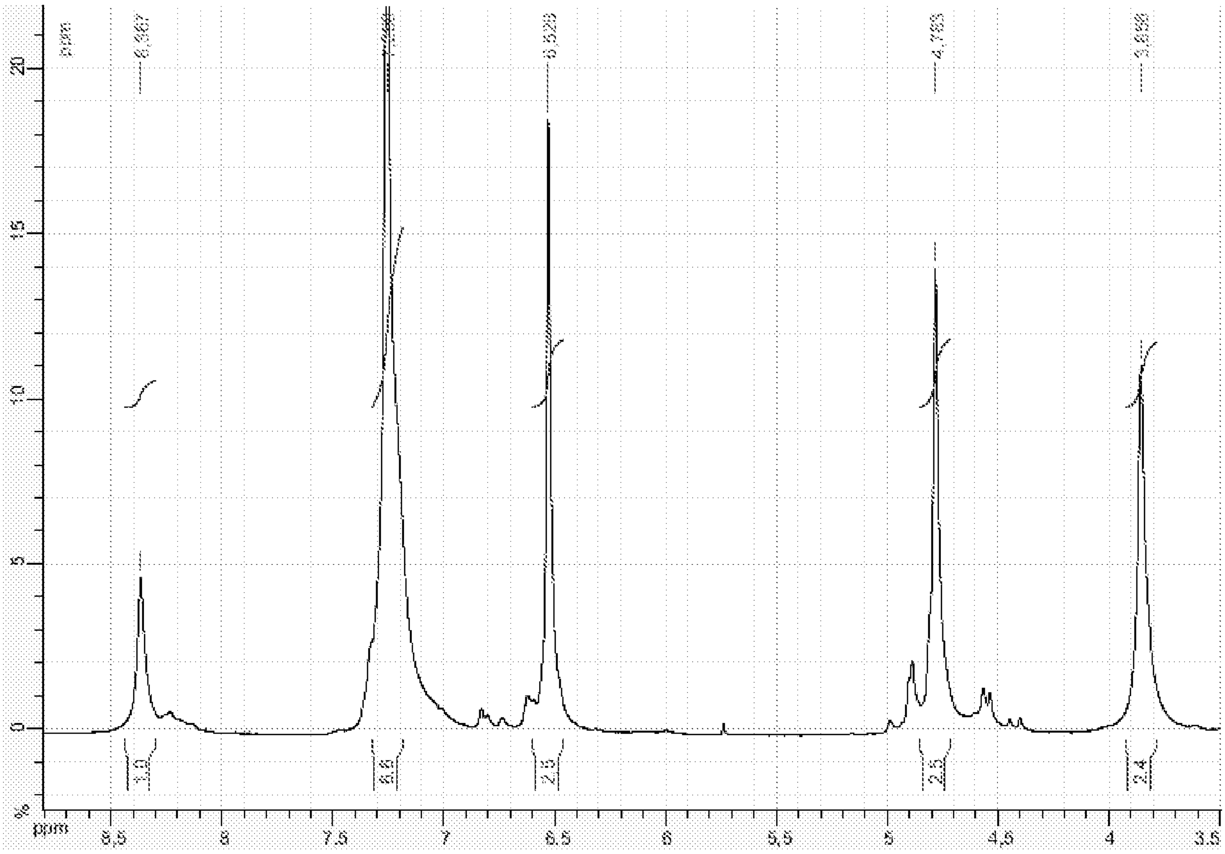
ppm
8.367
6.528
4.783
3.869
20
15
10
5
0
%
1.0
6.8
2.5
2.5
2.4
ppm
8.5
8
7.5
7
6.5
6
5.5
5
4.5
4
3.5

FIGURE 6

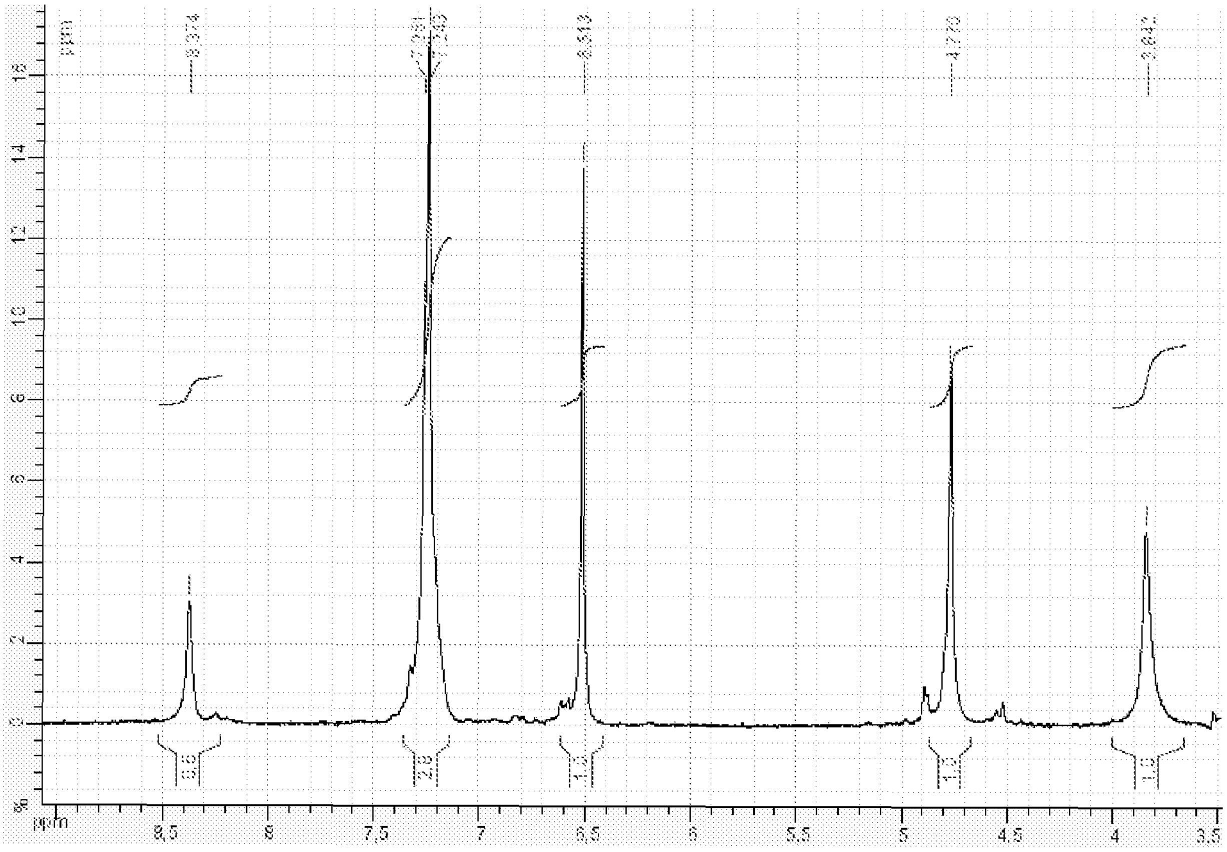

FIGURE 7

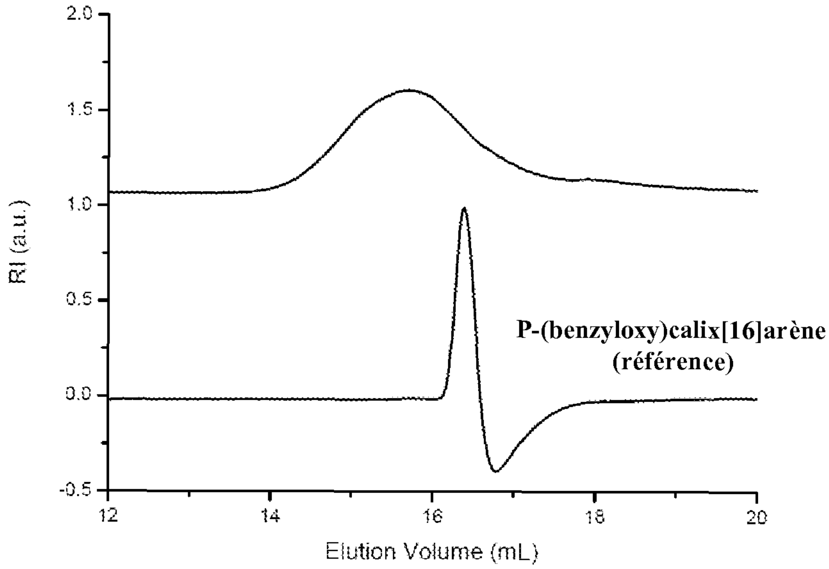

FIGURE 8

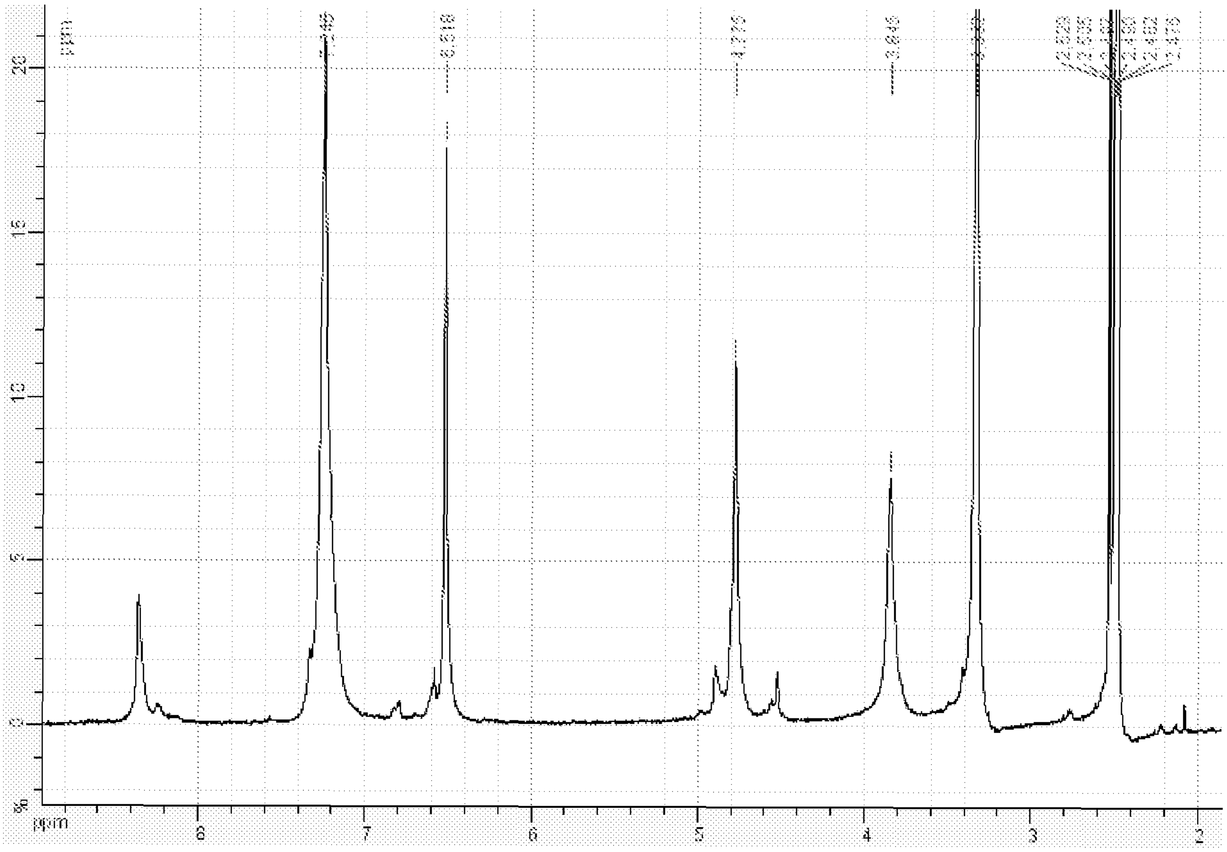


FIGURE 9

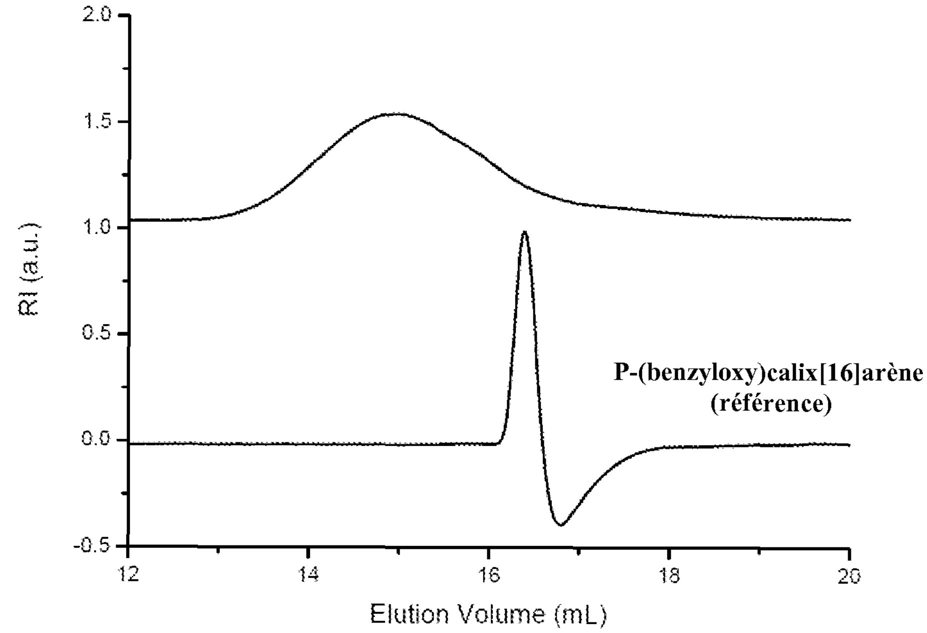

**FIGURE 10a**

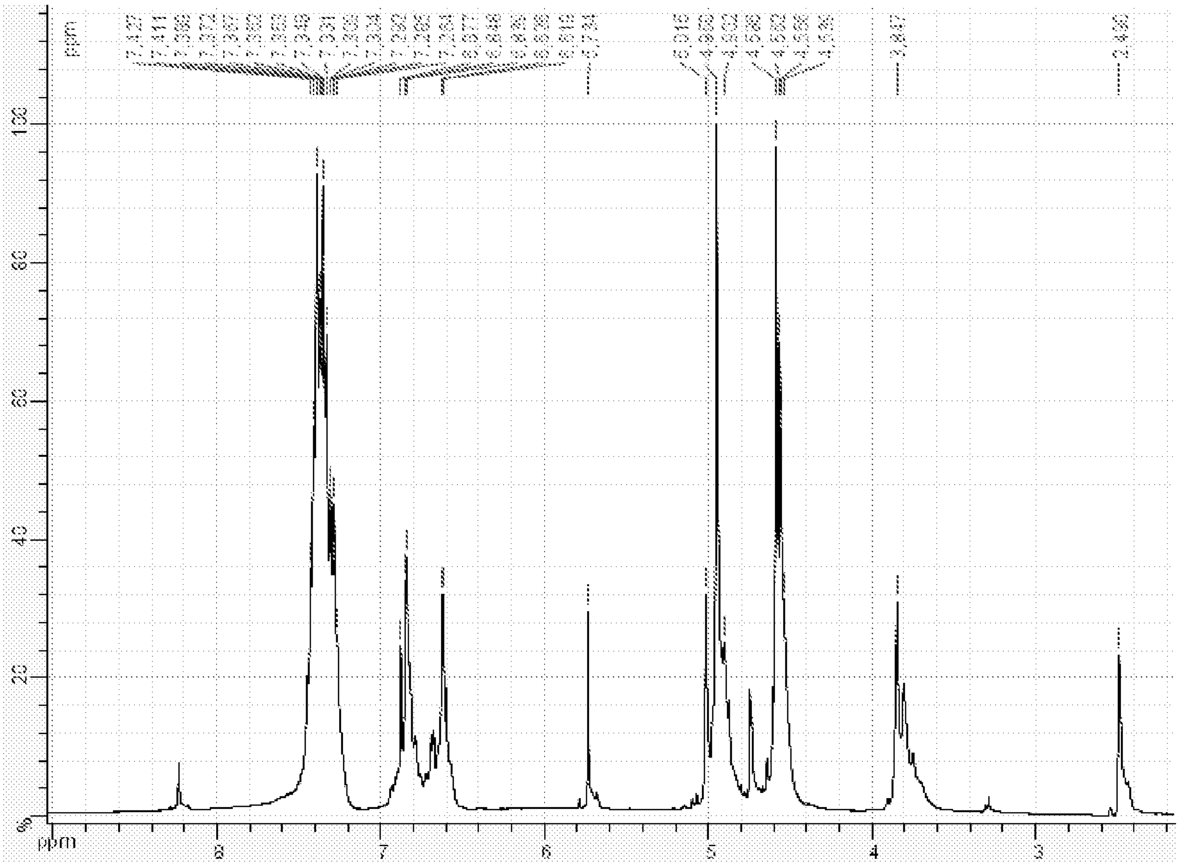

**FIGURE 10b**

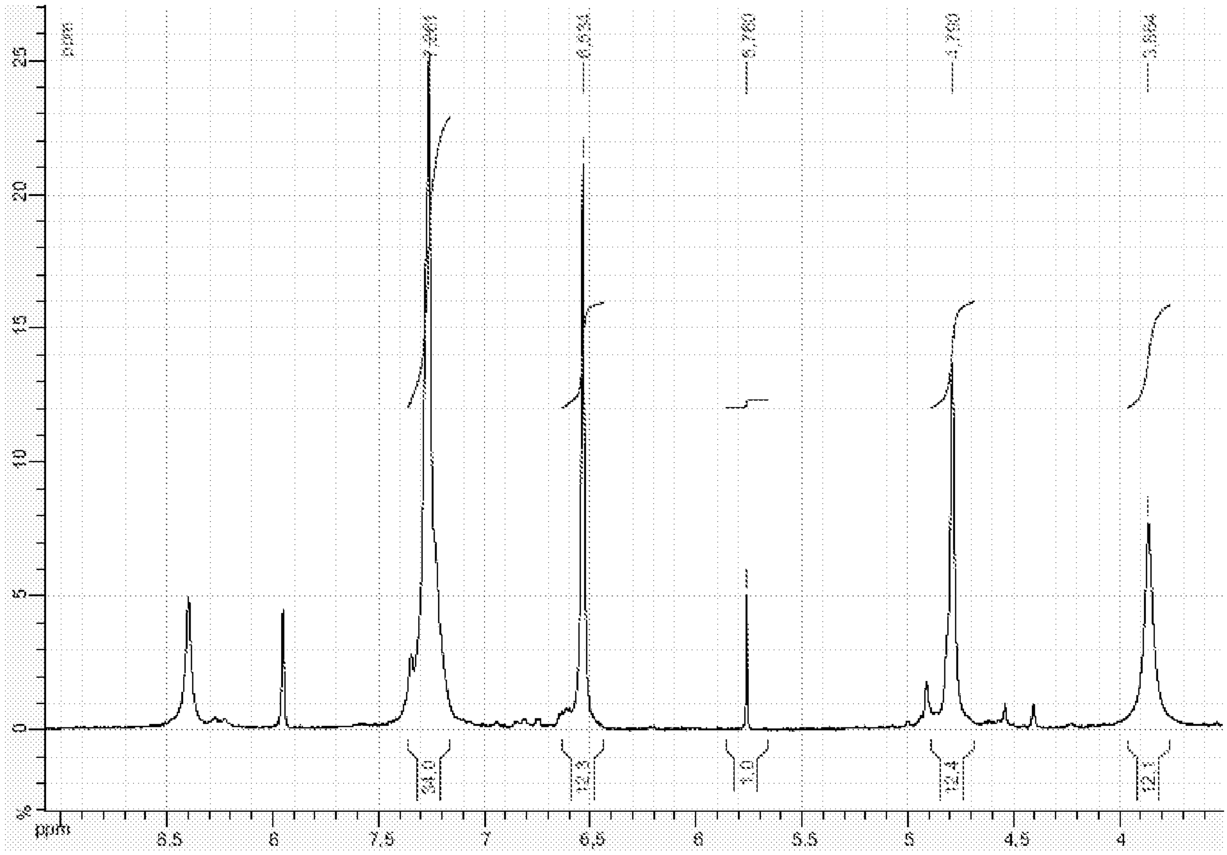

FIGURE 10c

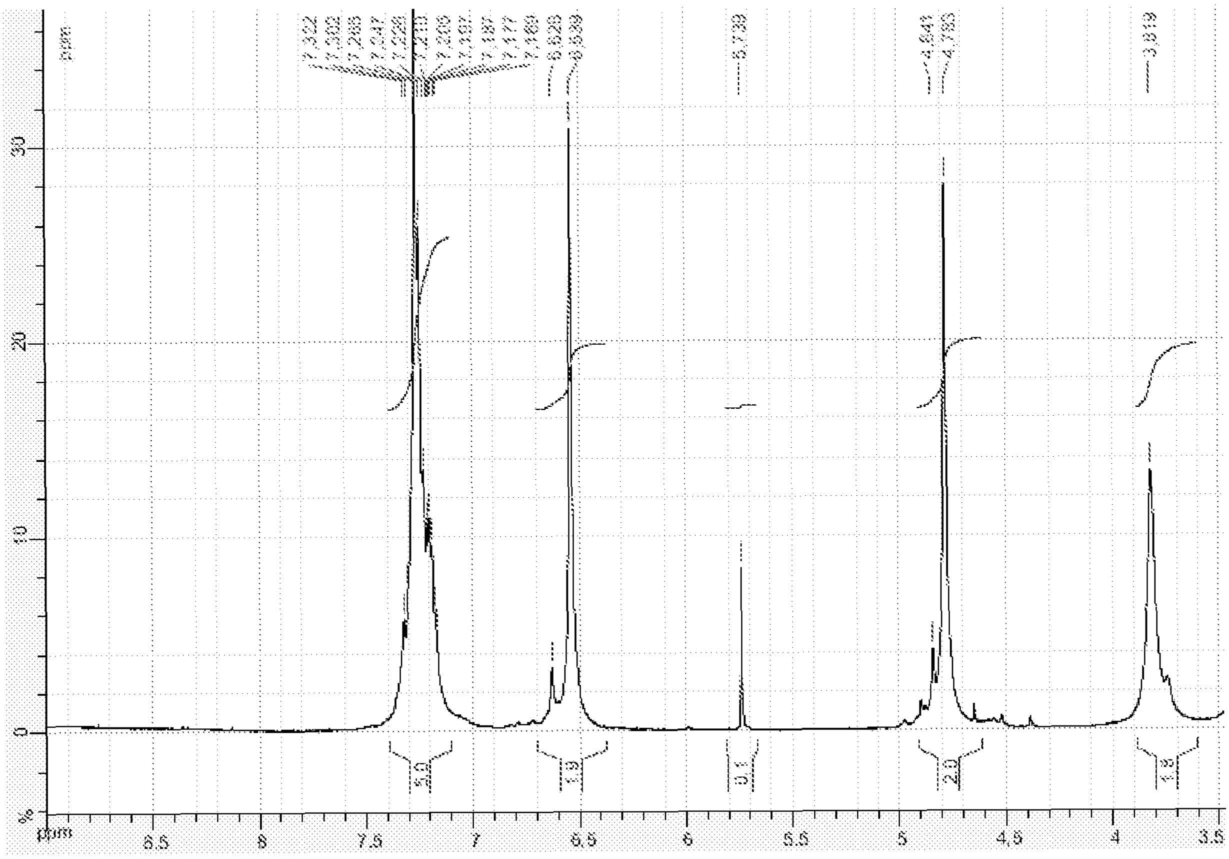
ppm
7.322
7.302
7.268
7.247
7.228
7.213
7.209
7.197
7.187
7.177
7.169
6.626
6.539
5.739
4.841
4.783
3.819
30
20
10
0
5.0
1.9
0.1
2.0
1.8
ppm
8.5
8
7.5
7
6.5
6
5.5
5
4.5
4
3.5

**FIGURE 11**

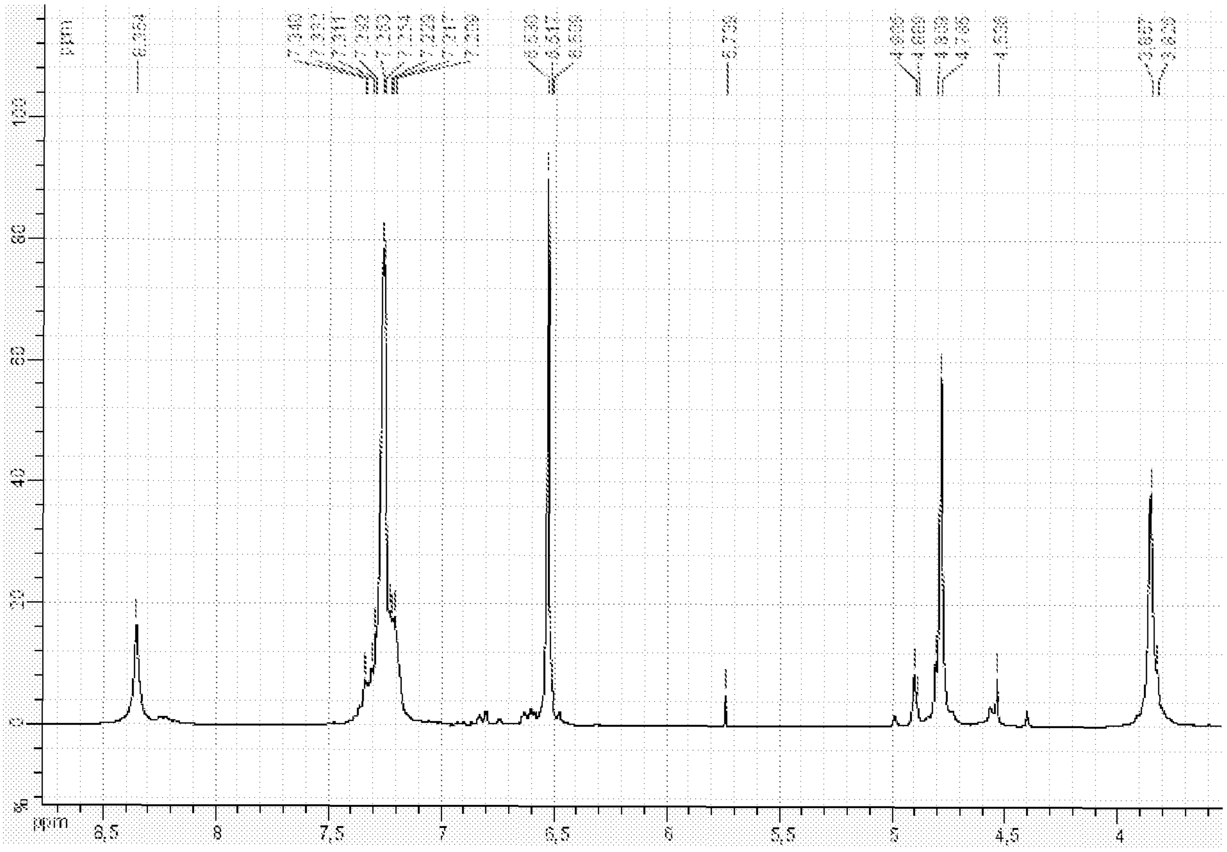


**FIGURE 12**

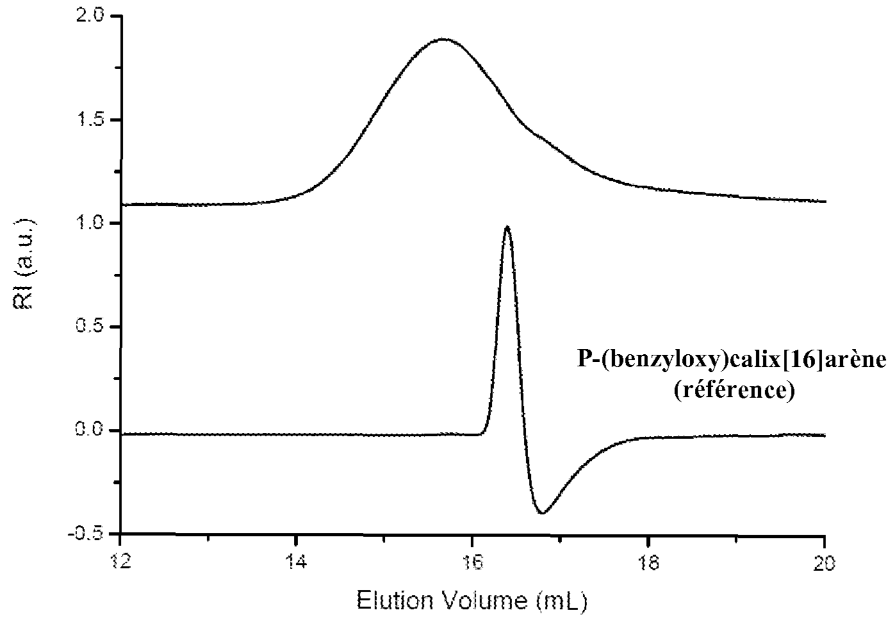

FIGURE 13

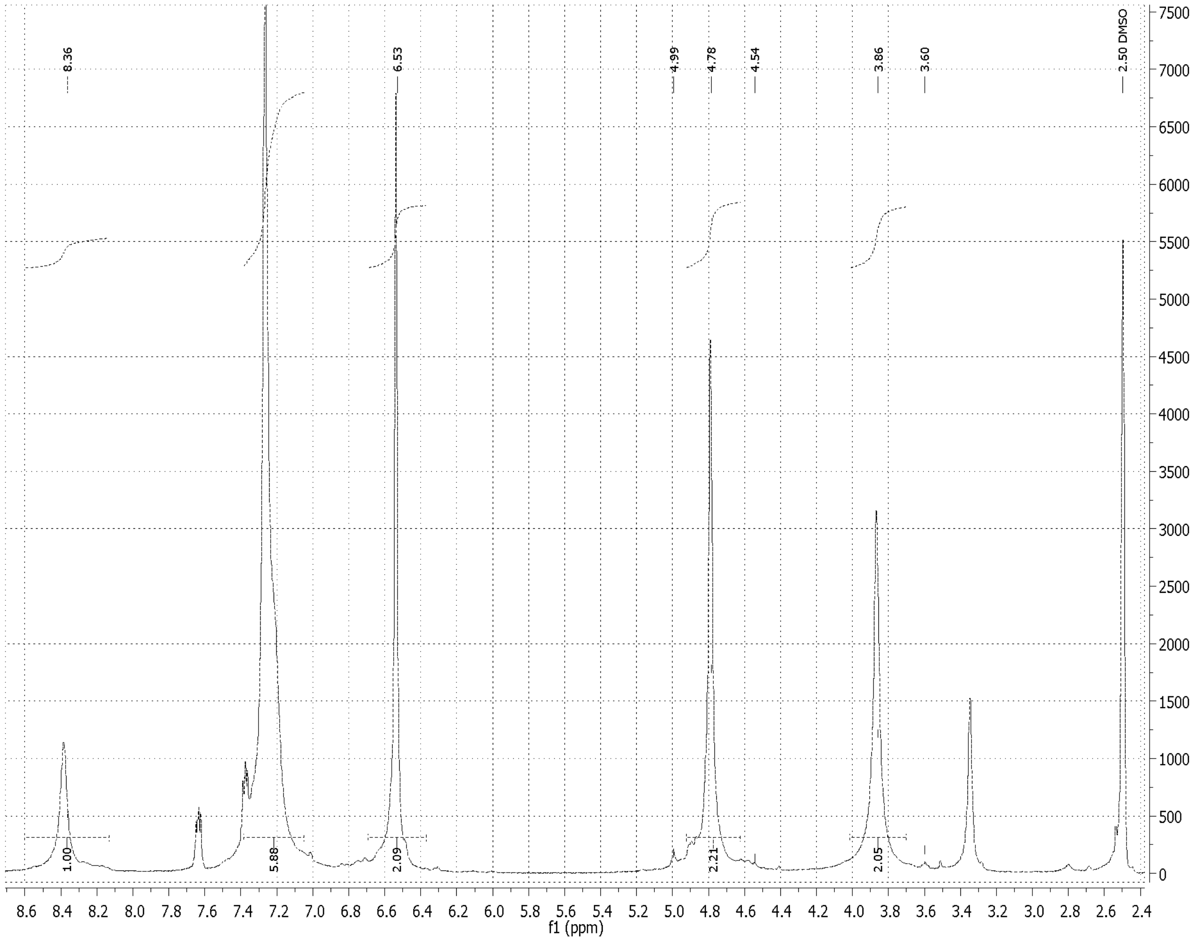


FIGURE 14

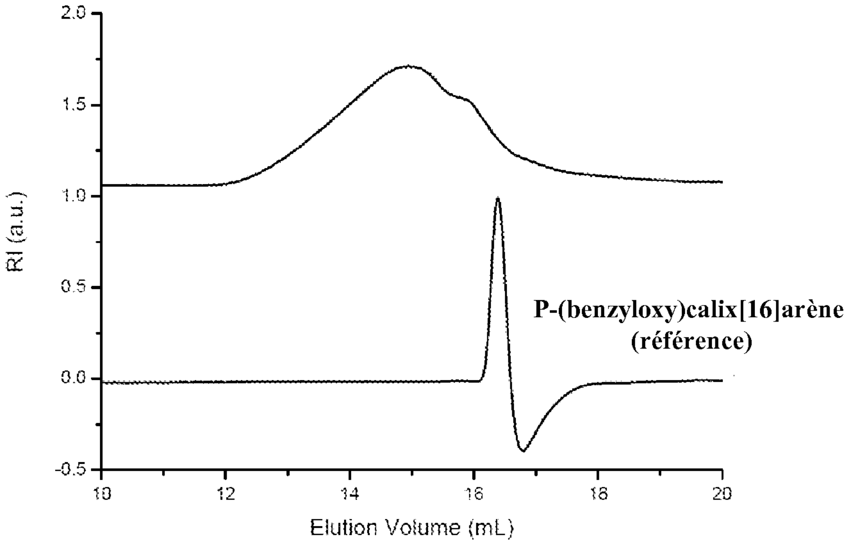

**FIGURE 15**

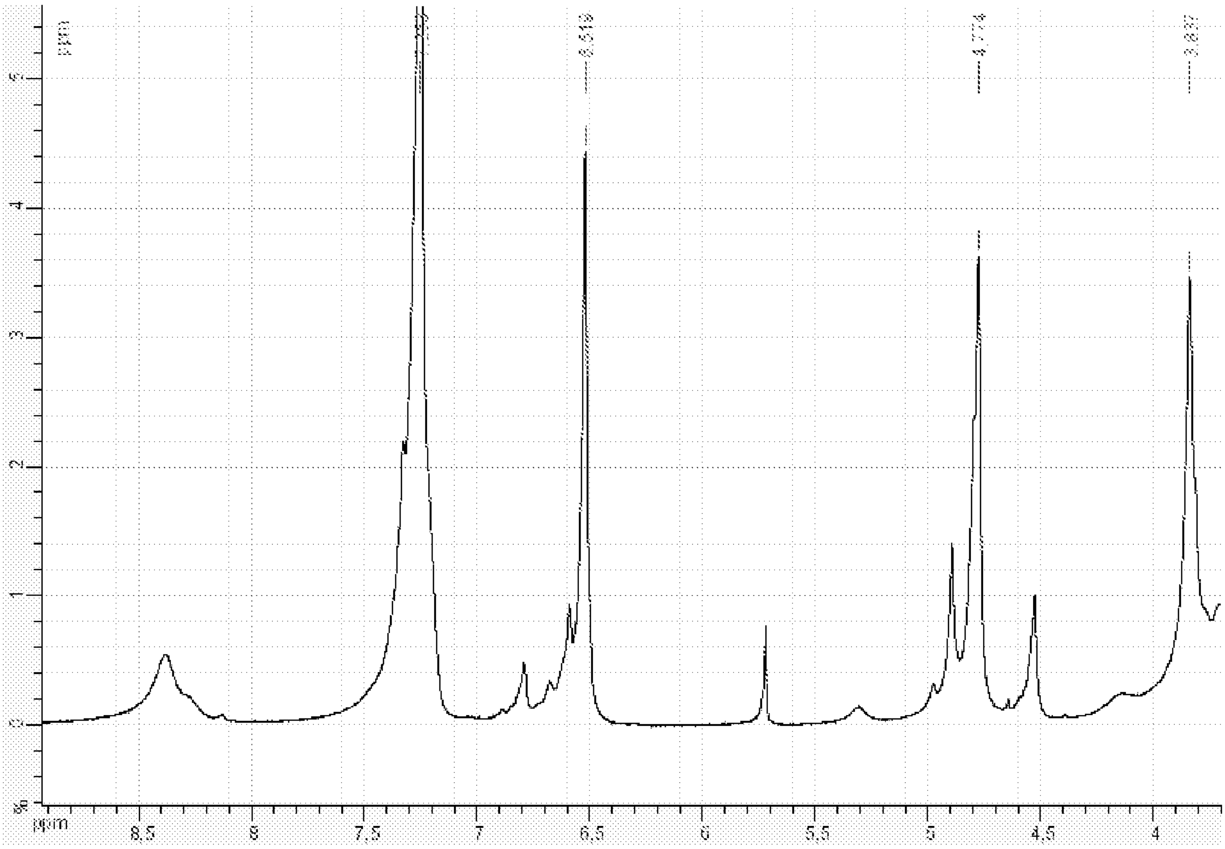

**FIGURE 16**

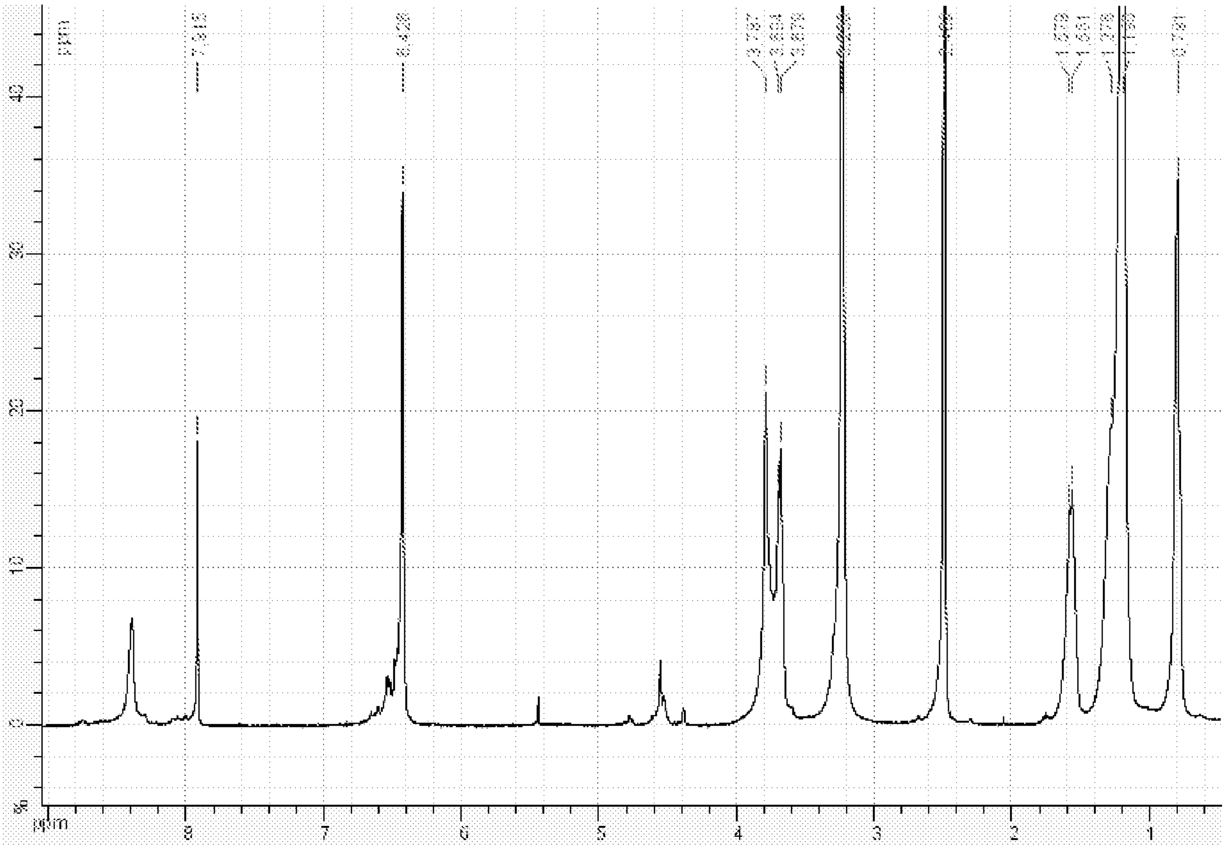

# FIGURE 17

## Figure 17.1

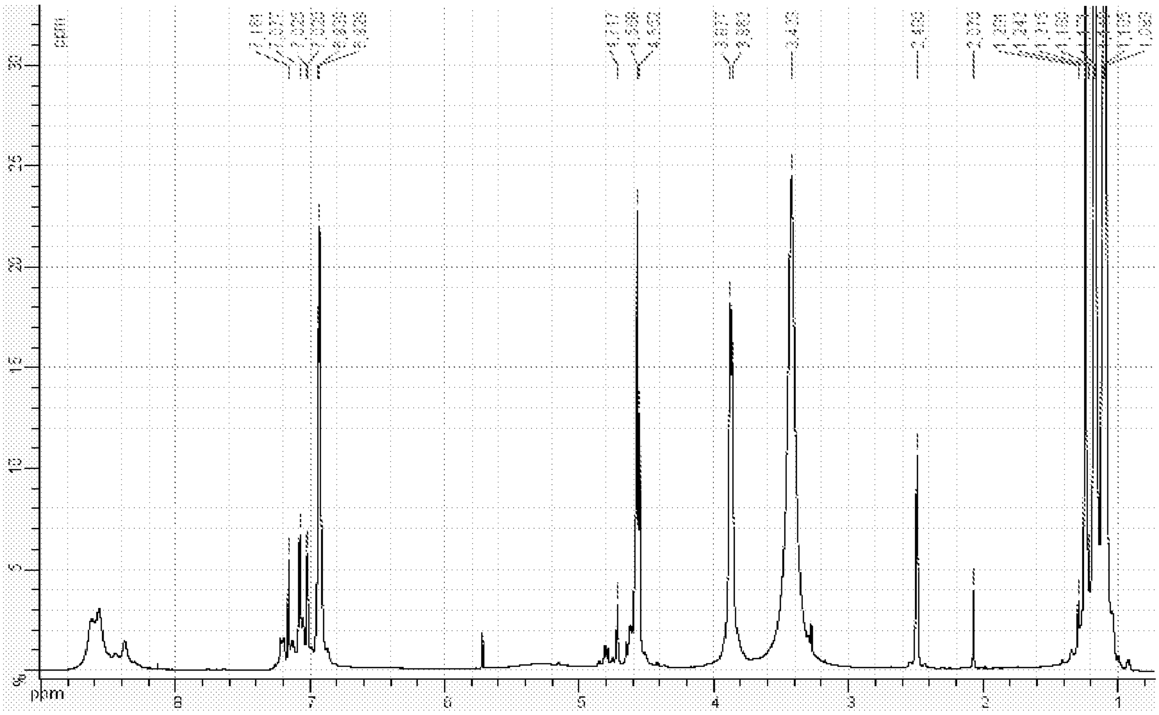

## Figure 17.2

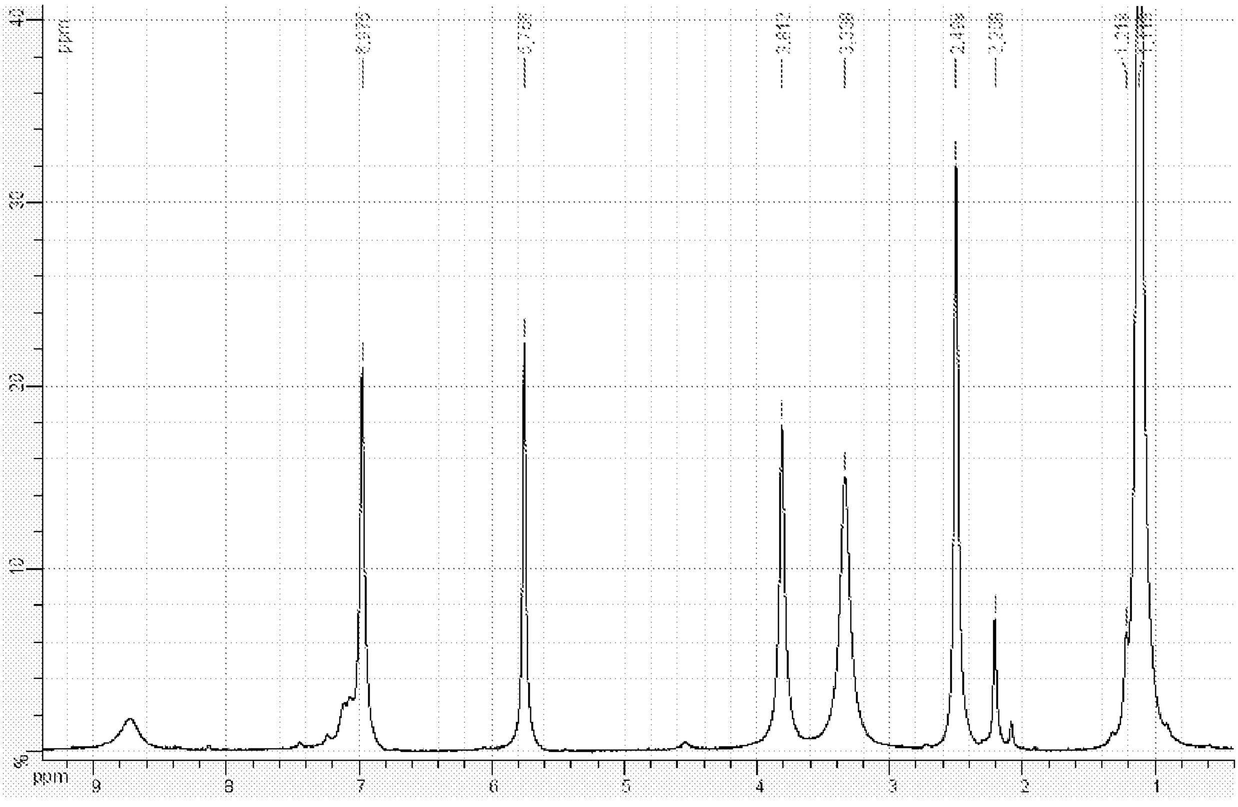

**FIGURE 18**

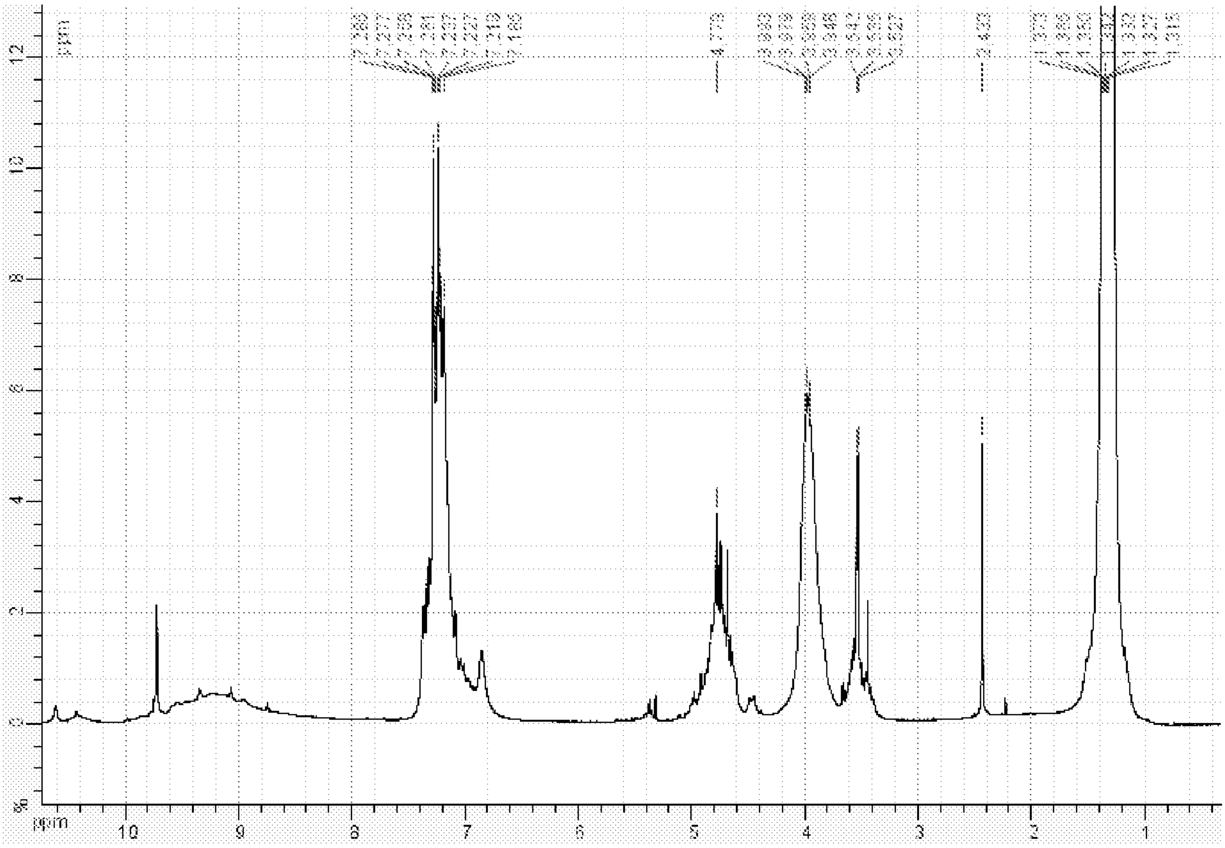

**FIGURE 19**

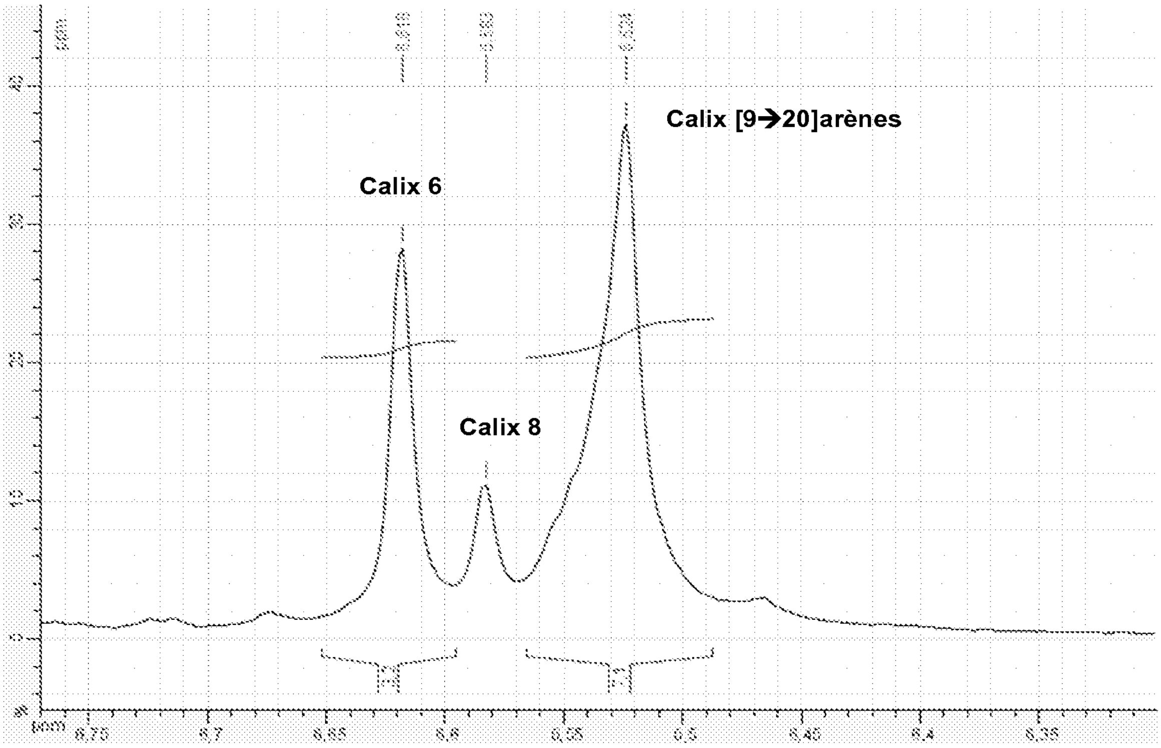

**FIGURE 20**

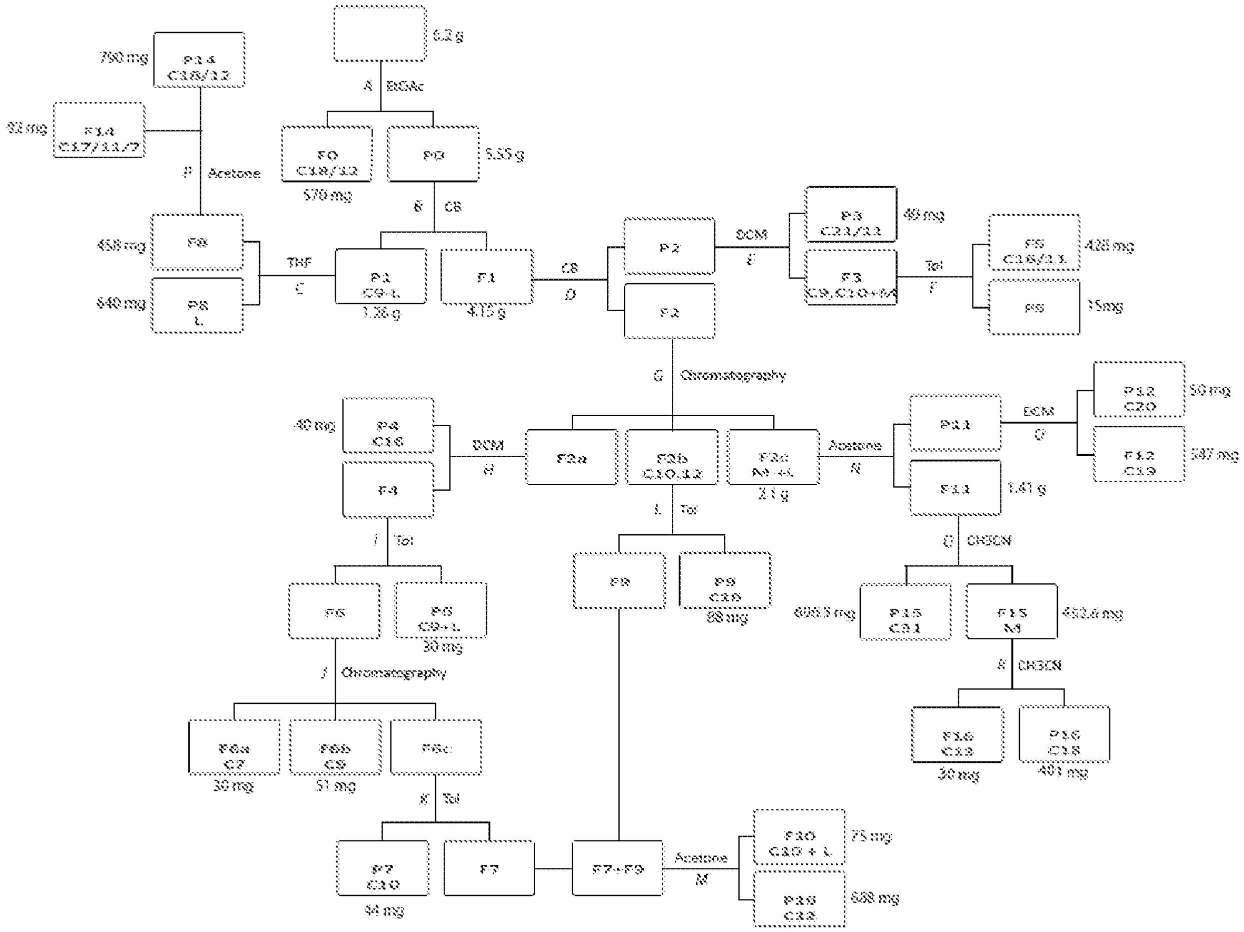

# FIGURE 21

## Figure 21.1 : Fraction F0

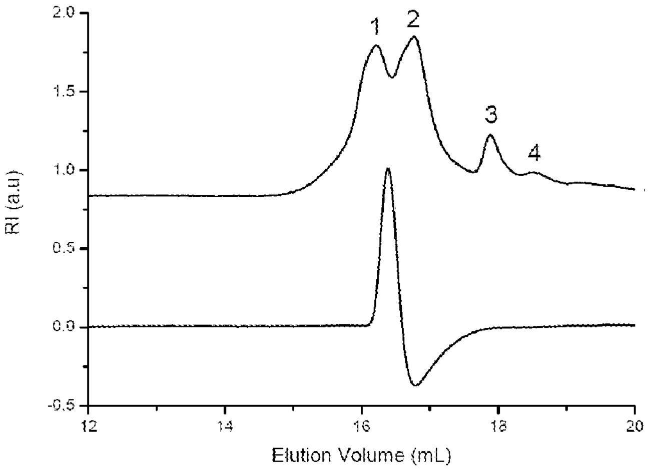


## Figure 21.2 : Fraction P3

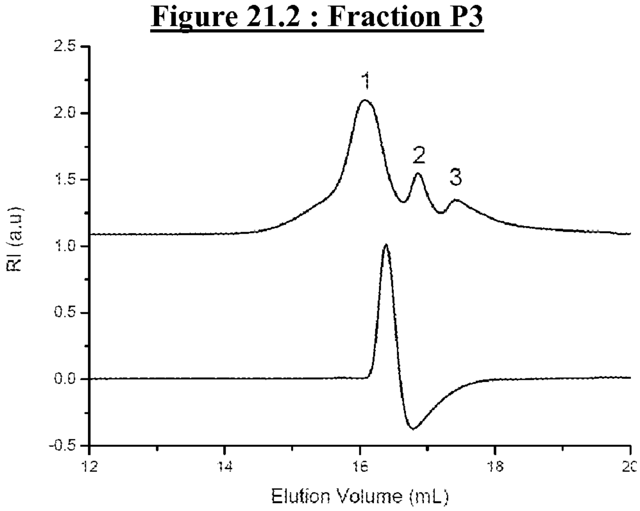


## Figure 21.3 : Fraction F5

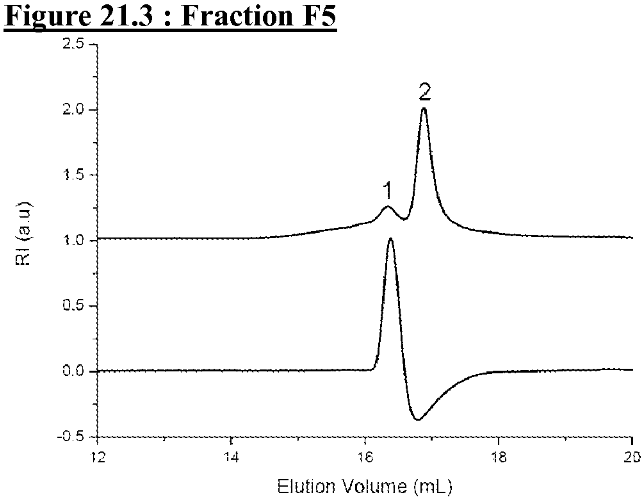

**Figure 21.4 : Fraction P12**

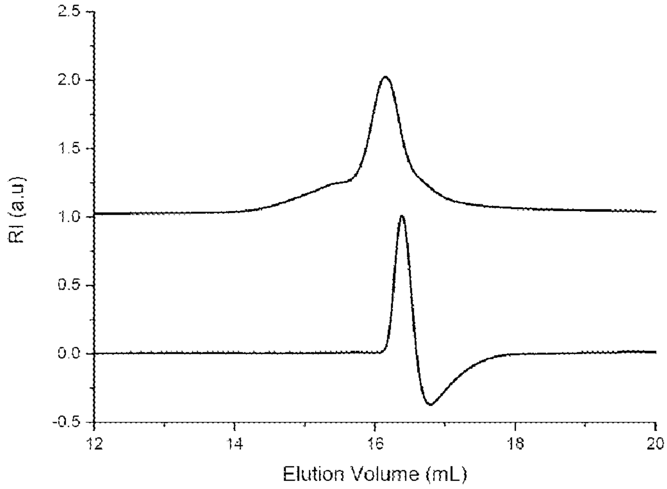


**Figure 21.5 : Fraction F12**

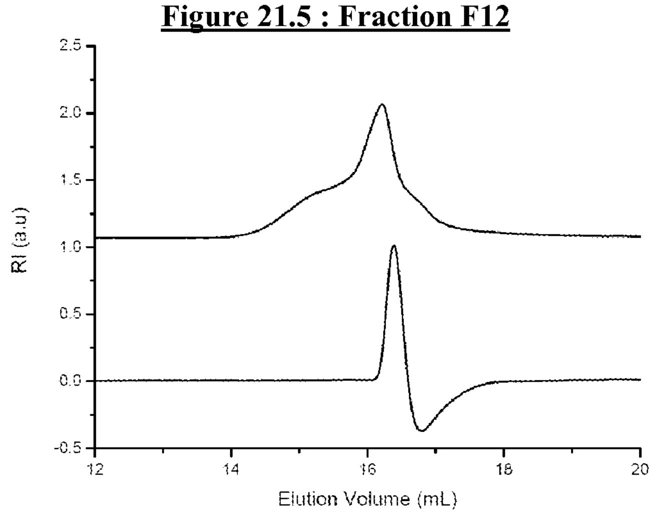


**Figure 21.6 : Fraction P14**

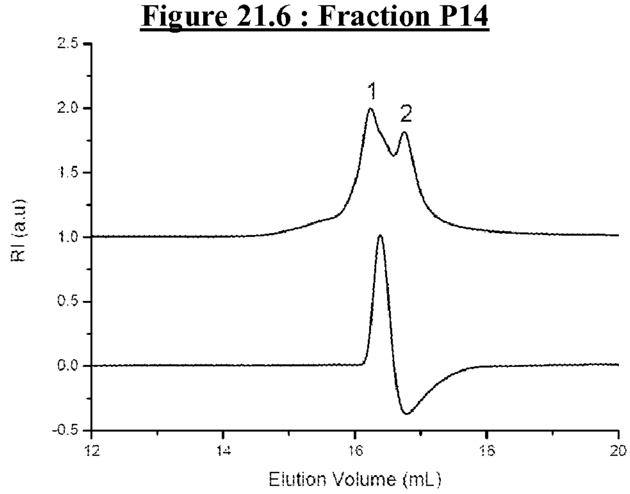

**Figure 21.7 : Fraction F14**

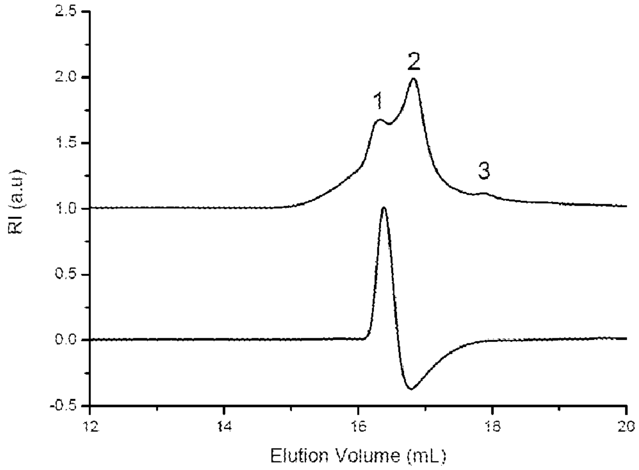


**Figure 21.8 : Fraction P15**

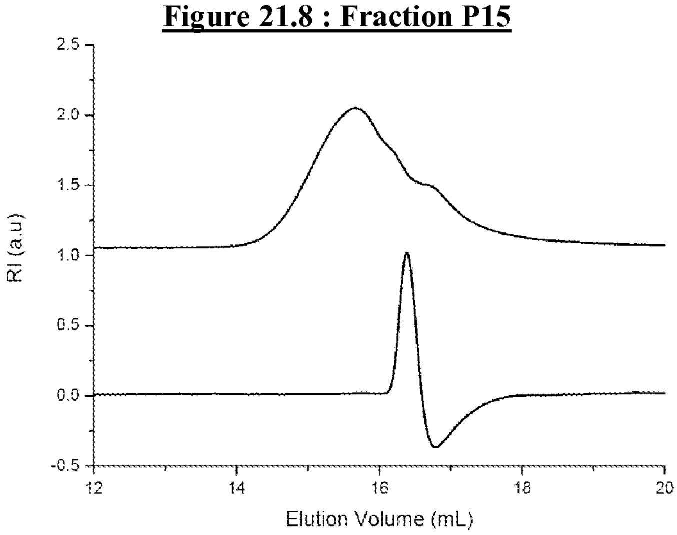


**Figure 21.9 : Fraction P16**

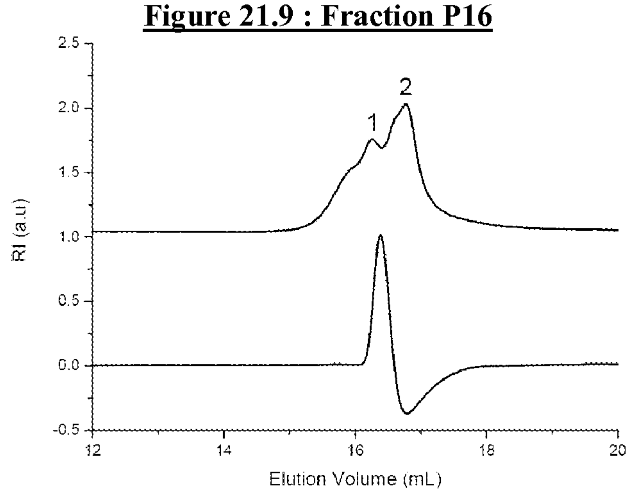

# FIGURE 22

## Figure 22.1 : Fraction F6b (Calix[9]arene)

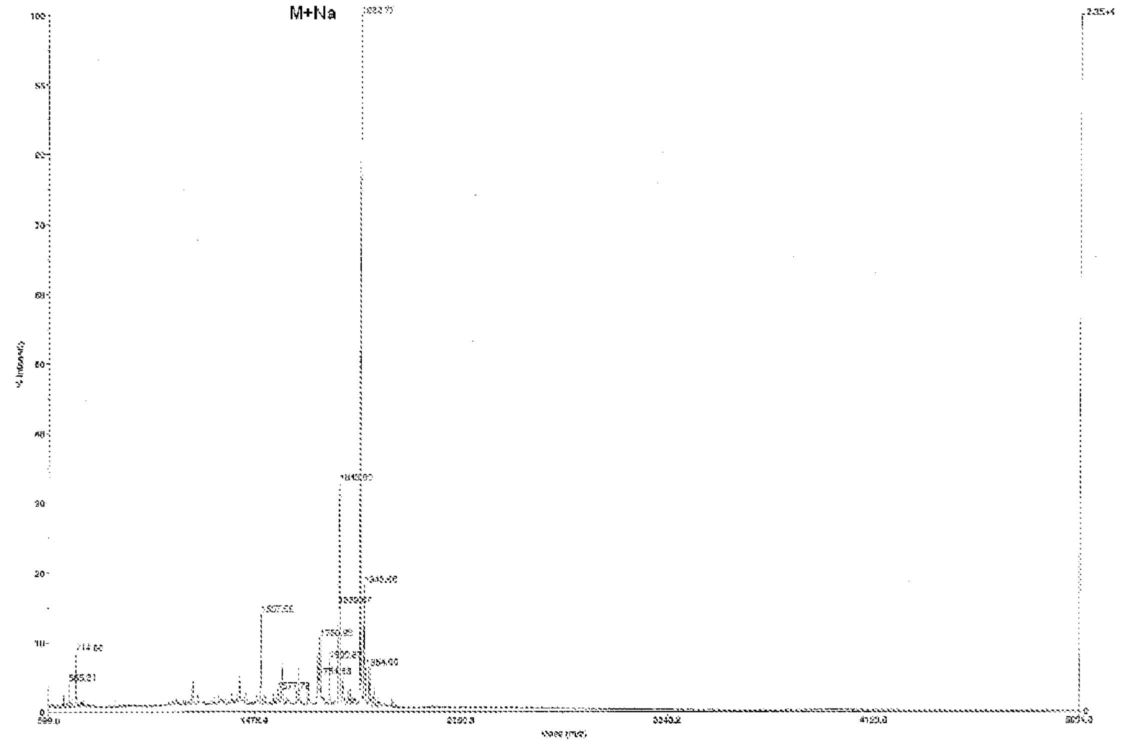


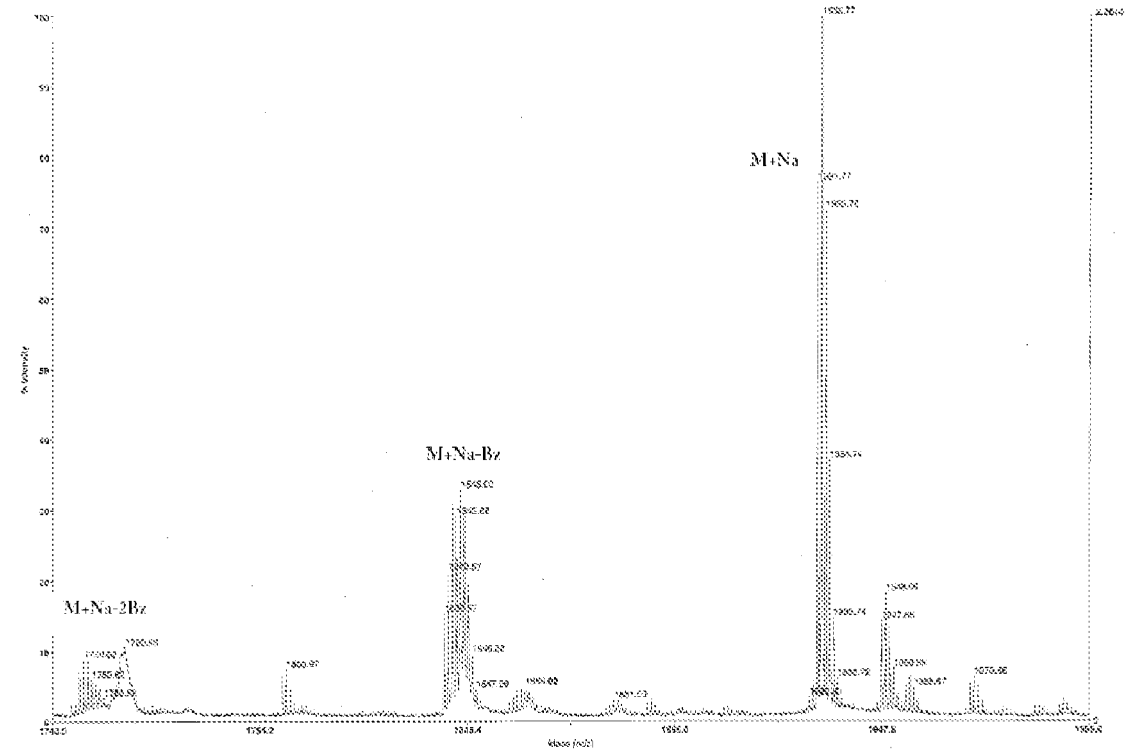

**Figure 22.2 : Fraction P7 (Calix[10]arene)**

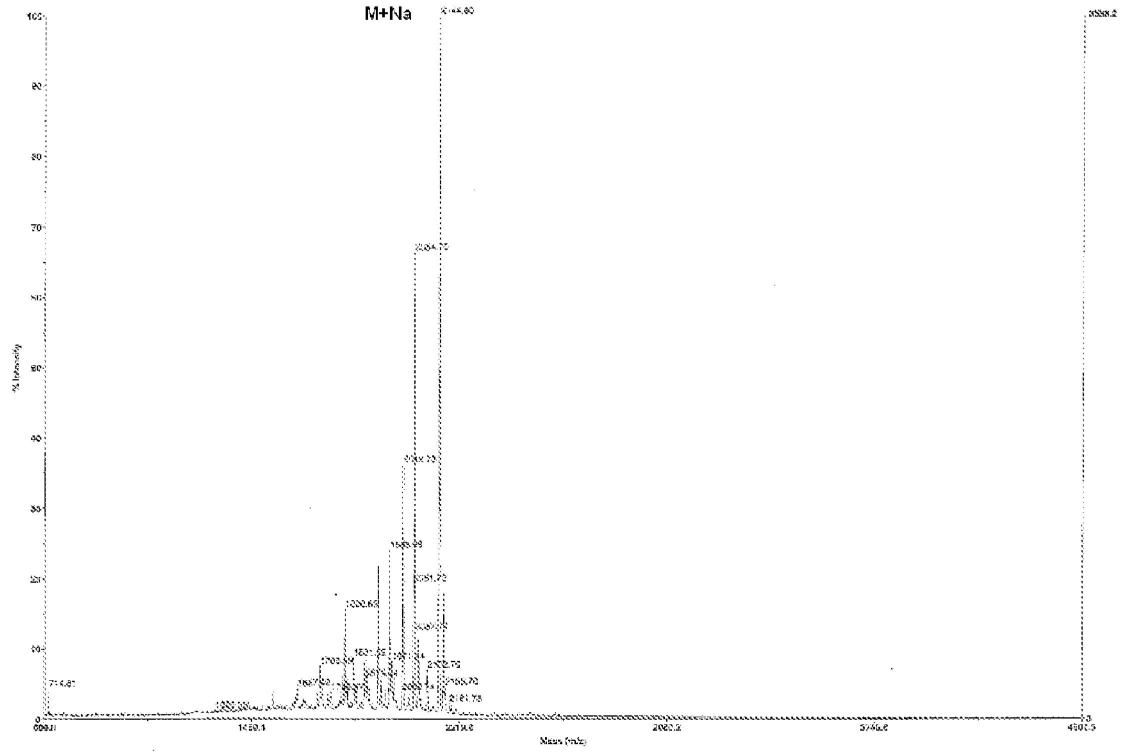


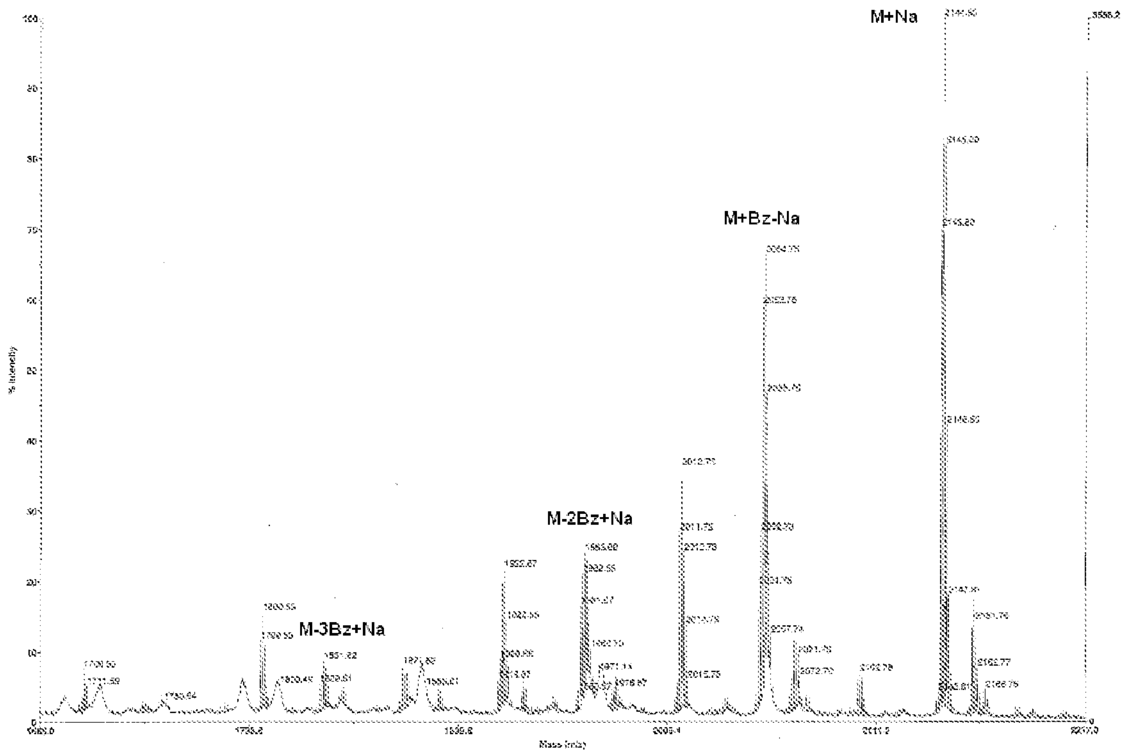

**Figure 22.3 : Fraction P10 (Calix[12]arene)**

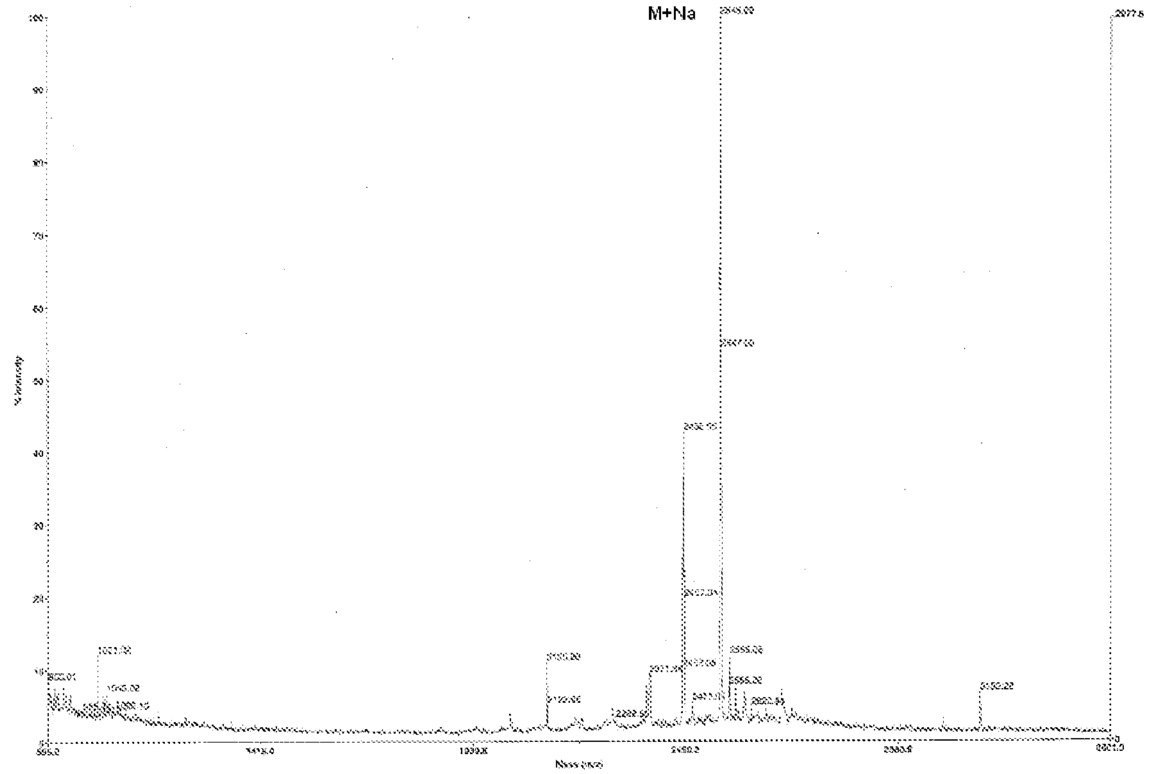


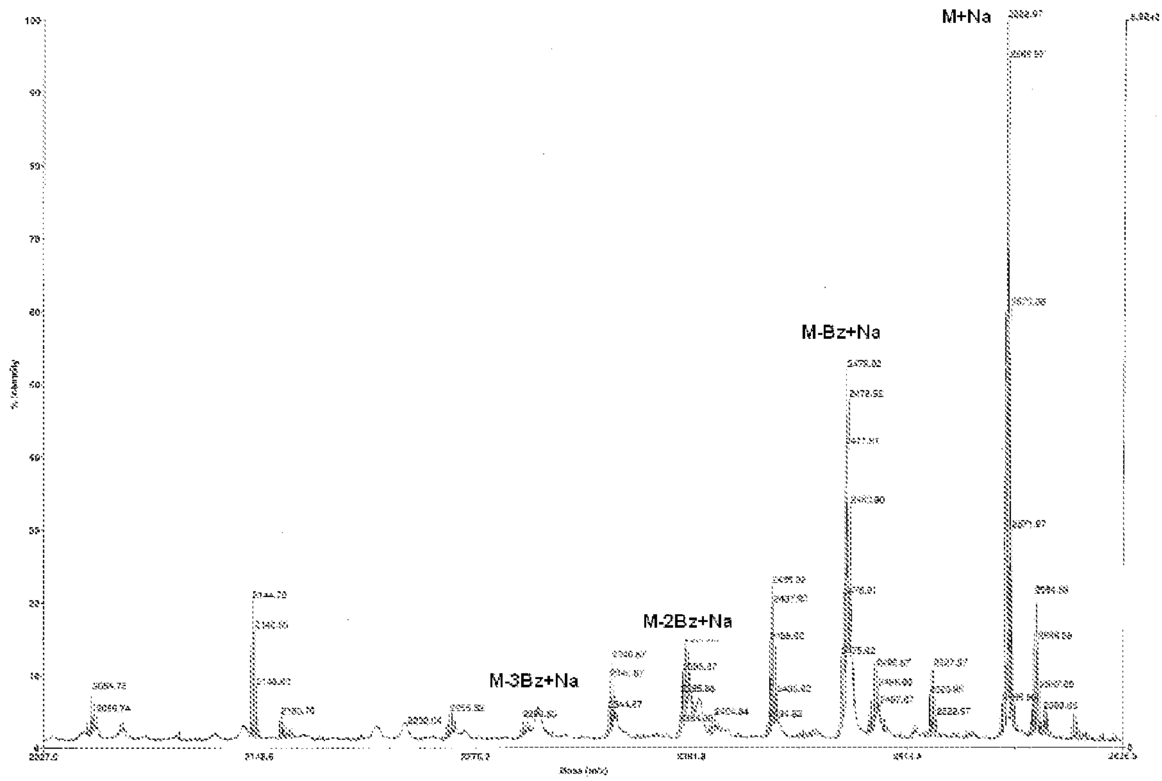

**Figure 22.4 : Fraction P4 (calix[16]arene)**

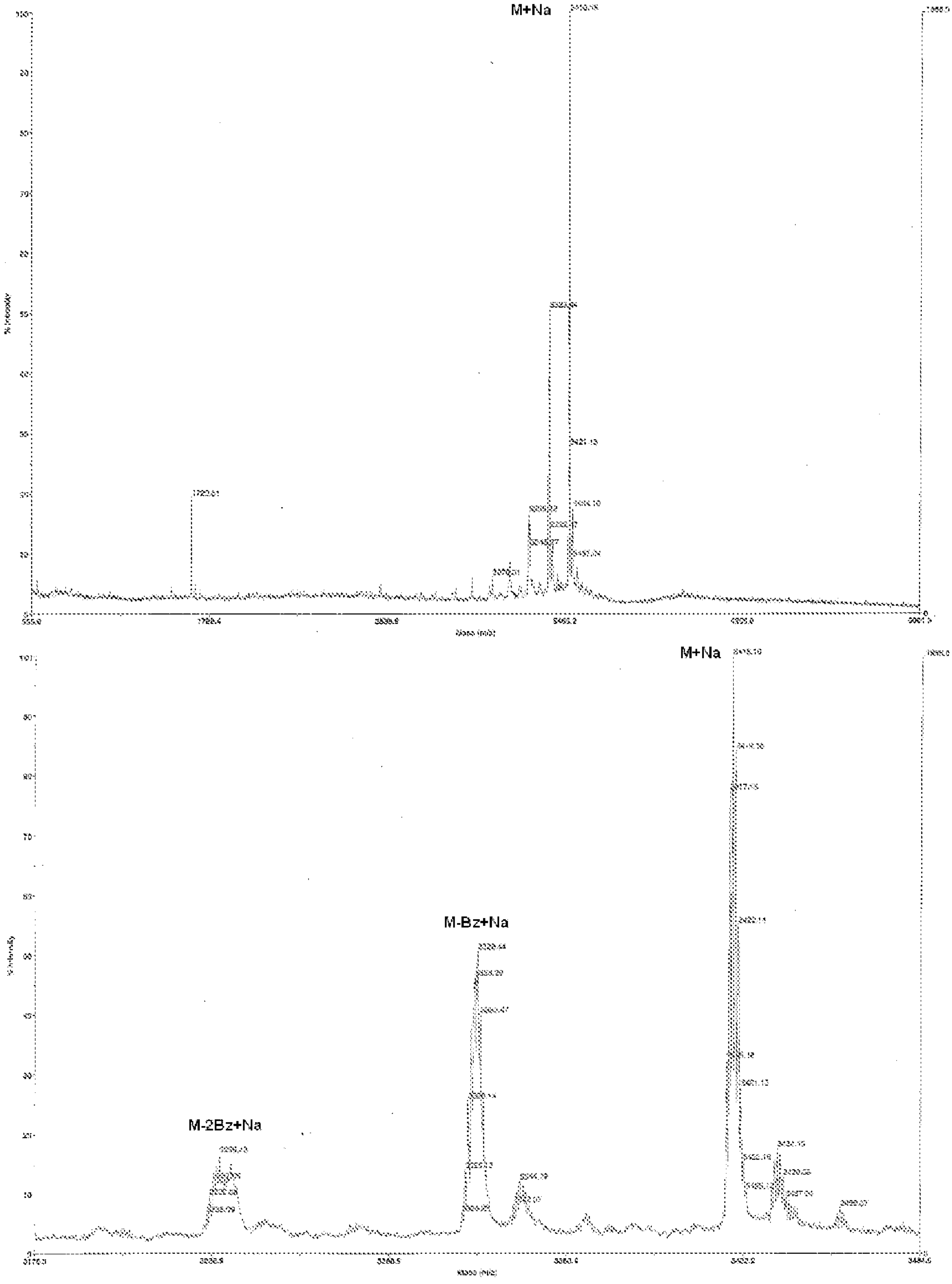

# FIGURE 23

## Figure 23.1 : Fraction F6b (Calix[9]arene)

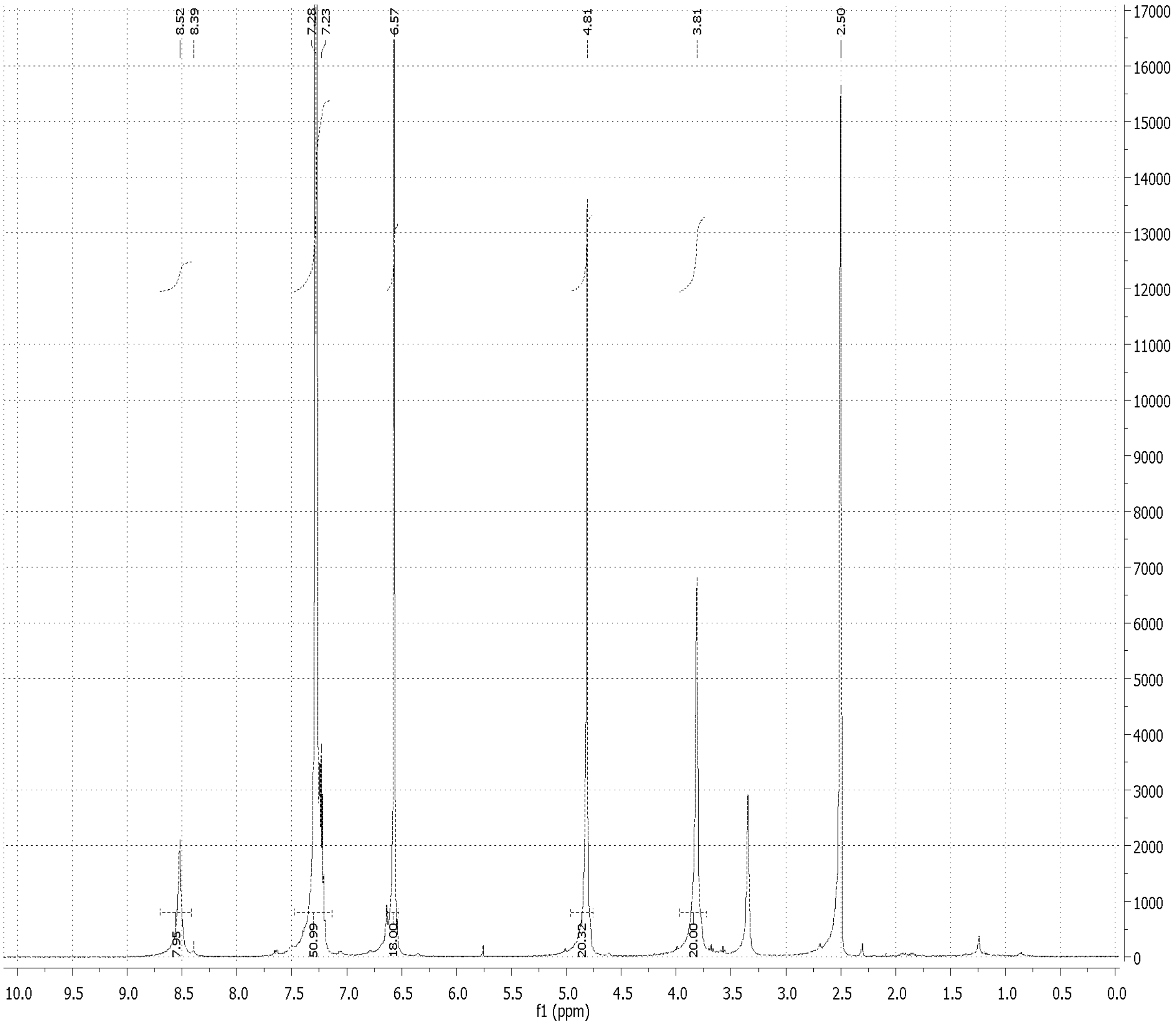

**Figure 23.2 : Fraction P7 (Calix[10]arene)**

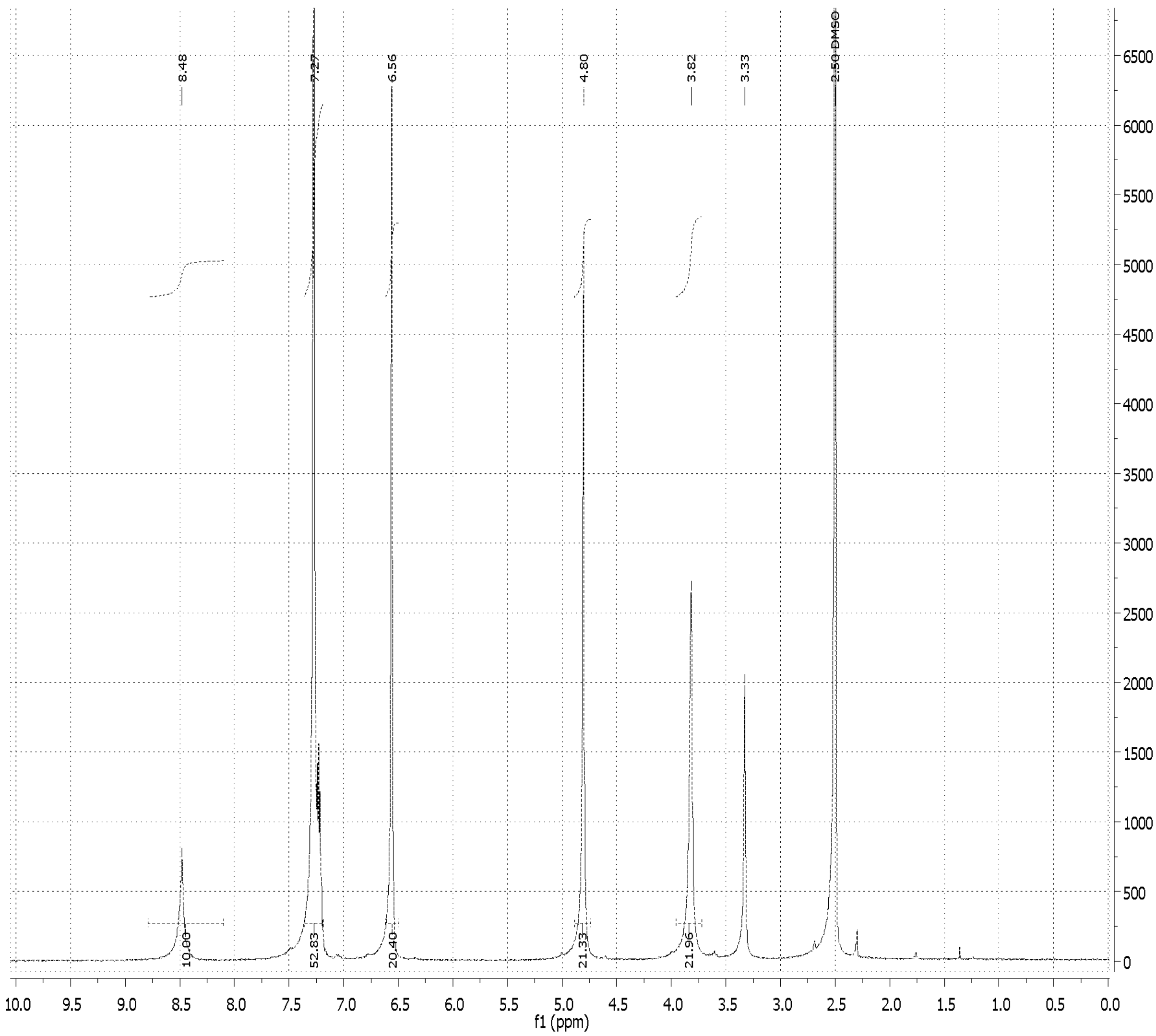

**Figure 23.3 : Fraction P10 (Calix[12]arene)**

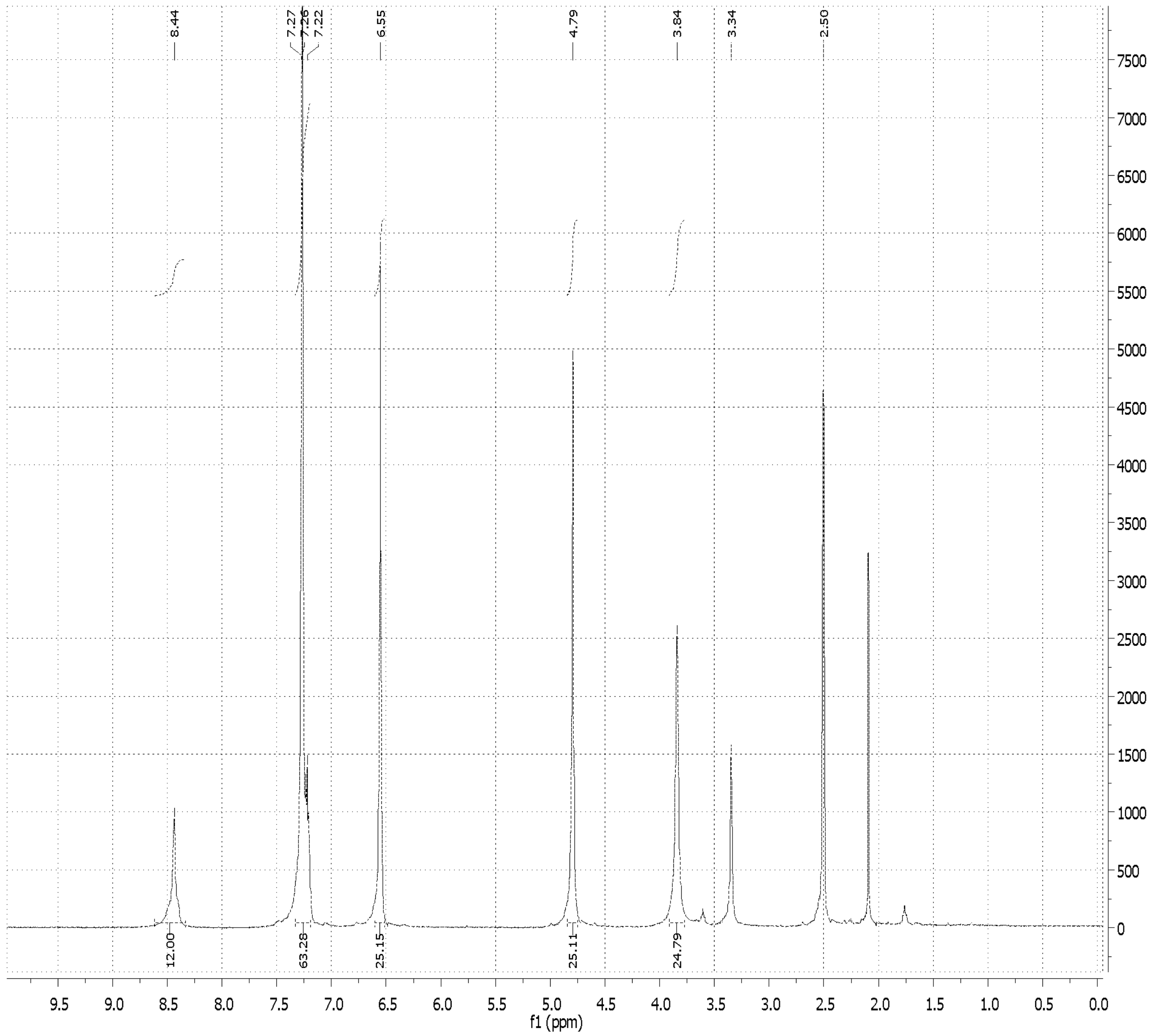

**Figure 23.4 : Fraction F16 (calix[13]arene)**

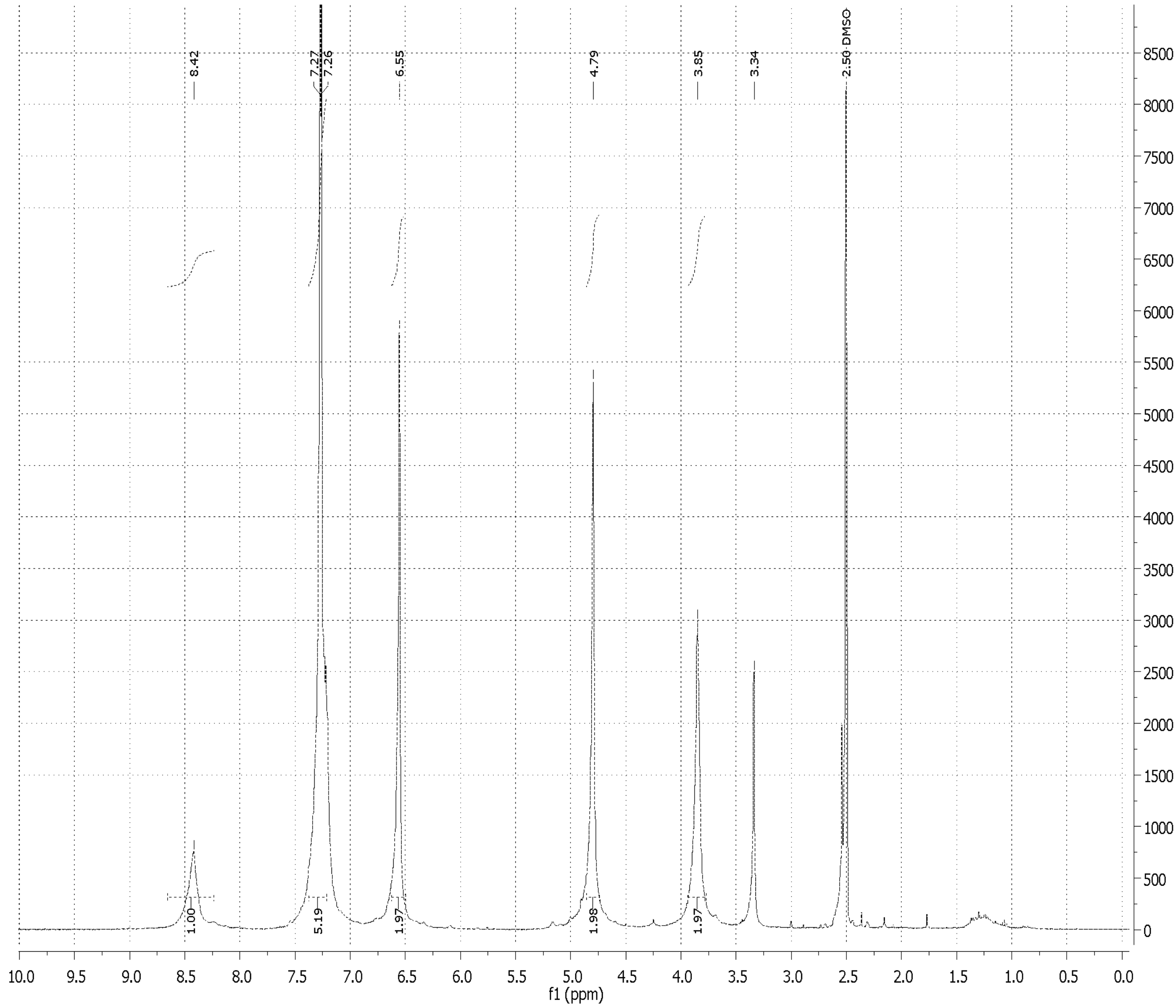

**Figure 23.5 : Fraction P4 (calix[16]arene)**

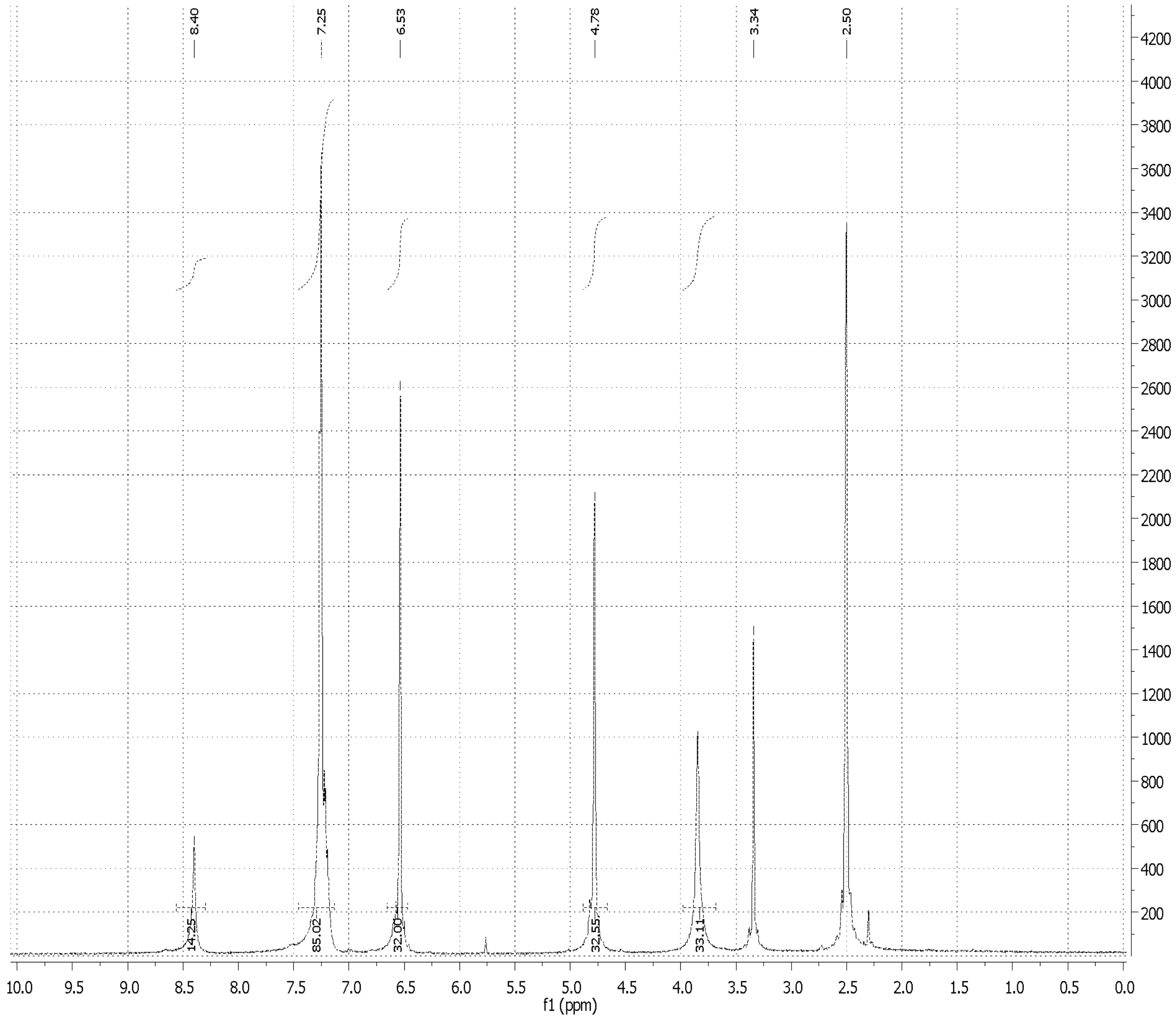

**Figure 24**

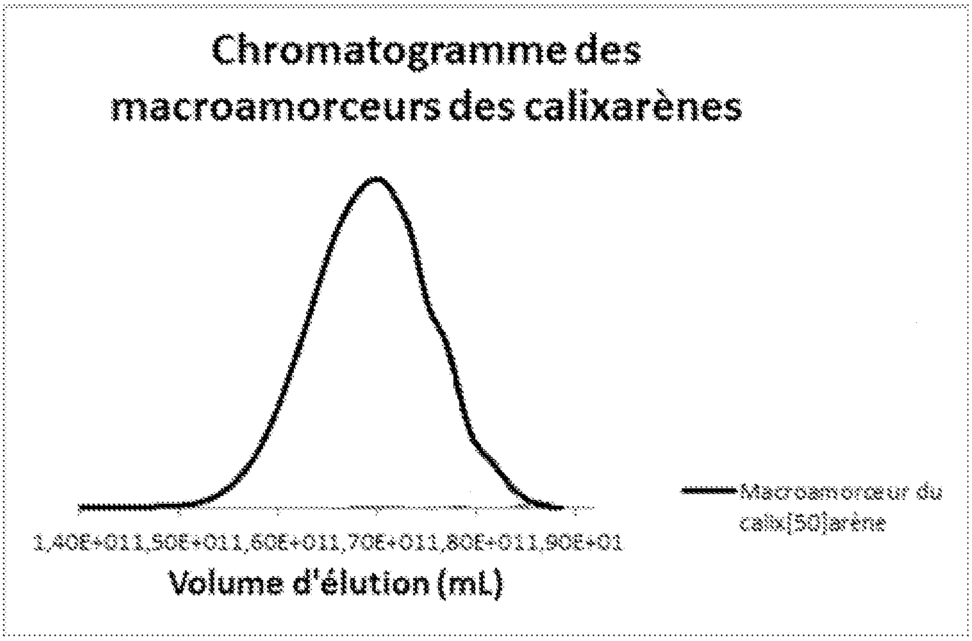


**Figure 25**

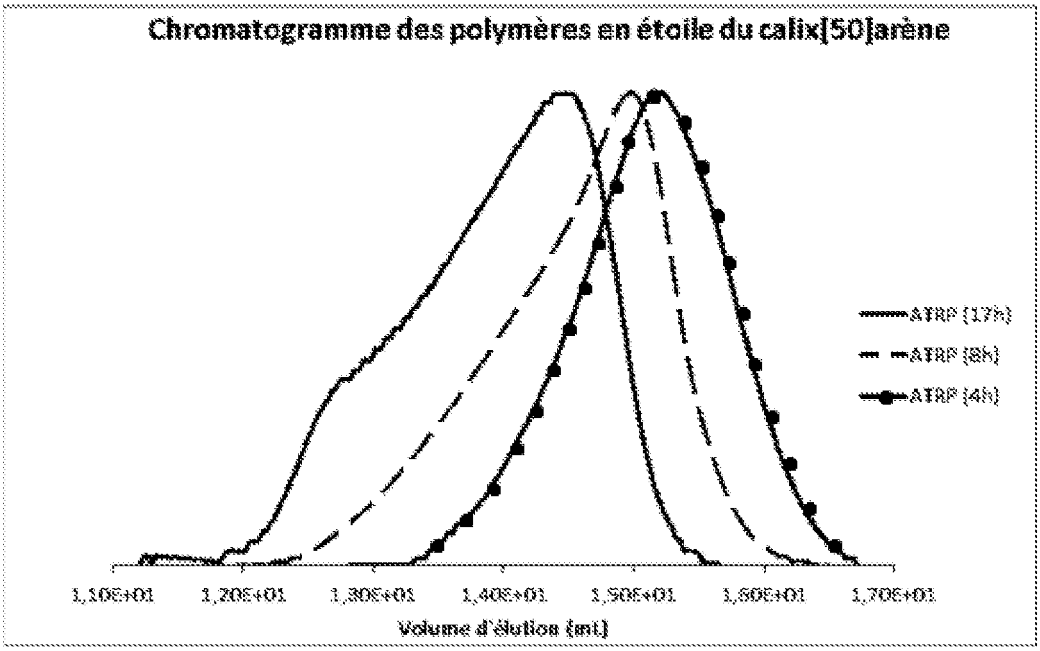

**Figure 26**

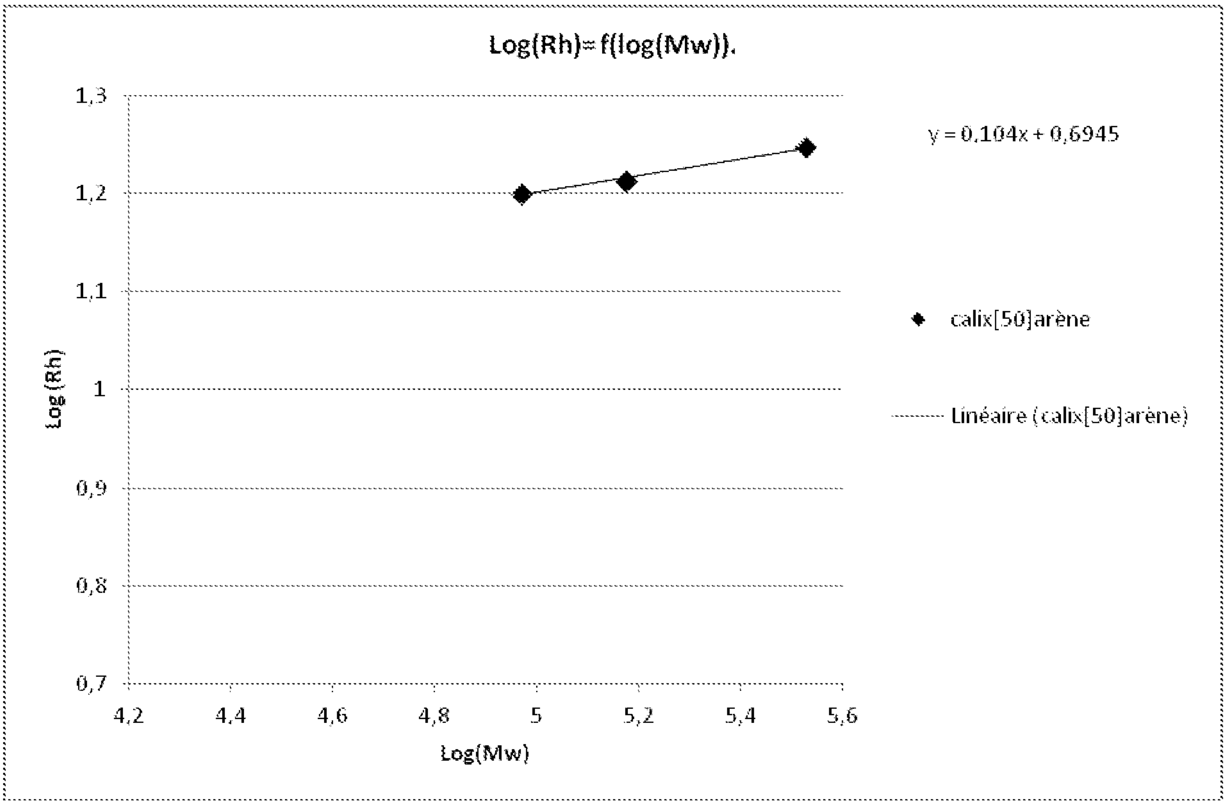
Log(Rh)= f(log(Mw)).
1,3
1,2
1,1
1
0,9
0,8
0,7
Log (Rh)
4,2
4,4
4,6
4,8
5
5,2
5,4
5,6
Log(Mw)
y = 0,104x + 0,6945
calix[50]arène
Linéaire (calix[50]arène)

**Figure 27**

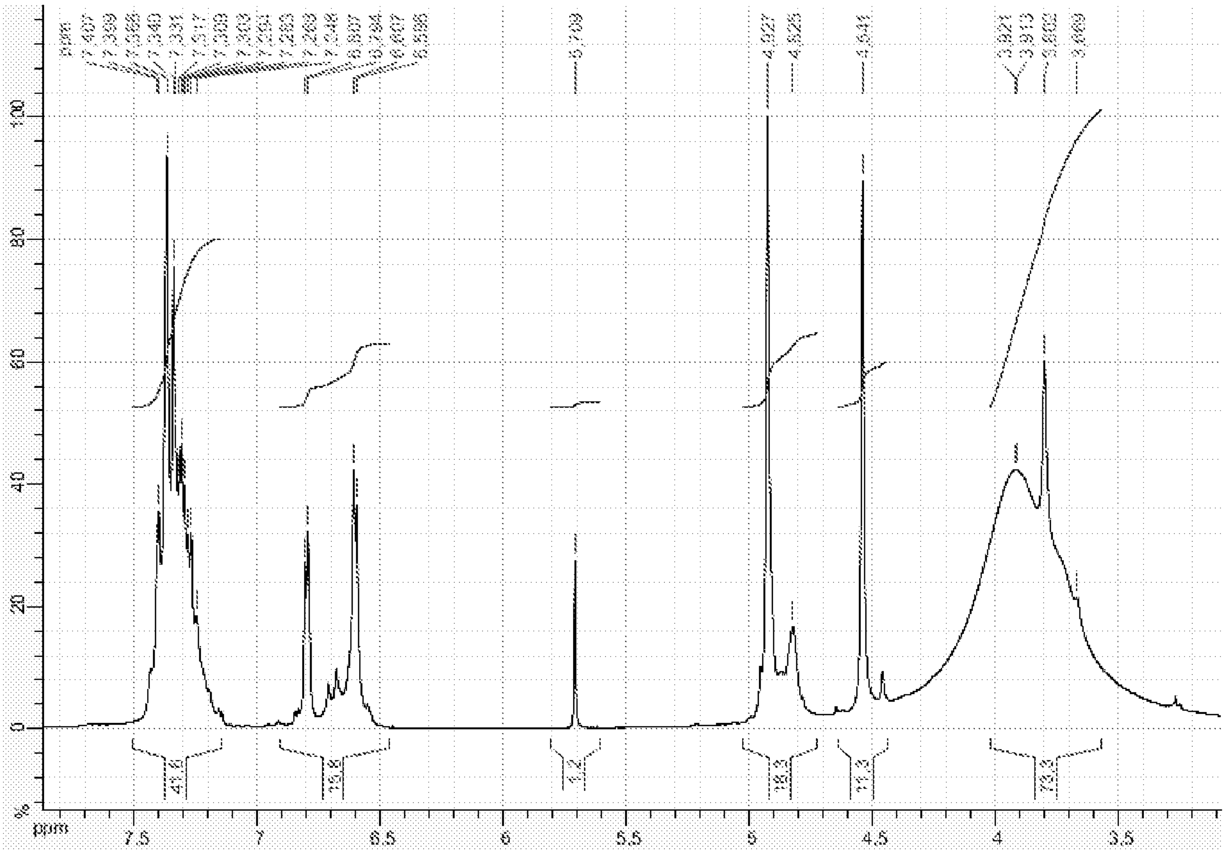

**Figure 28**

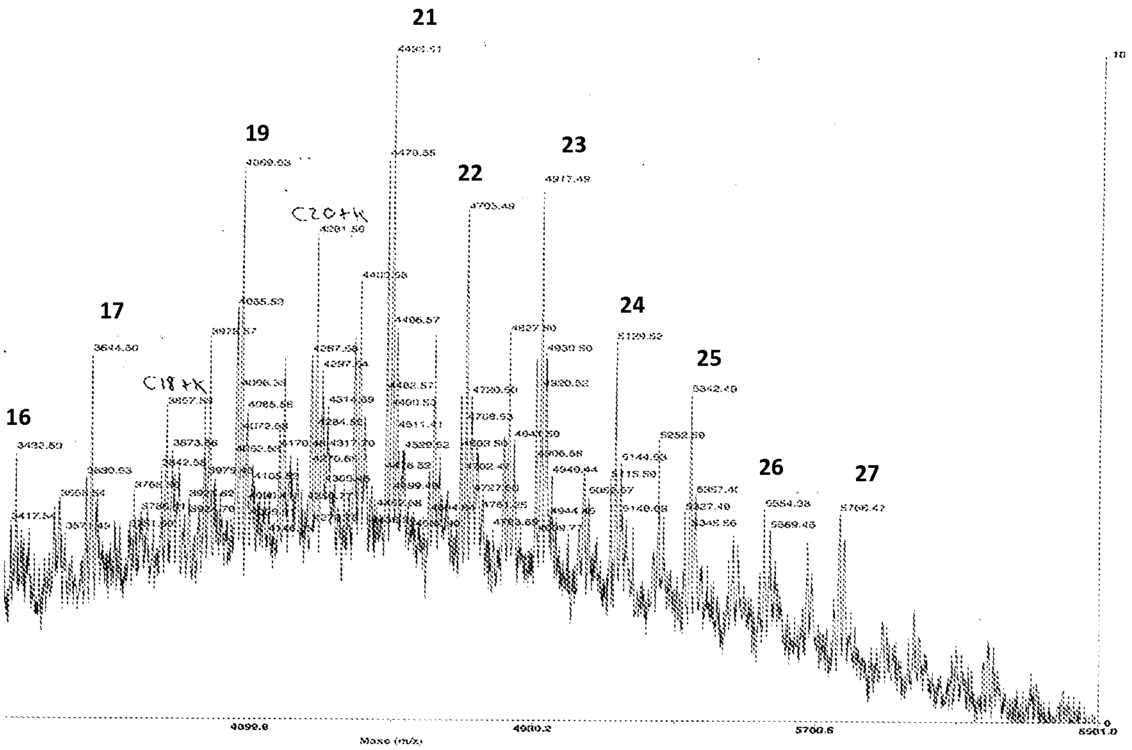

**Figure 29a**

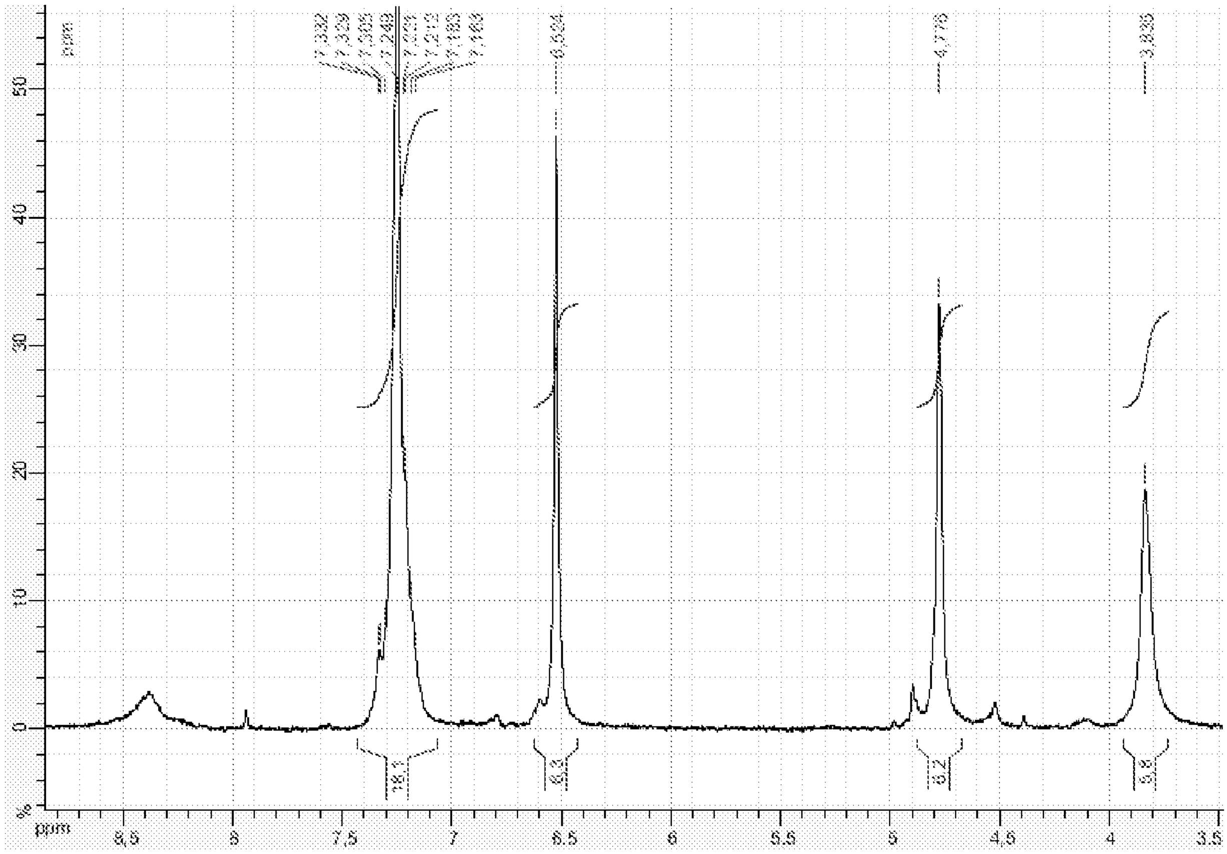

**Figure 29b**

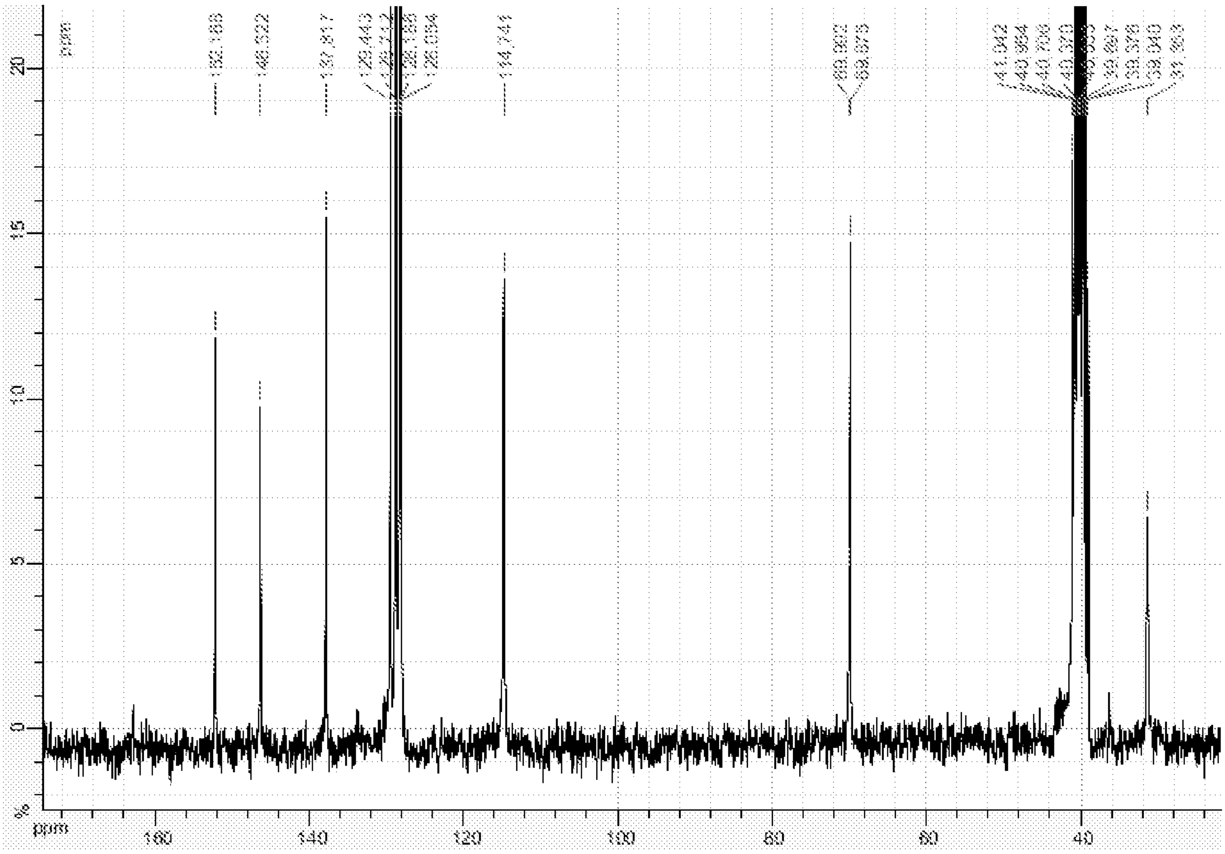
ppm
152.183
146.522
137.817
129.443
128.185
128.054
114.741
69.902
69.675
41.042
40.954
40.706
40.370
39.040
20
15
10
5
0
%
ppm
160
140
120
100
80
60
40

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- CA 2251070


### Littérature non-brevet citée dans la description


- **DE GUTSCHE ; D. GUTSCHE et al.** Calixarenes: An Introduction. The Royal Society of Chemistry, 2008
- **Z. ASFARI ; V. BÖHMER ; J. HARROWFIELD ; J. VICENS.** Calixarenes 2001. Kluwer, 2001
- **T. PATRICK ; P. EGAN.** *J. Org. Chem.,* 1977, vol. 42, 382
- **T. PATRICK ; P. EGAN.** *J. Org. Chem.,* 1978, vol. 43, 4280
- **Z. ASFARI ; J. VICENS.** *Tetrahedron Lett.,* 1988, vol. 29, 2659
- **F. VOCANSON ; M. PERRIN ; R. LAMARTINE.** *J. Inclusion Phenom. Macrocyclic Chem.,* 2001, vol. 39, 127
- **JERRY L. ATWOOD et al.** *Org. Lett.,* 1999, vol. 1, 1523
- **B. DAHWAN et al.** *Macromolec. Chem.,* 1987, vol. 188, 921
- **C. D. GUTSCHE et al.** *Org. Synth.,* 1990, vol. 68, 234
- **C. D. GUTSCHE et al.** *Org. Synth.,* 1990, vol. 68, 238
- **GUTSCHE et al.** *J. Am. Chem. Soc.,* 1999, vol. 121, 4136
- **GARSKA et al.** *Macromolecular Materials and Engineering,* 2012, vol. 297, 785
- **C. D. GUTSCHE.** *JACS,* 1999